# EUROPÄISCHE PATENTSCHRIFT

(11) **EP 2 875 092 B1**
(45) Veröffentlichungstag und Bekanntmachung des Hinweises auf die Patenterteilung: **15.02.2017**
(21) Anmeldenummer: 13732367.1
(22) Anmeldetag: 27.06.2013
(51) Int. Cl.: C09K 11/06, H05B 33/14, H01L 51/50, C07D 219/02, C07C 209/10, C07D 333/76, C07C 211/61, C07C 213/02, C07D 405/12, C07C 217/94, C07D 209/86, C07F 7/08, C07D 307/91, H01L 51/00

(54) **VERBINDUNGEN UND ORGANISCHE ELEKTROLUMINESZIERENDE VORRICHTUNGEN**
COMPOUNDS AND ORGANIC ELECTROLUMINESCENT DEVICES
COMPOSÉS ET DISPOSITIFS ELECTROLUMINESCENTS ORGANIQUES

(30) Priorität: 23.07.2012 EP 12005369
(43) Veröffentlichungstag der Anmeldung: 27.05.2015
(73) Patentinhaber: Merck Patent GmbH, 64293 Darmstadt (DE)
(72) Erfinder: MUJICA-FERNAUD, Teresa, 64283 Darmstadt (DE); MONTENEGRO, Elvira, 69469 Weinheim (DE); PARHAM, Amir Hossain, 65929 Frankfurt am Main (DE); BUESING, Arne, 65929 Frankfurt am Main (DE); VOGES, Frank, 67098 Bad Duerkheim (DE)
(86) Internationale Anmeldenummer: PCT/EP2013/001891
(87) Internationale Veröffentlichungsnummer: WO 2014/015937

(56) Entgegenhaltungen:
- EP-A1- 2 415 752
- WO-A1-2004/020387
- WO-A1-2007/072838
- WO-A1-2007/086701
- WO-A1-2012/034627
- DE-A1-102010 045 405
- JP-B2- 3 824 385
- US-A1- 2012 161 615

## Beschreibung

Die vorliegende Erfindung betrifft neue organische Verbindungen, die Verwendung von Verbindung in einer elektrolumineszierenden Vorrichtung, sowie eine elektrolumineszierende Vorrichtung enthaltend wenigstens eine der Verbindungen. Weiterhin betrifft die vorliegende Erfindung ein Verfahren zur Herstellung der Verbindungen sowie Zusammensetzungen und Formulierungen enthaltend wenigstens eine der Verbindungen.

Die Entwicklung von funktionellen Verbindungen zur Verwendung in elektrolumineszierenden Vorrichtungen ist aktuell Gegenstand intensiver Forschung. Ziel ist hierbei insbesondere die Entwicklung von Verbindungen, mit denen verbesserte Eigenschaften der elektrolumineszierenden Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektroluminesziernede Vorrichtung werden gemäß der vorliegenden Erfindung unter anderem organische lichtemittierende Transistoren (OLETs), organische Feld-Quench-Devices (OFQDs), organische lichtemittierende elektrochemische Zellen (OLECs, LECs oder LEECs), organische Laserdioden (O-Laser) und organische Elektrolumineszenzvorrichtungen (OLEDs) verstanden.

Von besonderem Interesse ist die Bereitstellung von Verbindungen zur Verwendung in den zuletzt genannten, als OLEDs (organische lichtemittierende Dioden) bezeichneten elektronischen Vorrichtungen. Der allgemeine Aufbau sowie das Funktionsprinzip von OLEDs ist dem Fachmann gut bekannt und unter anderem in US 4539507, US 5151629, EP 0676461 und WO 1998/27136 beschrieben.

Betreffend die Leistungsdaten von OLEDs sind noch weitere Verbesserungen erforderlich, insbesondere in Hinblick auf eine breite kommerzielle Verwendung, beispielsweise in Anzeigevorrichtungen oder als Lichtquellen. Von besonderer Bedeutung sind in diesem Zusammenhang die Lebensdauer, die Effizienz und die Betriebsspannung der OLEDs sowie die realisierten Farbwerte. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

In diesem Zusammenhang besteht insbesondere Bedarf an alternativen Lochtransportmaterialien. Bei Lochtransportmaterialien gemäß dem Stand der Technik steigt im Allgemeinen die Spannung mit zunehmender Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

Im Stand der Technik wird die Verwendung von verschiedenen Fluorenen als Ladungstransportmaterial in elektronischen und elektrolumineszierenden Vorrichtungen beschrieben.

Bei den im Stand der Technik beschriebenen Verbindungen handelt es sich allgemein um Verbindungen, die in Position 9 des Fluorens eine symmetrische Substitution aufweisen.

So z.B. WO2007072838 A1. In JP 05303221 werden Fluorene offenbart, die in Position 2 oder 4 mit einer Amingruppe substituiert sein können. Die Verbindungen mit der Amingruppe in Position 4 des Fluorens enthalten Phenyl-Reste. Einige wenige dieser Verbindungen zeigen eine Asymmetrische Substitution in Position 9 des Fluorens. Die Verbindungen werden als Photorezeptoren eingesetzt.

Trotz der bereits bekannten Verbindungen besteht unverändert Bedarf an neuen Lochtransport- und Lochinjektionsmaterialien zur Verwendung in OLEDs. Insbesondere besteht Bedarf an Materialien, mit denen die oben genannten, hoch erwünschten Verbesserungen der Leistungsdaten und Eigenschaften der OLEDs erreicht werden können.

Ebenfalls besteht Bedarf an neuen Matrixmaterialien zur Verwendung in OLEDs sowie in anderen elektronischen Vorrichtungen. Insbesondere besteht Bedarf an Matrixmaterialien für phosphoreszierende Dotanden sowie an Matrixmaterialien für Mixed-Matrix-Systeme, welche bevorzugt zu guter Effizienz, hoher Lebensdauer und geringer Betriebsspannung der elektronischen Vorrichtungen führen.

Der vorliegenden Erfindung liegt somit die Aufgabe zu Grunde, elektroluminesziernede Vorrichtungen und Verbindungen, welche sich zur Verwendung in elektrolumineszierenden Vorrichtungen wie beispielsweise fluoreszierenden oder phosphoreszierende OLEDs eignen, bereitzustellen und welche insbesondere als Lochinjektionsmaterialien und/oder Lochtransportmaterial in einer Loch¬Transport- bzw. Exzitonenblockierschicht oder als Matrix¬material in einer emittieren¬den Schicht, eingesetzt werden können.

Im Rahmen der vorliegenden Erfindung wurde überraschend gefunden, dass sich Verbindungen der unten angegebenen Formel (1) ausgezeichnet für die oben genannten Verwendungen in elektroluminesziernden Vorrichtungen eignen.

Gegenstand der Erfindung ist somit eine elektrolumineszierende Vorrichtung enthaltend wenigstens eine Verbindung der Formel (1) 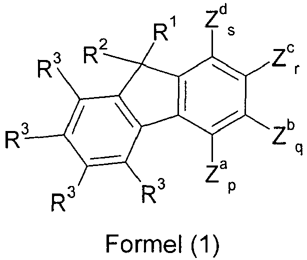 wobei für die verwendeten Symbole und Indices gilt:
p, q, r, s
   sind 0 oder 1, wobei p + q + r + s = 1 gilt, bevorzugt ist p =1 oder r = 1 oder s = 1, ganz bevorzugt ist p = 1 oder r = 1;
Z^{a}₀, Z^{b}₀, Z^{c}0, Z^{d}₀
   sind gleich oder verschieden bei jedem Auftreten gleich R⁴
Z^{a}₁, Z^{b}₁, Z^{c}₁, Z^{d}₁ sind gleich 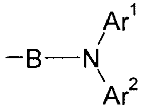
B ist eine Einfachbindung, eine bivalente Arylgruppe mit 6 bis 30 Ringatomen oder eine bivalente Heteroarylgruppe mit 5 bis 30 Ringatomen, die jeweils mit einem oder mehreren Resten R⁶ substituiert sein können,
   bevorzugt eine Einfachbindung oder eine Phenylen-, Biphenylen-, Terphenylen-, Naphthylen-, Pyridinylen-, Pyrimidinylen-, Pyrazinylen-, Pyridazinylen-, Triazinylen-, Dibenzofuranylen-, Dibenzothiophenylen- Fluorenylen-, oder Carbazoylengruppe, die mit einem oder mehreren Resten R⁶ substituiert sein können,
   ganz bevorzugt eine Einfachbindung oder eine Phenylen-, Biphenylen-, Terphenylen-, Naphthylen-, Dibenzofuranylen- oder Dibenzothiophenylen Fluorenylen-, oder Carbazoylengruppe, die mit einem oder mehreren Resten R⁶ substituiert sein können,
   ganz besonders bevorzugt ist B eine Einfachbindung oder eine Phenylengruppe, die mit einem oder mehreren Resten R⁶ substituiert sein kann,
   insbesondere bevorzugt ist B eine Einfachbindung,
   wobei, wenn B eine Einfachbindung ist, das Stickstoffatom direkt an das Fluoren gebunden ist;
Ar¹, Ar²
   sind bei jedem Auftreten gleich oder verschieden ein Arylgruppe mit 10 bis 60 Ringatomen oder eine Heteroarylgruppe 10 bis 60 Ringatomen, welche mit einem oder mehreren Resten R⁵, die gleich oder verschieden voneinander sind, substituiert sein können, wobei beide Gruppen Ar¹ oder Ar² jeweils mindestens zwei oder mehr aromatische oder heteroaromatische Ringe enthalten,
   wobei zwei der aromatischen oder heteroaromatischen Ringe in Ar¹ und/oder zwei der aromatischen oder heteroaromatischen Ringe in Ar² kondensiert sein können, bevorzugt liegen sie nicht kondensiert vor,
   und wobei zwei der aromatischen oder heteroaromatischen Ringe in Ar¹ durch eine bivalente Gruppe -O-, -S-, -Si(R⁵)₂-, -C(R⁵)₂ oder-NR⁵- verbrückt sein können bzw. zwei der aromatischen oder heteroaromatischen Ringe in Ar² durch eine bivalente Gruppe -O-, - S-, -Si(R⁵)₂-, -C(R⁵)₂,- oder -NR⁵- verbrückt sein können, wobei unverbrückte Ringe bevorzugt sind
   und wobei ein aromatischer oder heteroaromatischer Ring aus Ar¹ mit einem aromatischen oder heteroaromatischen Ring aus Ar² durch eine bivalente Gruppe -O-, -S-, -Si(R⁵)₂-, -NR⁵- oder -C(R⁵)₂- verbrückt sein kann, wobei unverbrückte Gruppen Ar¹ und Ar² bevorzugt sind;
R¹, R², R³, R⁴, und R⁵
   sind H, D, F, Cl, Br, I, C(=O)R⁶, CN, Si(R⁶)₃, NO₂, N(R⁶)₂, P(=O)(R⁶)₂, S(=O)R⁶, S(=O)₂R⁶, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, oder eine Aryloxygruppe mit 5 bis 60 aromatischen Ringatomen, die mit einem oder mehreren Resten R⁶ substituiert sein kann, oder einer Aralkylgruppe mit 5 bis 60 aromatischen Ringatomen, die jeweils mit einem oder mehreren Resten R⁶ substituiert sein kann, wobei die Reste R¹ und R² nicht identisch sein dürfen und die Reste R³ bis R⁵ bei jedem Auftreten gleich oder verschieden sein können aber identisch mit entweder R¹ oder mit R² sein kann;
R⁶ ist bei jedem Auftreten gleich oder verschieden H, D, F, Cl, Br, I, C(=O)R⁷, CN, Si(R⁷)₃, NO₂, P(=O)(R⁷)₂, S(=O)R⁷, S(=O)₂R⁷, N(R⁷)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁷ substituiert sein können und wobei eine oder mehrere CH₂-Gruppen in den oben genannten Gruppen durch -R⁷C=CR⁷-, -C=C-, Si(R⁷)₂, C=O, C=S, C=NR⁷, -C(=O)O-, -C(=O)NR⁷-, P(=O)(R⁷), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁷ substituiert sein kann, oder eine Aryloxy- oder Heteroaryloxygruppe mit 5 bis 30 aromatischen Ringatomen, die durch einen oder mehrere Reste R⁷ substituiert sein kann, wobei zwei oder mehr benachbarte Substituenten R⁶ miteinander ein mono- oder polycyclisches Ringsystem bilden können;
R⁷ ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R⁷ miteinander ein mono- oder polycyclisches Ringsystem bilden können.

In einer bevorzugten Ausführungsform enthält die Verbindung nach Formel (1) keine kondensierten aromatischen oder heteroaromatischen Ringsysteme mit mehr als 10 Ringatomen.

Durch die asymmetrische Substitution in Position 9 des Fluorens entsteht, bei geeigneter Substitution des Fluorens in den anderen Positionen, ein chriales Molekül. In der vorliegenden Erfindung sind neben den enantiomerenreinen R- und S-Formen auch die Racemate umfasst. Die erfindungsgemäßen elektrolumineszierenden Vorrichtungen enthalten daher entweder wenigstens eine der Verbindung nach Formel (1) in der R- oder S-Form oder als Racemat, bevorzugt als Racemat.

Die Zählweise am Fluoren ist dabei wie folgt festgelegt.

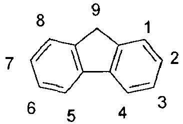

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Es ist weiterhin bevorzugt, wenn B in der Verbindung nach Formel (1) eine o-Phenylen-, m-Phenylen- oder p-Phenylengruppe, eine 1,4-Naphthylen-, 2,4-Naphthylen-, 1,5-Naphthylen- oder 2,5-Naphthylengruppe, eine 3,7-Dibenzofuranylengruppe oder eine 3,7-Dibenzothiophenylengruppe ist, wobei ganz bevorzugt ist, wenn B eine o-Phenylen-, m-Phenylen- oder p-Phenylengruppe und ganz besonders bevorzugt ist, wenn B eine p-Phenylengruppe ist, wobei die Gruppen mit einem oder mehreren Resten R⁴ substituiert sein können, die bei jedem Auftreten gleich oder verschieden sein können, wobei bevorzugt ist, wenn die Gruppen unsubstituiert sind.

Bevorzugt im Sinne der vorliegenden Erfindung ist eine elektrolumineszierende Vorrichtung enthaltend wenigstens eine Verbindung der allgemeinen Formel (2) 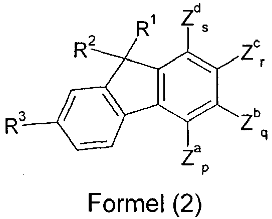 wobei für die verwendeten Indices und Symbole obige Definitionen gelten.

Weiterhin bevorzugt ist eine elektrolumineszierende Vorrichtung enthaltend wenigstens eine Verbindung der allgemeinen Formel (1) oder (2), dadurch charakterisiert, dass die Reste R¹ und R², die verschieden voneinander sind, eine geradkettige Alkyl- oder Alkoxygruppe mit 1 bis 20 C-Atomen oder eine verzweigte oder cyclische Alkyl- oder Alkoxygruppe mit 3 bis 20 C-Atomen sind, wobei die oben genannten Gruppen jeweils mit einem oder mehreren Resten R⁶ substituiert sein können oder aus ein aromatisches oder heteroaromatisches Ringsystem mit 6 bis 30 aromatischen Ringatomen, das jeweils durch einen oder mehrere Reste R⁶ substituiert sein kann, oder eine Aryloxygruppe mit 5 bis 60 aromatischen Ringatomen, die mit einem oder mehreren Resten R⁶ substituiert sein kann, oder einer Aralkylgruppe mit 5 bis 60 aromatischen Ringatomen, die jeweils mit einem oder mehreren Resten R⁶ substituiert sein kann.

Weiterhin ganz bevorzugt ist, wenn die Reste R¹ und R², die verschieden voneinander sind, eine geradkettige Alkylgruppe mit 1 bis 20 C-Atomen oder eine verzweigte oder cyclische Alkylgruppe mit 3 bis 20 C-Atomen sind, wobei die oben genannten Gruppen jeweils mit einem oder mehreren Resten R⁶ substituiert sein können oder ein aromatisches oder heteroaromatisches Ringsystem mit 6 bis 30 aromatischen Ringatomen, das jeweils durch einen oder mehrere Reste R⁶ substituiert sein kann.

Weiterhin ganz besonders bevorzugt ist, wenn einer der beiden Reste R¹ und R² eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁶ substituiert sein können, wobei insbesondere bevorzugt ist, wenn einer der beiden Reste R¹ und R² eine Methyl-, Ethyl-, n-/i-Propyl- oder n-/i-/t-Butylgruppe ist und der andere der beiden Reste R¹ und R² ein aromatisches oder heteroaromatisches Ringsystem mit 6 bis 30 aromatischen Ringatomen ist, das jeweils durch einen oder mehrere Reste R⁶ substituiert sein kann, wobei das Ringsystem insbesondere bevorzugt ausgewählt ist aus der Gruppe bestehend aus einer Phenyl-, Biphenyl-, Terphenyl- oder Pyridylgruppe.

Weiterhin bevorzugt ist eine elektrolumineszierende Vorrichtung enthaltend wenigstens eine Verbindung der allgemeinen Formel (1) oder (2), dadurch charakterisiert, dass R³ bei jedem Auftreten gleich oder verschieden, bevorzugt gleich, ausgewählt ist aus H, D, F, Cl, Br, I, N(R⁶)₂, einer geradkettigen Alkyl-, Alkoxylgruppe mit 1 bis 20 C-Atomen oder einer verzweigten oder cyclischen Alkyl-, Alkoxygruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R⁶ substituiert sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, oder eine Aryloxygruppe mit 5 bis 60 aromatischen Ringatomen, die mit einem oder mehreren Resten R⁶ substituiert sein kann, oder einer Aralkylgruppe mit 5 bis 60 aromatischen Ringatomen, die jeweils mit einem oder mehreren Resten R⁶ substituiert sein kann, wobei zwei oder mehr Reste R³ miteinander verknüpft sein können und einen Ring bilden.

Weiterhin ganz bevorzugt ist, wenn der Rest R³ bei jedem Auftreten gleich oder verschieden, bevorzugt gleich, ausgewählt ist aus H, D, F, Cl, Br, I, N(R⁶)₂, einer geradkettigen Alkylgruppe mit 1 bis 20 C-Atomen oder einer verzweigten oder cyclischen Alkylgruppe mit 3 bis 20 C-Atomen, wobei die oben genannten Gruppen jeweils mit einem oder mehreren Resten R⁶ substituiert sein können und wobei ein oder mehrere H-Atome in den oben genannten Gruppen durch D, F, Cl, Br, I, CN oder NO₂ ersetzt sein können, oder einem aromatischen oder heteroaromatischen Ringsystem mit 6 bis 30 aromatischen Ringatomen, das jeweils durch einen oder mehrere Reste R⁶ substituiert sein kann. Einige der besonders bevorzugten aromatischen oder heteroaromatischen Ringsysteme für die Reste R³ sind ausgewählt aus der Gruppe bestehend aus einer Phenyl-, Biphenyl-, Terphenyl-, Quarterphenyl-, Pyridyl-, Triazinyl-, Naphthly-, Fluorenyl-, Dibenzothiophenyl-, Dibenzofuranylgruppe, wobei die jeweiligen Gruppen mit einem oder mehreren Resten R⁶ substituiert sein können.

In einer ganz besonders bevorzugten Ausführungsform betrifft die vorliegende Erfindung eine elektrolummineszierende Vorrichtung enthaltend wenigstens eine Verbindung der allgemeinen Formel (1), die dadurch charakterisiert ist, dass R³ gleich H ist.

In einer weiteren ganz besonders bevorzugten Ausführungsform betrifft die vorliegende Erfindung eine elektrolumineszierende Vorrichtung enthaltend wenigstens eine Verbindung der allgemeinen Formel (1), die dadurch charakterisiert ist, dass R³ eine geradkettigen Alkylgruppe mit 1 bis 20 C-Atomen oder einer verzweigten oder cyclischen Alkylgruppe mit 3 bis 20 C-Atomen ist.

In noch einer weiteren ganz besonders bevorzugten Ausführungsform betrifft die vorliegende Erfindung eine elektrolumineszierende Vorrichtung enthaltend wenigstens eine Verbindung der allgemeinen Formel (1), die dadurch charakterisiert ist, dass R³ ein aromatisches oder heteroaromatisches Ringsystem mit 6 bis 30 aromatischen Ringatomen darstellt.

Weiterhin bevorzugt ist eine elektrolumineszierende Vorrichtung enthaltend wenigstens eine Verbindung der allgemeinen Formel (1) oder (2), dadurch charakterisiert, dass R⁴ bei jedem Auftreten gleich oder verschieden ausgewählt ist aus H, D, F, Cl, Br, I, -N(R⁶)₂ eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁶ substituiert sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, oder eine Aryloxygruppe mit 5 bis 60 aromatischen Ringatomen, die mit einem oder mehreren Resten R⁶ substituiert sein kann, oder einer Aralkylgruppe mit 5 bis 60 aromatischen Ringatomen, die jeweils mit einem oder mehreren Resten R⁶ substituiert sein kann, wobei zwei oder mehr Reste R⁴ miteinander verknüpft sein können und einen Ring bilden können, wobei bevorzugt ist, wenn die Reste R⁴ keinen Ringschluss bilden.

In einer bevorzugten Ausführungsform betrifft die vorliegende Erfindung eine elektrolumineszierende Vorrichtung enthaltend wenigstens eine Verbindung der allgemeinen Formel (3) 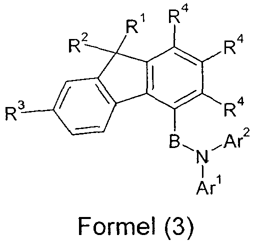 wobei für die verwendeten Symbole und Indices obige Definitionen gelten.

In einer weiterhin bevorzugten Ausführungsform betrifft die vorliegende Erfindung eine elektrolumineszierende Vorrichtung enthaltend wenigstens eine Verbindung der allgemeinen Formel (4) 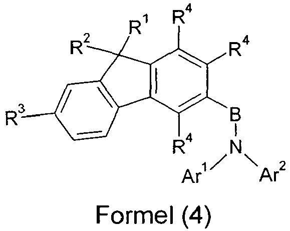 wobei für die verwendeten Symbole und Indices obige Definitionen gelten.

In einer weiterhin bevorzugten Ausführungsform betrifft die vorliegende Erfindung eine elektrolumineszierende Vorrichtung enthaltend wenigstens eine Verbindung der allgemeinen Formel (5) 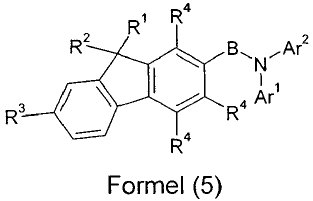 wobei für die verwendeten Symbole und Indices obige Definitionen gelten.

In einer weiterhin bevorzugten Ausführungsform betrifft die vorliegende Erfindung eine elektrolumineszierende Vorrichtung enthaltend wenigstens eine Verbindung der allgemeinen Formel (6) 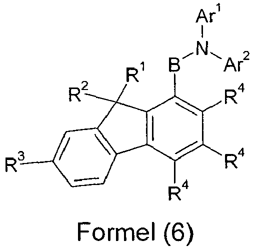 wobei für die verwendeten Symbole und Indices obige Definitionen gelten.

In einer ganz bevorzugten Ausführungsform betrifft die vorliegende Erfindung eine elektrolumineszierende Vorrichtung enthaltend wenigstens eine Verbindung der allgemeinen Formel (7) 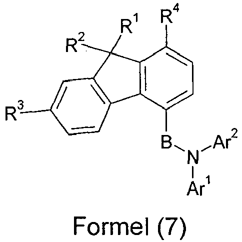 wobei für die verwendeten Symbole und Indices obige Definitionen gelten.

In einer weiterhin ganz bevorzugten Ausführungsform betrifft die vorliegende Erfindung eine elektrolumineszierende Vorrichtung enthaltend wenigstens eine Verbindung der allgemeinen Formel (8) 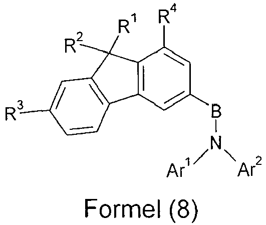 wobei für die verwendeten Symbole und Indices obige Definitionen gelten.

In einer weiterhin ganz bevorzugten Ausführungsform betrifft die vorliegende Erfindung eine elektrolumineszierende Vorrichtung enthaltend wenigstens eine Verbindung der allgemeinen Formel (9) 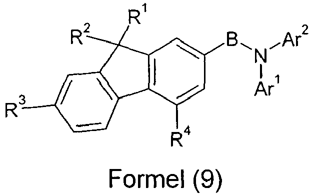 wobei für die verwendeten Symbole und Indices obige Definitionen gelten.

In einer ganz besonders bevorzugten Ausführungsform betrifft die vorliegende Erfindung eine elektrolumineszierende Vorrichtung enthaltend wenigstens eine Verbindung der allgemeinen Formel (10) 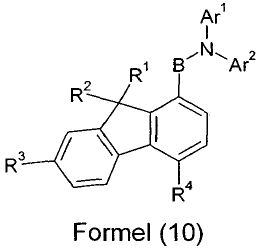 wobei für die verwendeten Symbole und Indices obige Definitionen gelten.

In einer weiterhin ganz bevorzugten Ausführungsform betrifft die vorliegende Erfindung eine elektrolumineszierende Vorrichtung enthaltend wenigstens eine Verbindung der allgemeinen Formel (11) 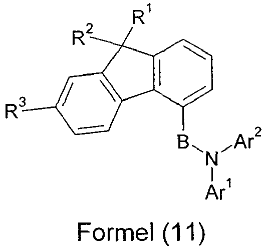 wobei für die verwendeten Symbole und Indices obige Definitionen gelten.

In einer weiterhin ganz bevorzugten Ausführungsform betrifft die vorliegende Erfindung eine elektrolumineszierende Vorrichtung enthaltend wenigstens eine Verbindung der allgemeinen Formel (12) 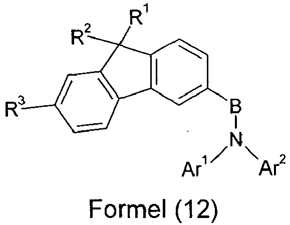 wobei für die verwendeten Symbole und Indices obige Definitionen gelten.

In einer weiterhin ganz bevorzugten Ausführungsform betrifft die vorliegende Erfindung eine elektrolumineszierende Vorrichtung enthaltend wenigstens eine Verbindung der allgemeinen Formel (13) 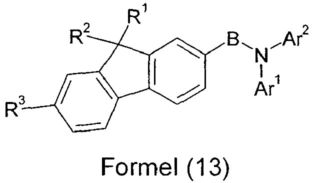 wobei für die verwendeten Symbole und Indices obige Definitionen gelten.

In einer ganz besonders bevorzugten Ausführungsform betrifft die vorliegende Erfindung eine elektrolumineszierende Vorrichtung enthaltend wenigstens eine Verbindung der allgemeinen Formel (14) 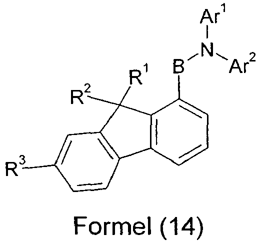 wobei für die verwendeten Symbole und Indices obige Definitionen gelten.

Weiterhin bevorzugt ist eine Verbindung der oben angegebenen Formeln (1) bis (14) in denen B ausgewählt ist aus den Gruppen der Formeln (15) bis (36), wobei diese Gruppen noch durch ein oder mehrere voneinander unabhängige Reste R⁶ substituiert sein können und wobei R⁶ wie oben angegeben definiert ist. 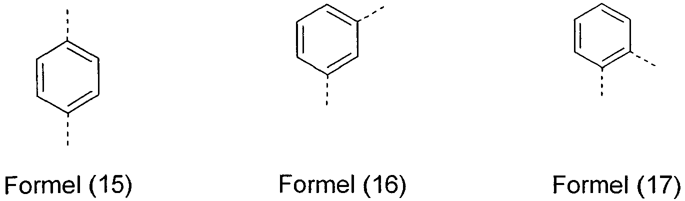 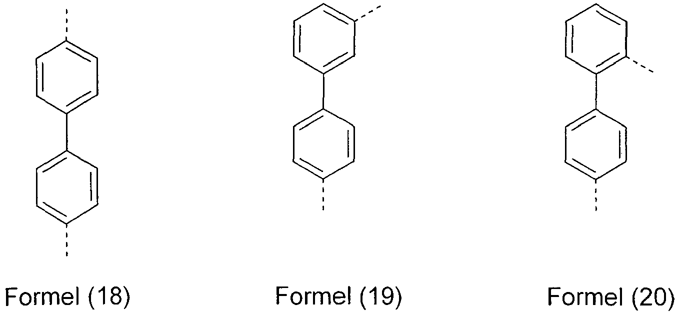 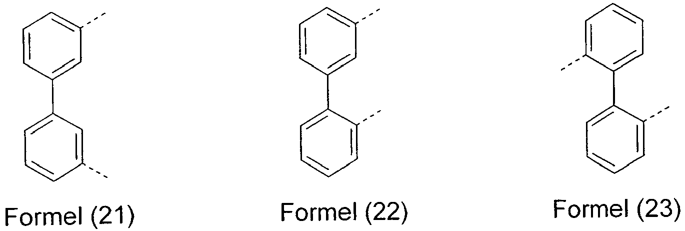 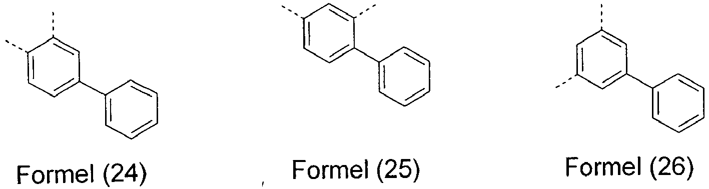 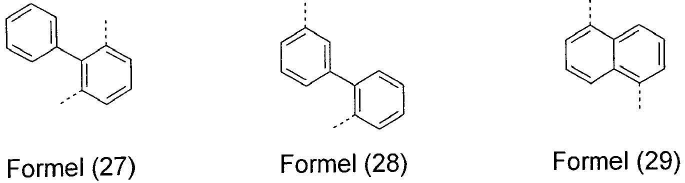 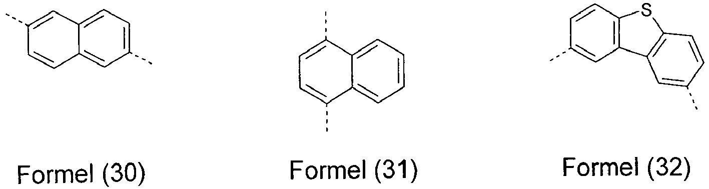 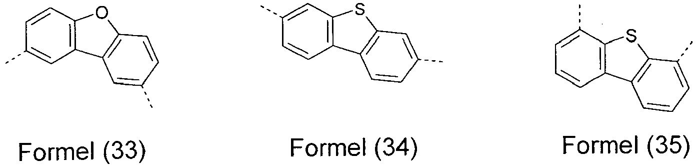 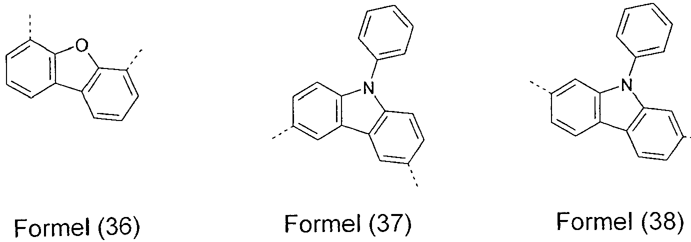 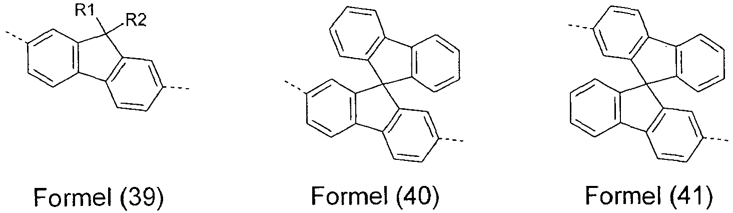 wobei die gestrichelten Linien die Verknüpfungspositionen kennzeichnen.

Ganz bevorzugt ist eine Verbindung der oben angegebenen Formeln (1) bis (14) in denen B ausgewählt ist aus den Gruppen der Formeln (15) bis (41), wobei diese Gruppen unsubstituiert sind.

Ganz besonders bevorzugt ist eine Verbindung der oben angegebenen Formeln (1) bis (14) in denen B der Formel (15) entspricht, wobei diese Gruppe unsubstituiert ist.

Insbesondere bevorzugt ist eine Verbindung der Formeln (1) bis (14), dadurch gekennzeichnet, dass B eine Einfachbindung ist, wobei dann gilt, dass das Stickstoffatom direkt über eine Einfachbindung an das Fluoren gebunden ist.

Bevorzugt sind Ar¹ und Ar² sind, gleich oder verschieden bei jedem Auftreten, bevorzugt ausgewählt aus einer Phenyl-Pyridyl-, Phenyl-Naphthyl-, Biphenyl-, Terphenyl- oder Quarterphenylgruppe, die mit einem oder mehreren Resten R⁶, die gleich oder verschieden voneinander sein können, substituiert sein können, wobei zwei der aromatischen oder heteroaromatischen Ringe in Ar¹ durch eine bivalente Gruppe -O-, -S-,-C(R⁵)₂- oder -Si(R⁵)₂- verbrückt sein können bzw. zwei der aromatischen oder heteroaromatischen Ringe in Ar² durch eine bivalente Gruppe -O-, -S-, -C(R⁵)₂- oder -Si(R⁵)₂- verbrückt sein können, wobei unverbrückte Ringe bevorzugt sind, und wobei ein aromatischer oder heteroaromatischer Ring aus Ar¹ mit einem aromatischen oder heteroaromatischen Ring aus Ar² durch eine bivalente Gruppe -O-, -S-, -Si(R⁵)₂-, -NR⁵- oder -C(R⁵)₂-verbrückt sein kann, wobei unverbrückte Gruppen Ar¹ und Ar² bevorzugt sind.

In einer ganz bevorzugten Ausführungsform der vorliegenden Erfindung sind Ar¹ und Ar², gleich oder verschieden bei jedem Auftreten, ausgewählt aus der folgenden Gruppen der Formeln (42) bis (142), die mit einem oder mehreren Resten R⁵ substituiert sein können. 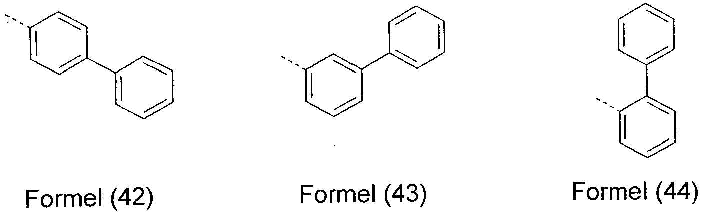 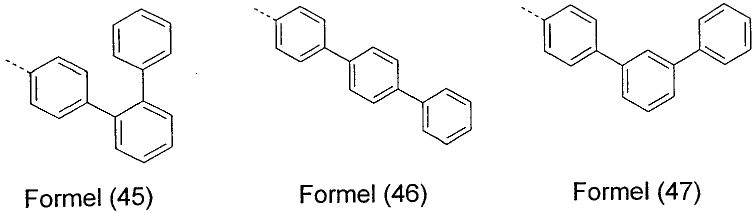 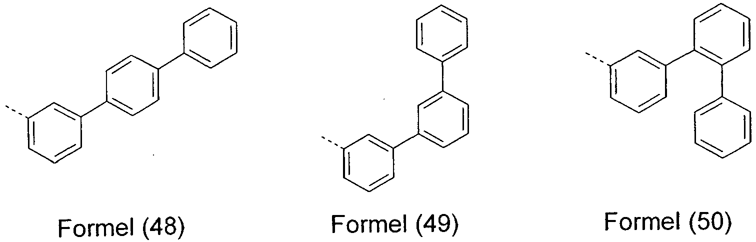 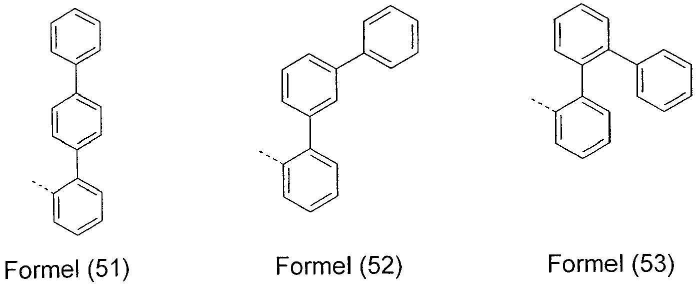 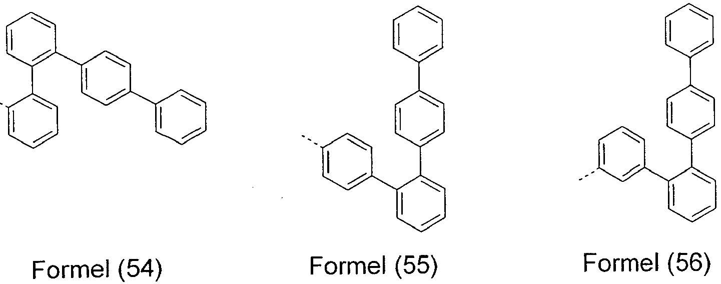 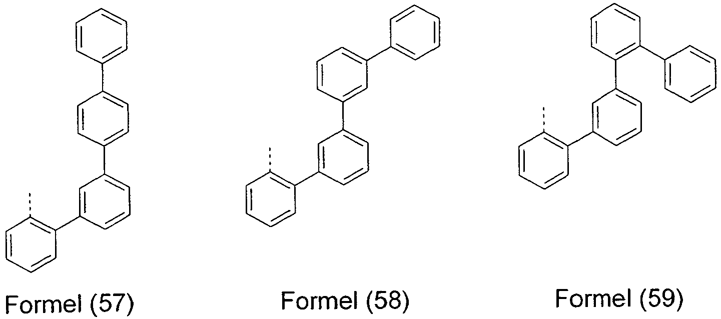 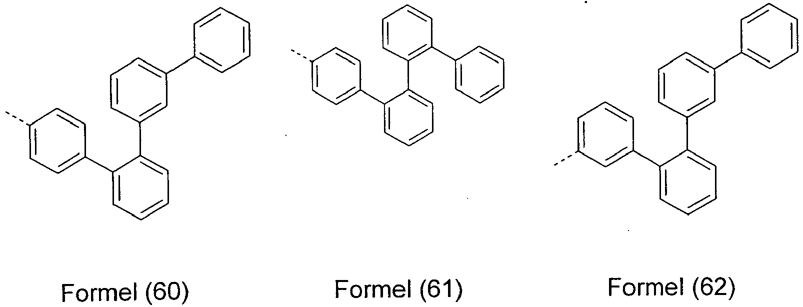 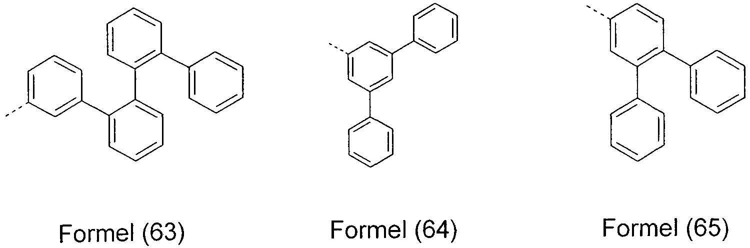 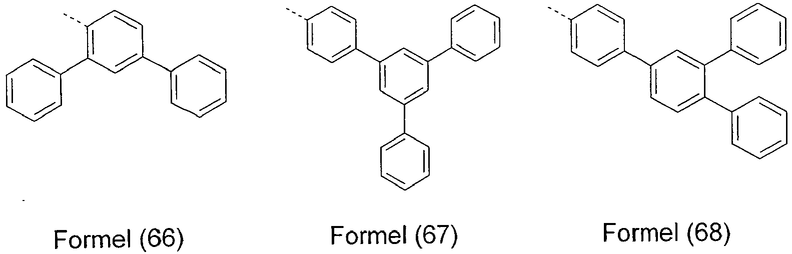 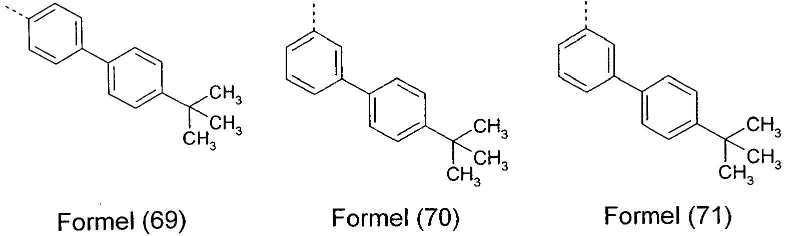 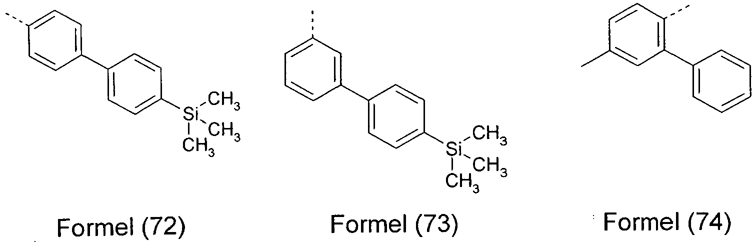 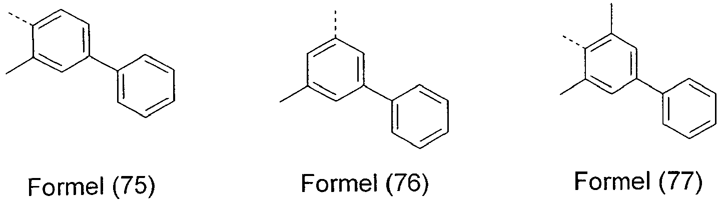 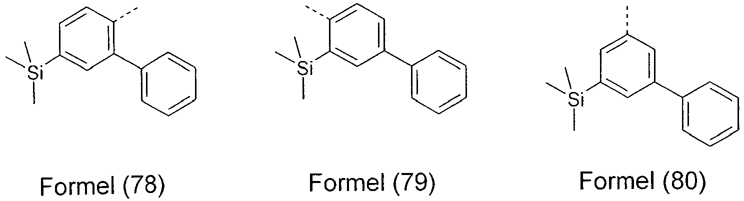 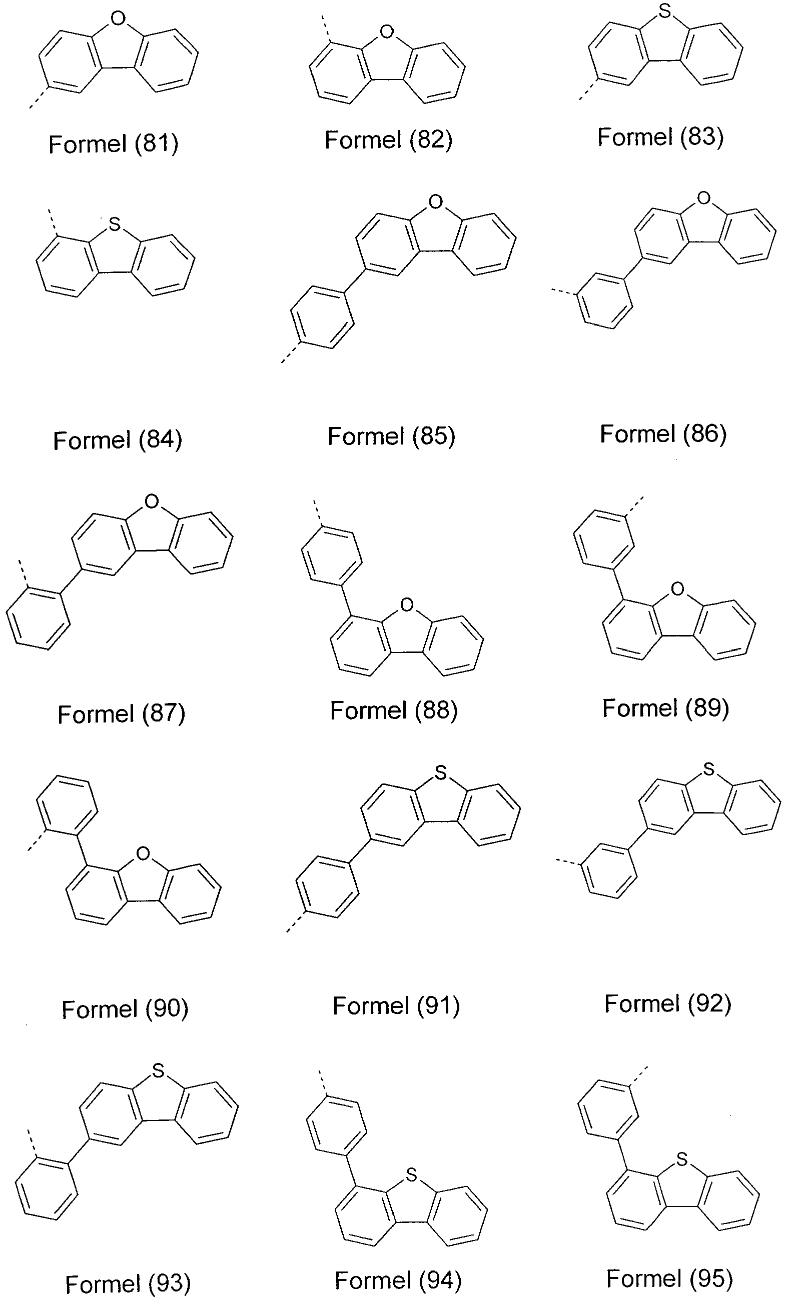 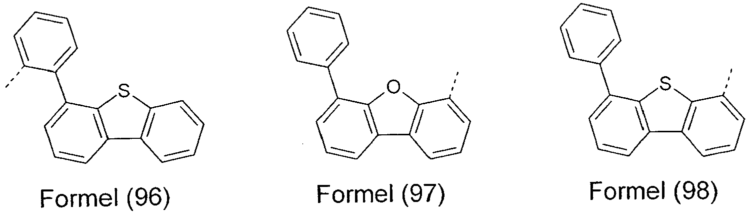 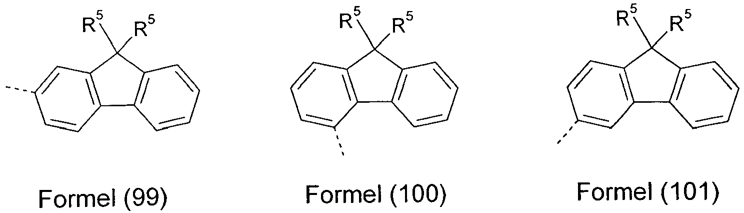 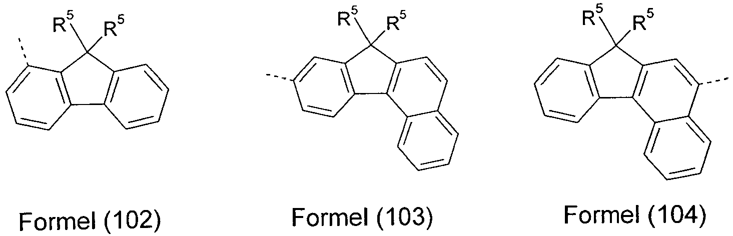 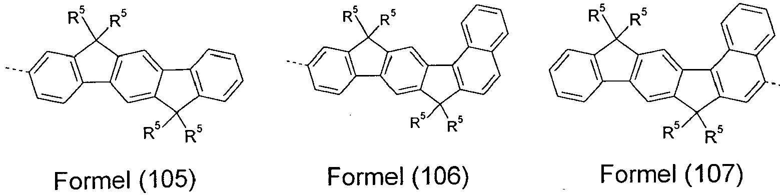 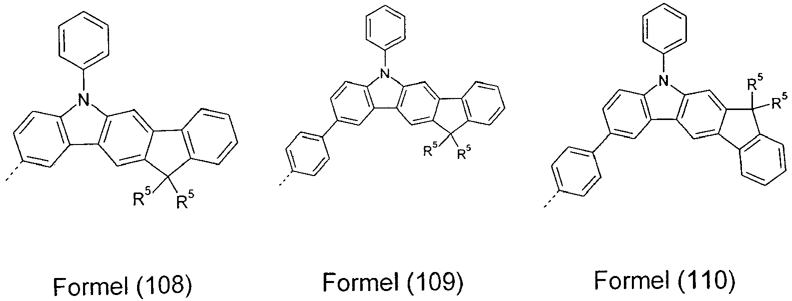 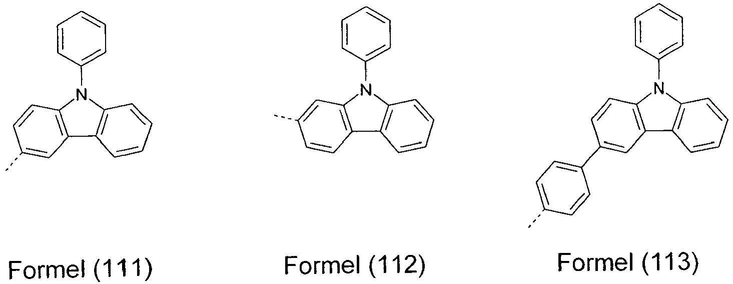 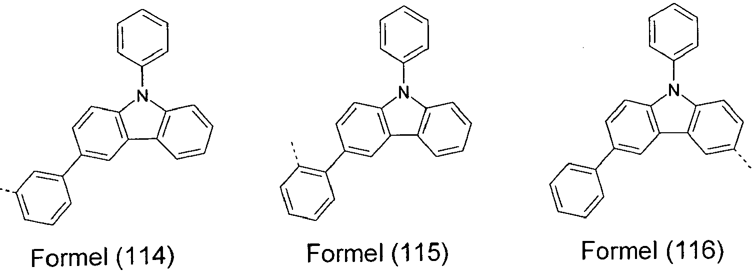 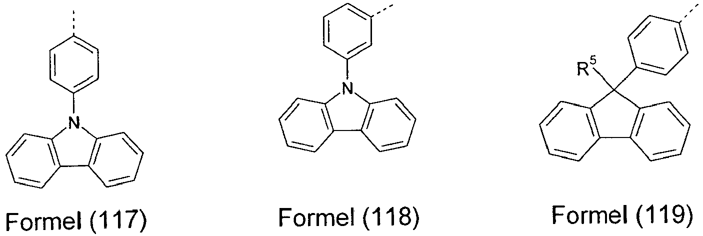 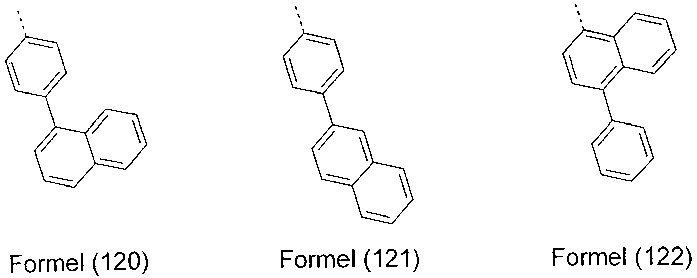 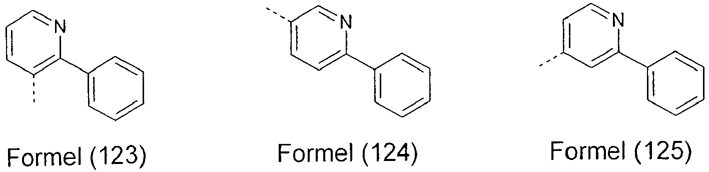 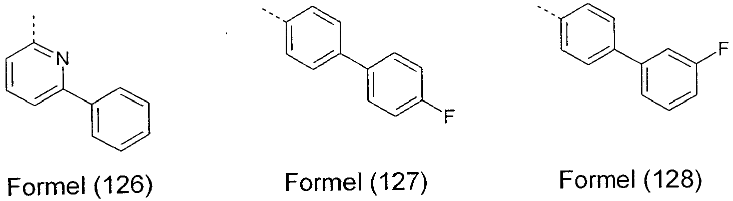 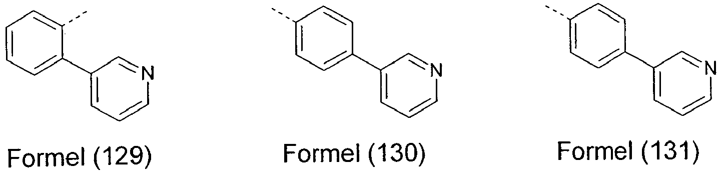 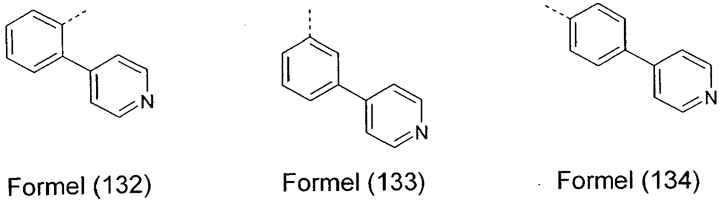 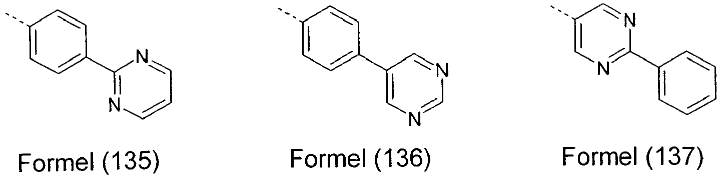 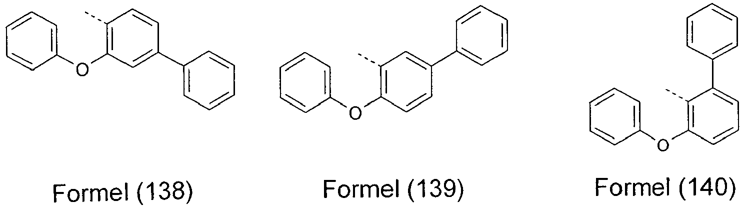 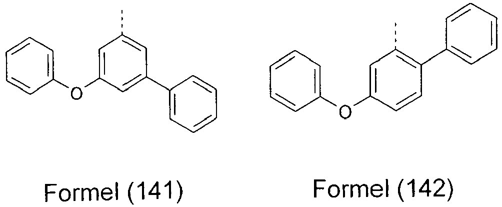 wobei die gestrichelte Linie die Verknüpfungsposition zum Stickstoffatom kennzeichnet.

Insbesondere bevorzugt ist eine elektrolumineszierende Vorrichtung enthaltend wenigstens eine Verbindung der Formel (143) bis (145), wobei für die verwendeten Symbole obige Definitionen gelten und wobei h und i unabhängig voneinander 0, 1, 2, 3 und 4 sein können und j und k unabhängig voneinander, 0, 1, 2, 3, 4 und 5 sein können.

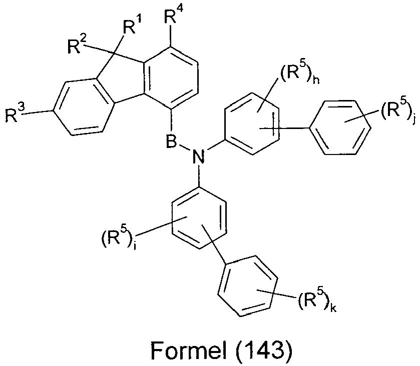

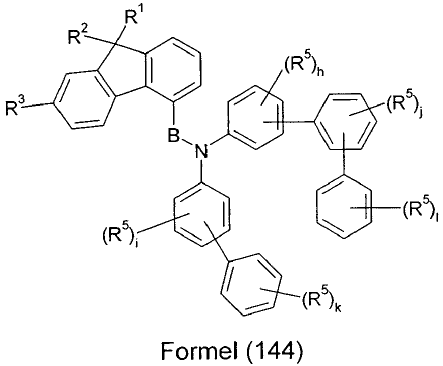

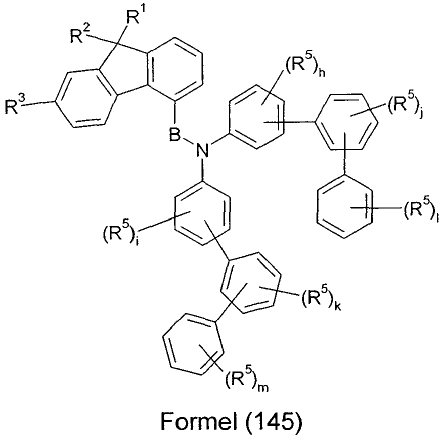

Noch mehr bevorzugt ist, wenn für die verwendeten Symbole in den Verbindungen der Formeln (143) bis (145) gilt:
R¹ und R²
   die verschieden voneinander sind, sind eine geradkettige Alkylgruppe mit 1 bis 20 C-Atomen oder eine verzweigte oder cyclische Alkylgruppe mit 3 bis 20 C-Atomen sind, wobei die oben genannten Gruppen jeweils mit einem oder mehreren Resten R⁶ substituiert sein können oder ein aromatisches oder heteroaromatisches Ringsystem mit 6 bis 30 aromatischen Ringatomen, das jeweils durch einen oder mehrere Reste R⁶ substituiert sein kann, wobei insbesondere bevorzugt ist, wenn einer der beiden Reste R¹ und R² eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁶ substituiert sein können, wobei weiterhin bevorzugt ist, wenn einer der beiden Reste R¹ und R² eine Methyl-, Ethyl-, n-/i-Propyl- oder n-/i-/t-Butylgruppe ist und der andere der beiden Reste R¹ und R² ein aromatisches oder heteroaromatisches Ringsystem mit 6 bis 30 aromatischen Ringatomen ist, das jeweils durch einen oder mehrere Reste R⁶ substituiert sein kann, wobei das Ringsystem insbesondere bevorzugt ausgewählt ist aus der Gruppe bestehend aus einer Phenyl-, Biphenyl-, Terphenyl- oder Pyridylgruppe;
R³
   ist H, D, F, Cl, Br, I, eine geradkettige Alkylgruppe mit 1 bis 20 C-Atomen oder eine verzweigte oder cyclische Alkylgruppe mit 3 bis 20 C-Atomen, wobei die oben genannten Gruppen jeweils mit einem oder mehreren Resten R⁶ substituiert sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, insbesondere bevorzugt ist R³ gleich H;
B
   ist eine Einfachbindung oder eine Phenylen-, Biphenylen-, Terphenylen-, Naphthylen-, Dibenzofuranylen- oder Dibenzothiophenylen Fluorenylen-, oder Carbazoylengruppe, die mit einem oder mehreren Resten R⁶ substituiert sein können, insbesondere bevorzugt ist B eine Einfachbindung;
und wobei R⁵ wie oben angegeben definiert ist, wobei insbesondere bevorzugt ist, wenn R⁵ gleich H ist.

Insbesondere bevorzugt ist auch eine elektrolumineszierende Vorrichtung enthaltend wenigstens eine Verbindung der Formel (146) bis (148), wobei für die verwendeten Symbole obige Definitionen gelten und wobei h und i unabhängig voneinander 0, 1, 2, 3 und 4 sein können und j und k unabhängig voneinander, 0, 1, 2, 3, 4 und 5 sein können.

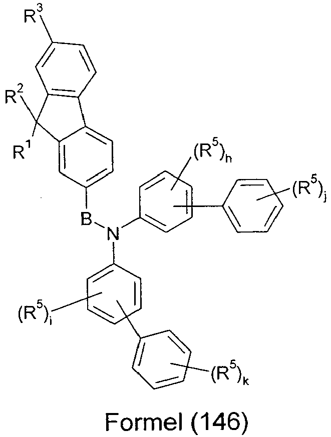

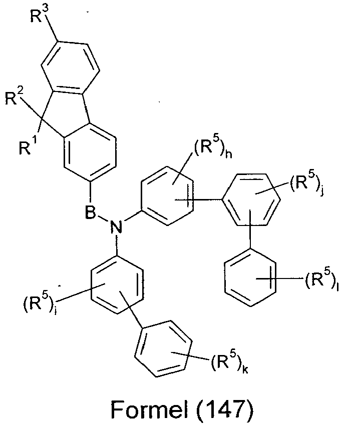

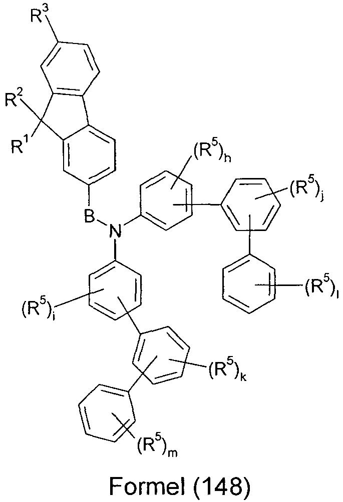

Noch mehr bevorzugt ist, wenn für die verwendeten Symbole in den Verbindungen der Formeln (146) bis (148) gilt:
R¹ und R²
   die verschieden voneinander sind, sind eine geradkettige Alkylgruppe mit 1 bis 20 C-Atomen oder eine verzweigte oder cyclische Alkylgruppe mit 3 bis 20 C-Atomen sind, wobei die oben genannten Gruppen jeweils mit einem oder mehreren Resten R⁶ substituiert sein können oder ein aromatisches oder heteroaromatisches Ringsystem mit 6 bis 30 aromatischen Ringatomen, das jeweils durch einen oder mehrere Reste R⁶ substituiert sein kann, wobei insbesondere bevorzugt ist, wenn einer der beiden Reste R¹ und R² eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁶ substituiert sein können, wobei weiterhin bevorzugt ist, wenn einer der beiden Reste R¹ und R² eine Methyl-, Ethyl-, n-/i-Propyl- oder n-/i-/t-Butylgruppe ist und der andere der beiden Reste R¹ und R² ein aromatisches oder heteroaromatisches Ringsystem mit 6 bis 30 aromatischen Ringatomen ist, das jeweils durch einen oder mehrere Reste R⁶ substituiert sein kann, wobei das Ringsystem insbesondere bevorzugt ausgewählt ist aus der Gruppe bestehend aus einer Phenyl-, Biphenyl-, Terphenyl- oder Pyridylgruppe;
R³
   ist H, D, F, Cl, Br, I, eine geradkettige Alkylgruppe mit 1 bis 20 C-Atomen oder eine verzweigte oder cyclische Alkylgruppe mit 3 bis 20 C-Atomen, wobei die oben genannten Gruppen jeweils mit einem oder mehreren Resten R⁶ substituiert sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, insbesondere bevorzugt ist R³ gleich H;
B
   ist eine Einfachbindung oder eine Phenylen-, Biphenylen-, Terphenylen-, Naphthylen-, Dibenzofuranylen- oder Dibenzothiophenylen Fluorenylen-, oder Carbazoylengruppe, die mit einem oder mehreren Resten R⁶ substituiert sein können, insbesondere bevorzugt ist B eine Einfachbindung;
und wobei R⁵ wie oben angegeben definiert ist, wobei insbesondere bevorzugt ist, wenn R⁵ gleich H ist.

In einer ganz besonders bevorzugten Ausführungsform betrifft die vorliegende Erfindung Verbindungen der allgemeinen Formel (1) dadurch charakterisiert, dass es sich um eine Monoamin- oder Diaminverbindung, insbesondere bevorzugt um eine Monoaminverbindung, handelt.

Die erfindungsgemäße Vorrichtung kann jede elektrolumineszierende Vorrichtung sein. Bevorzugt im Sinne der vorliegenden Erfindung handelt es sich bei der elektrolumineszierenden Vorrichtung um einen organischen lichtemittierenden Transistor (OLETs), eine organische Feld Quench-Vorrichtung (OFQDs), eine organische lichtemittierende elektrochemische Zellen (OLECs, LECs oder LEECs), eine organische Laserdiode (O-Laser) und organische lichtemittierende Diode (OLEDs). Ganz bevorzugt handelt es sich um eine organische lichtemittierende elektrochemische Zellen (OLECs, LECs oder LEECs) oder um eine organische lichtemittierende Diode (OLEDs). Ganz besonders bevorzugt handelt es sich bei der erfindungsgemäßen Elektrolumineszenzvorrichtung um eine organische lichtemittierende Diode (OLEDs).

Die Verbindungen der allgemeinen Formel (1) werden insbesondere in den folgenden Schichten und mit der folgenden Funktion in den elektrolumineszierenden Vorrichtungen eingesetzt:
- als Lochtransportmaterial in einer Lochtransport- oder Lochinjektionsschicht
- als Excitonenblockiermaterial,
- als Elektronenblockiermaterial,
- als Matrixmaterial in einer emittierenden Schicht,
- als Emittierendematerial in einer emittierende Schicht

Die Synthese der Verbindungen kann nach Verfahren hergestellt werden, die dem Fachmann aus dem Stand der Technik bekannt sind. Die Herstellung kann, beispielsweise, mittels Halogenierung, Buchwald-Kupplung und Suzuki-Kupplung erfolgen.

Das folgende Reaktionsschema zeigt einen bevorzugten Syntheseweg zur Herstellung der Verbindungen der Formel (1). Zur Synthese der Verbindungen wird an das Fluoren-Verbindung **A** in einer Buchwald-Kupplung mit einem Amin **B** der Formel Ar¹-NH-Ar² umgesetzt 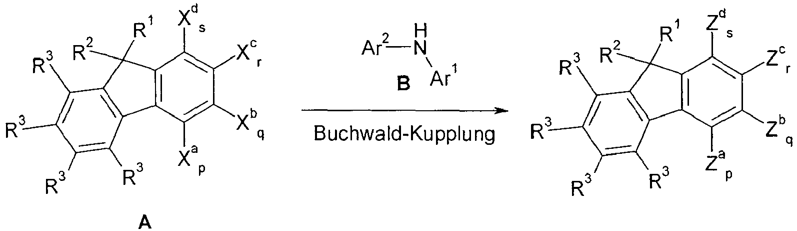 wobei für die verwendeten Symbole und Indices obige Definitionen gelten und wobei
X^{a}₀, X^{b}₀, X^{c}₀,X^{d}₀ gleich oder verschieden bei jedem Auftreten gleich R⁴ und
X^{a}₁, X^{b}₁, X^{c}₁, X^{d}₁ sind gleich -B-Y, wobei Y eine Abgangsgruppe, beispielsweise Halogen, sind.

Ein anderer bevorzugter Syntheseweg zur Herstellung der Verbindungen wird im folgenden Reaktionsschema dargestellt. Der Syntheseweg umfasst zwei Kupplungsreaktionen: zunächst wird das das Fluoren-Verbindung **A** in einer ersten Buchwald-Kupplung mit einem Amin **B** der Formel Ar¹-NH₂ umgesetzt. Schließlich erfolgt eine zweite Buchwald-Kupplung mit einer Verbindung D, beispielsweise mit einer Bromaryl-Verbindung. 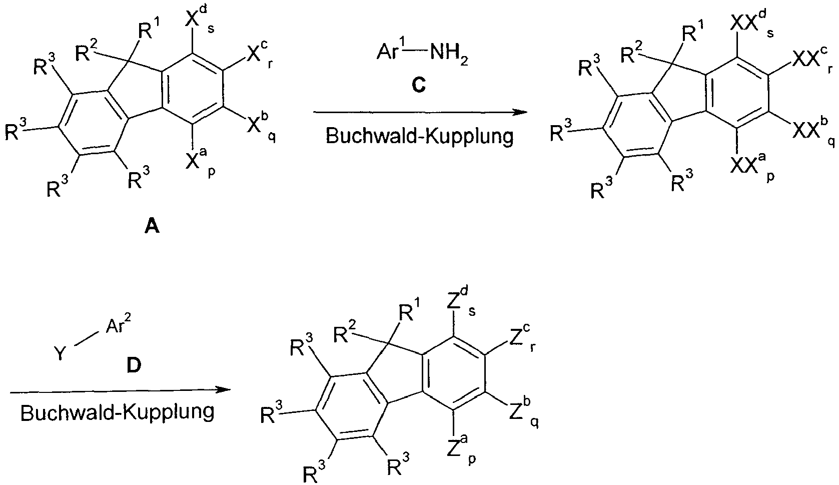 wobei Y wieder eine Abgangsgruppe, beispielsweise Halogen, ist;
und wobei
XX^{a}₀, XX^{b}₀,XX^{c}₀, XX^{d}₀ gleich oder verschieden bei jedem Auftreten gleich R³ und
XX^{a}₁, XX^{b}₁, XX^{c}₁, XX^{d}₁ gleich-B-NH-Ar¹ sind.

Synthesewege für die Ausgangsverbindungen **A, B**, **C** und **D,** welche in der Synthese der Verbindungen eingesetzt werden sind dem Fachmann geläufig. Weiterhin werden in den Ausführungsbeispielen einige explizite Syntheseverfahren im Detail dargestellt.

Bevorzugte Kupplungsreaktionen zur Herstellung der Verbindung der allgemeinen Formel (1) sind Buchwald-Kupplungen.

Bevorzugte Verbindungen für elektrolumineszierende Vorrichtungen sind in der nachfolgenden Tabelle exemplarisch aufgeführt. 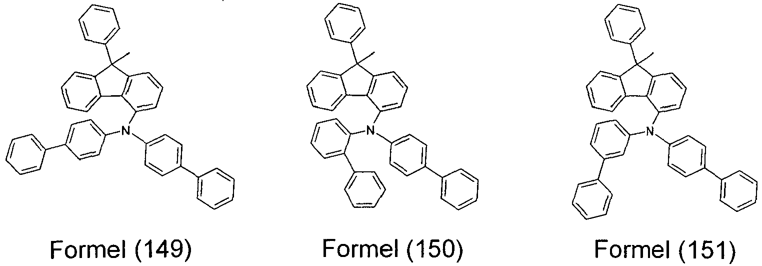 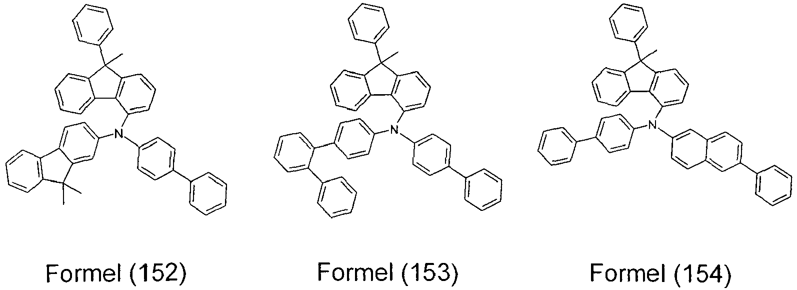 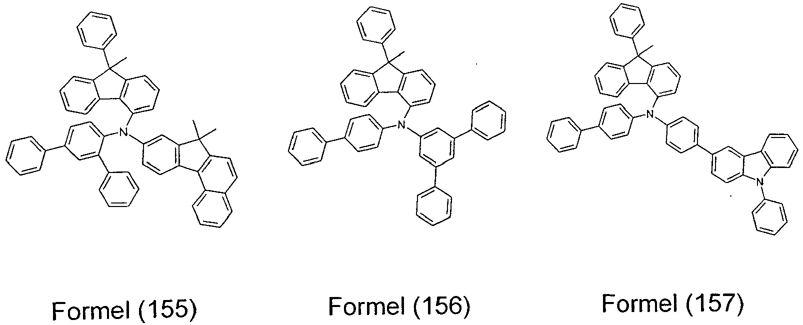 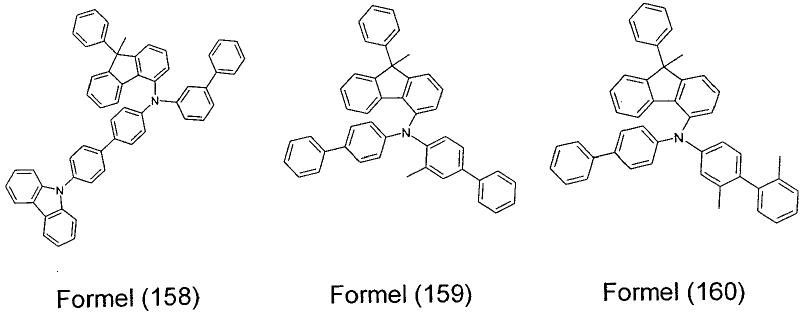 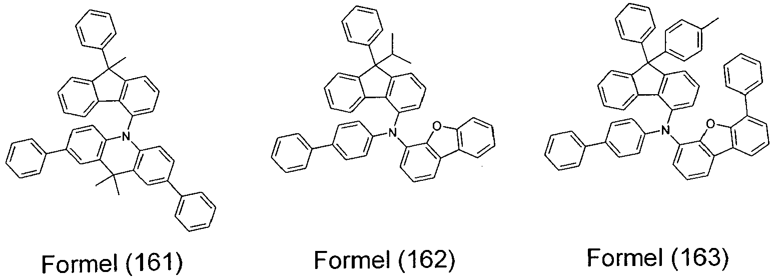 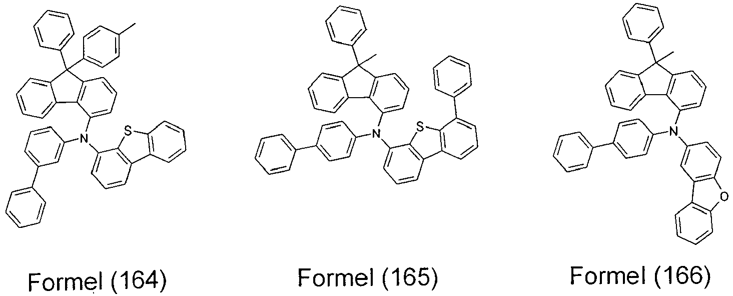 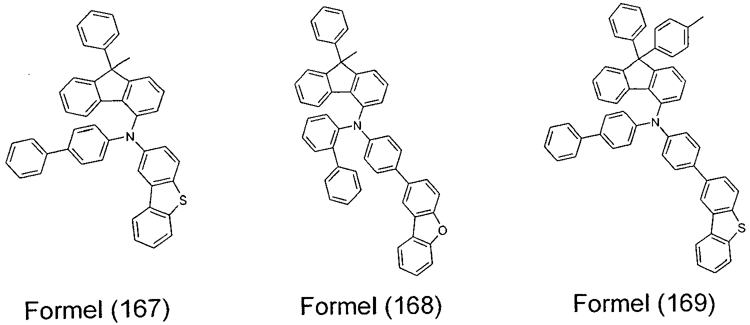 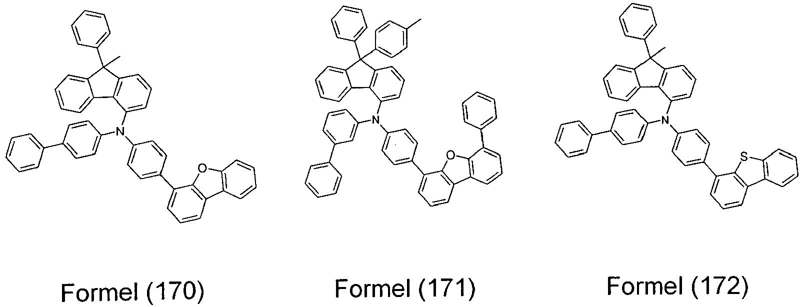 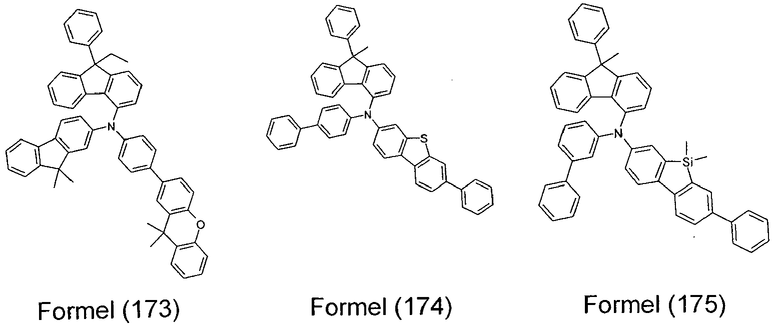 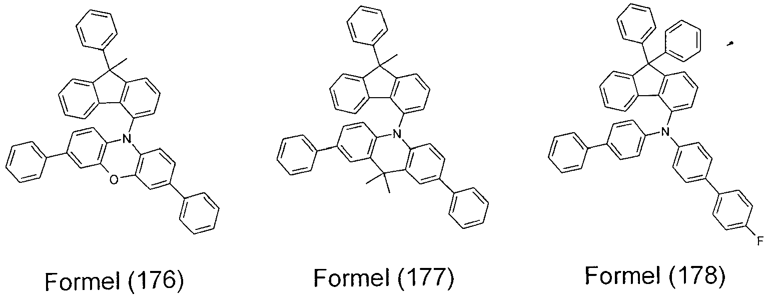 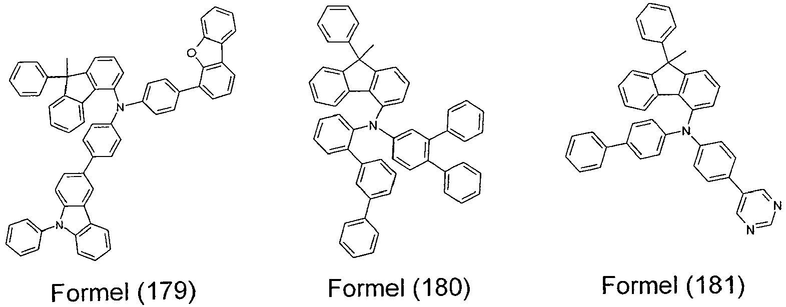 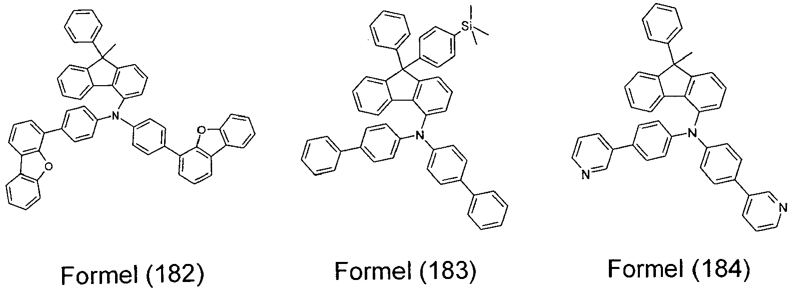 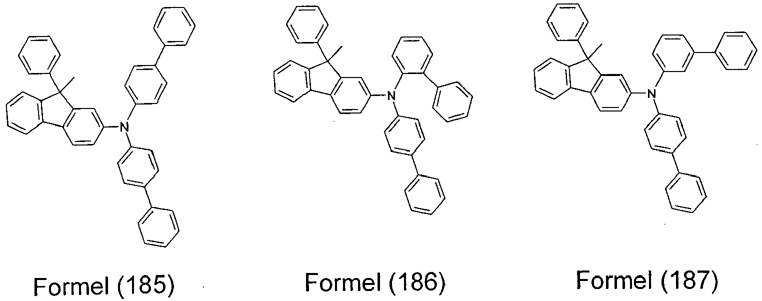 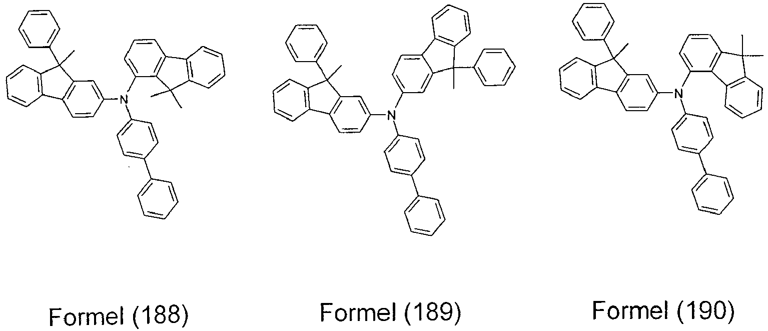 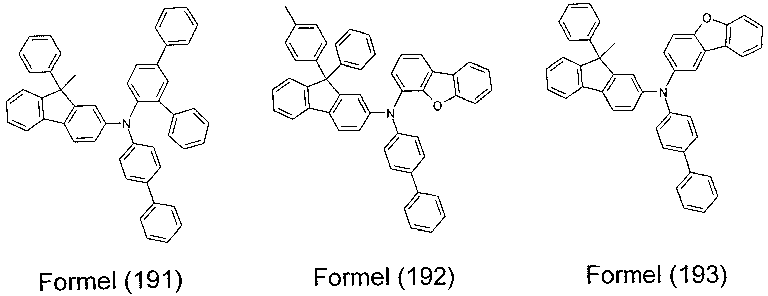 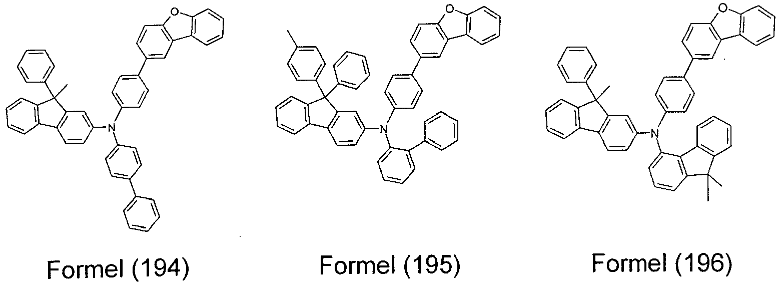 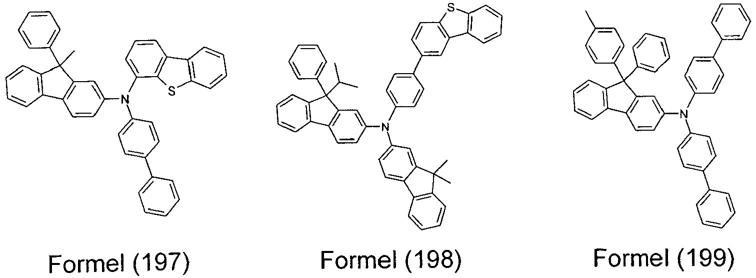 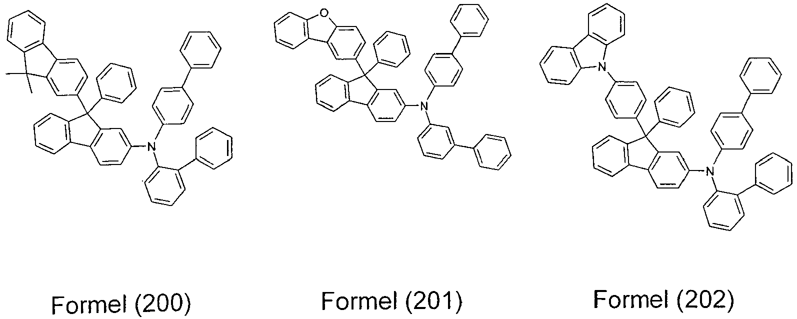 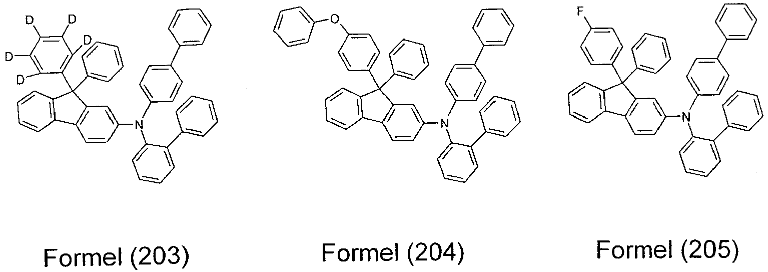 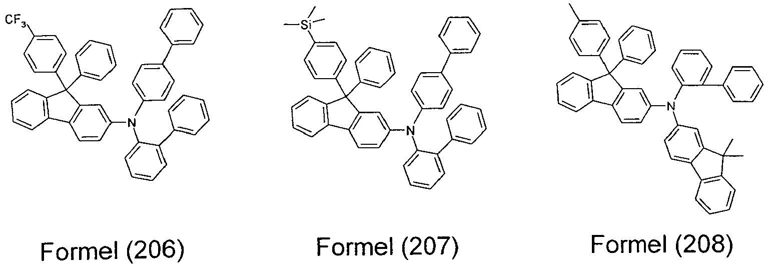 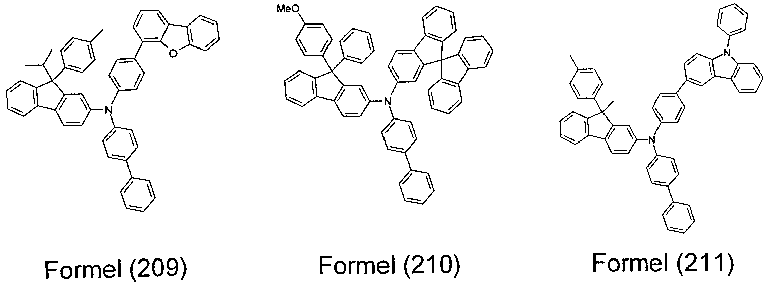 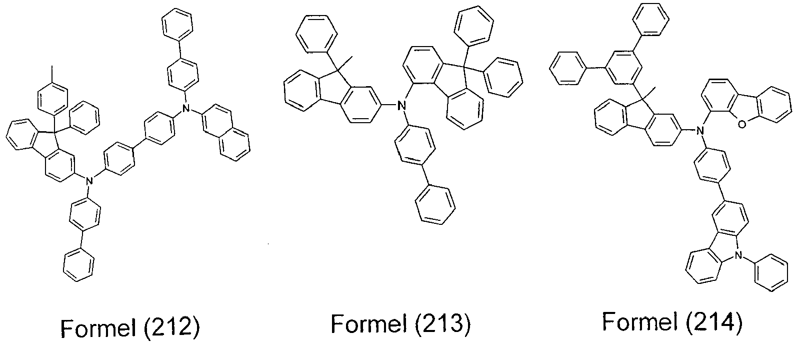 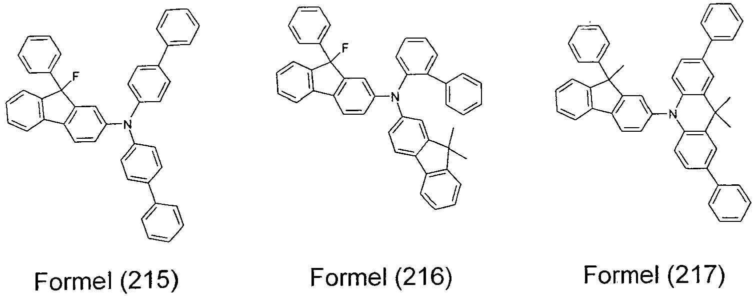 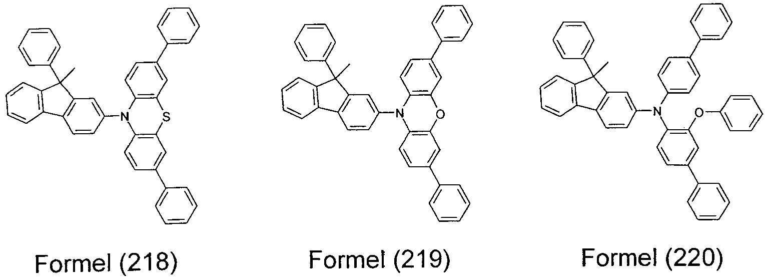 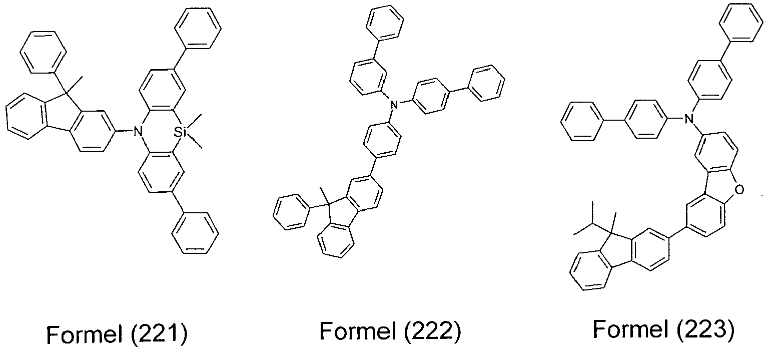 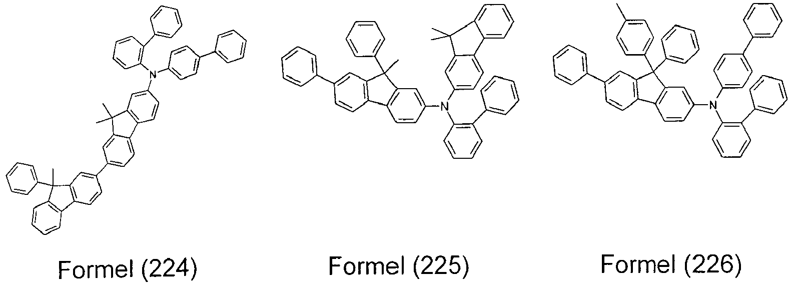 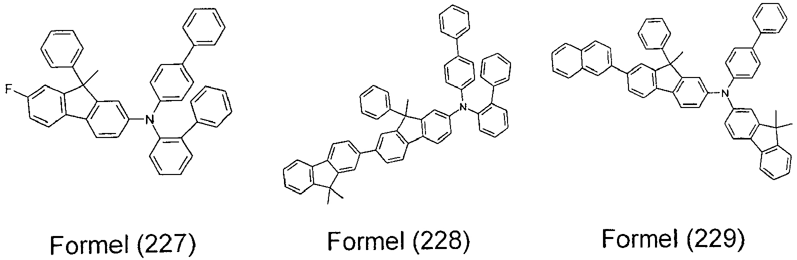 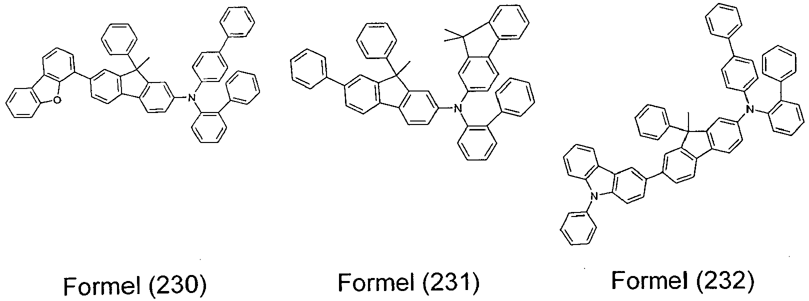 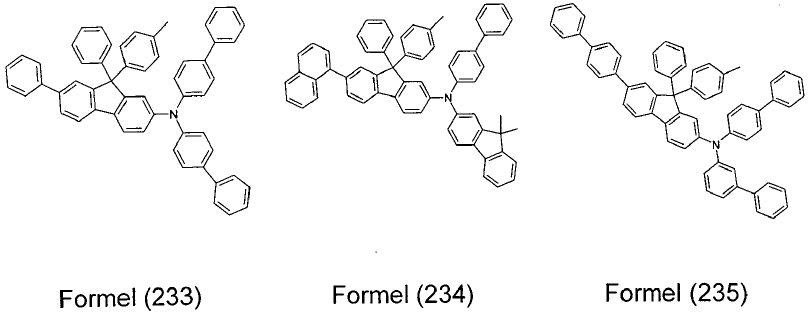 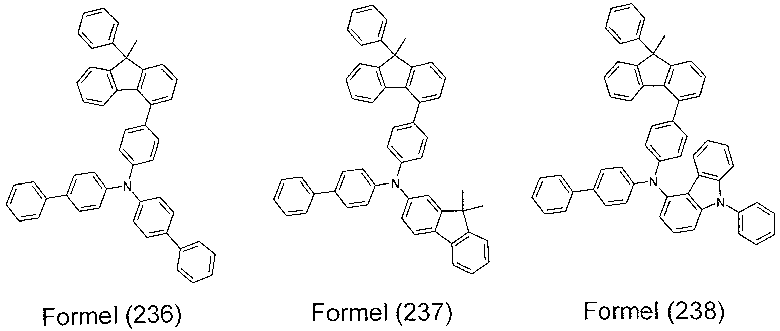 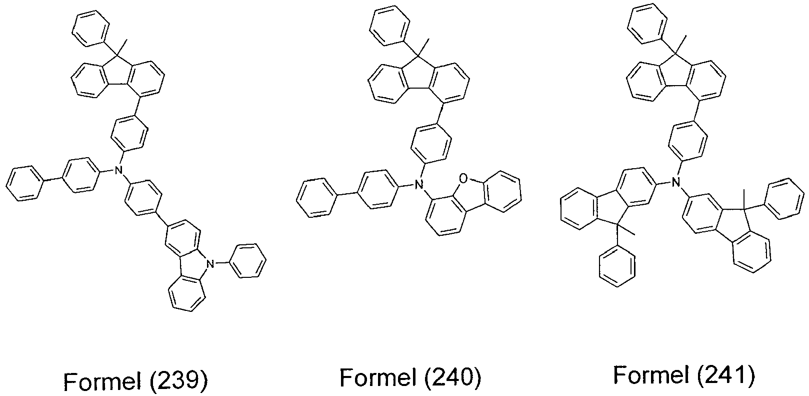 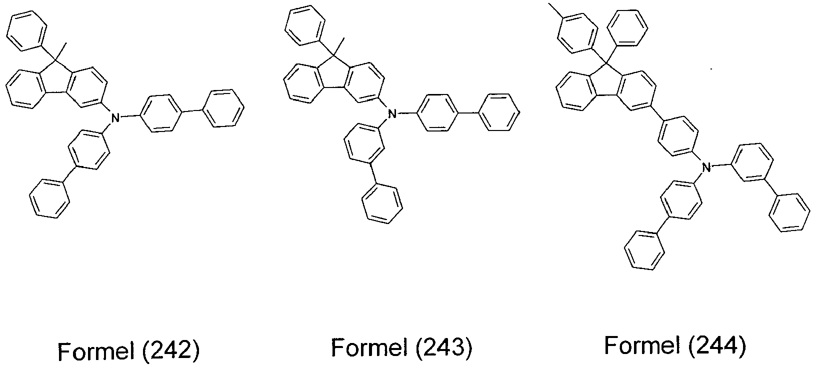 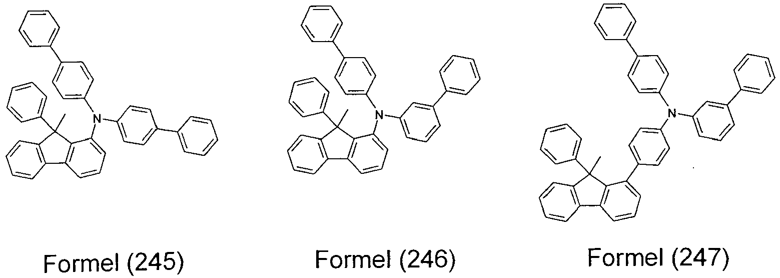 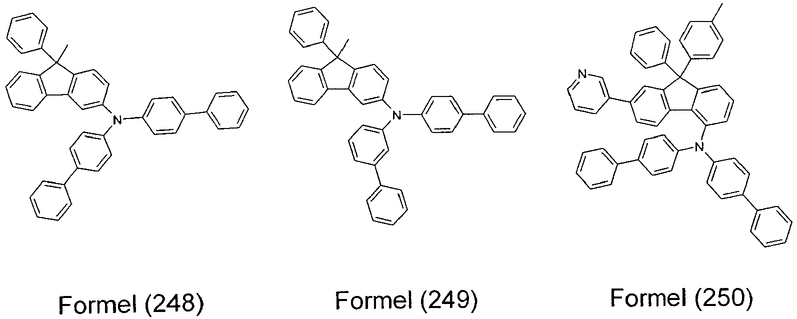 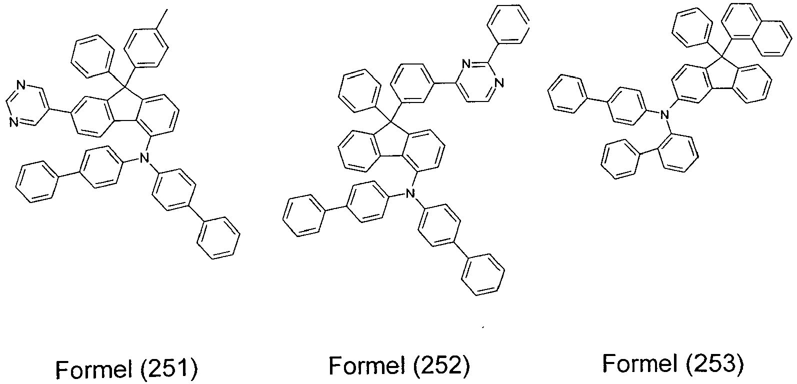 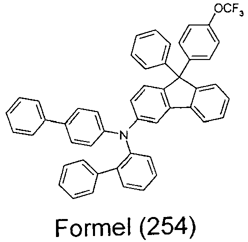 Gegenstand der vorliegenden Erfindung ist auch eine Verbindung der allgemeinen Formel (255) 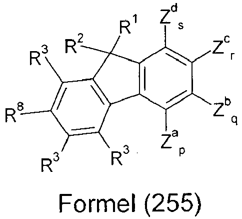 wobei für die verwendeten Symbole und Indices gilt:
p, q, r, s
   sind 0 oder 1, wobei p + q + r + s = 1 gilt, bevorzugt ist p =1 oder r = 1 oder s = 1, ganz bevorzugt ist p = 1 oder r = 1;
Z^{a}₀, Z^{b}₀, Z^{c}₀, Z^{d}₀
   sind gleich oder verschieden bei jedem Auftreten gleich R⁴
Z^{a}₁, Z^{b}₁, Z^{c}₋₁, Z^{d}₁ sind gleich 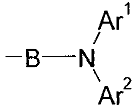
B ist eine Einfachbindung, eine bivalente Arylgruppe mit 6 bis 30 Ringatomen oder eine bivalente Heteroarylgruppe mit 5 bis 30 Ringatomen, die jeweils mit einem oder mehreren Resten R⁶ substituiert sein können,
   bevorzugt eine Einfachbindung oder eine Phenylen-, Biphenylen-, Terphenylen-, Naphthylen-, Pyridinylen-, Pyrimidinylen-, Pyrazinylen-, Pyridazinylen-, Triazinylen-, Dibenzofuranylen-, Dibenzothiophenylen- Fluorenylen-, oder Carbazoylengruppe, die mit einem oder mehreren Resten R⁶ substituiert sein können,
   ganz bevorzugt eine Einfachbindung oder eine Phenylen-, Biphenylen-, Terphenylen-, Naphthylen-, Dibenzofuranylen- oder Dibenzothiophenylen Fluorenylen-, oder Carbazoylengruppe, die mit einem oder mehreren Resten R⁶ substituiert sein können,
   ganz besonders bevorzugt ist B eine Einfachbindung oder eine Phenylengruppe, die mit einem oder mehreren Resten R⁶ substituiert sein kann,
   insbesondere bevorzugt ist B eine Einfachbindung,
   wobei, wenn B eine Einfachbindung ist, das Stickstoffatom direkt an das Fluoren gebunden ist;
Ar¹, Ar²
   sind bei jedem Auftreten gleich oder verschieden eine nicht kondensierte Arylgruppe mit 10 bis 60 Ringatomen oder eine Heteroarylgruppe 10 bis 60 Ringatomen, welche mit einem oder mehreren Resten R⁵, die gleich oder verschieden voneinander sind, substituiert sein können, wobei beide Gruppen Ar¹ oder Ar² jeweils mindestens zwei oder mehr aromatische oder heteroaromatische Ringe enthalten,
   wobei zwei der aromatischen oder heteroaromatischen Ringe in Ar¹ durch eine bivalente Gruppe -O-, -S-, -Si(R⁵)₂-, -C(R⁵)₂- oder-NR⁵- verbrückt sein können bzw. zwei der aromatischen oder heteroaromatischen Ringe in Ar² durch eine bivalente Gruppe -O-, - S-, -Si(R⁵)₂-, -C(R⁵)₂- oder -NR⁵- verbrückt sein können, wobei unverbrückte Ringe bevorzugt sind
   und wobei ein aromatischer oder heteroaromatischer Ring aus Ar¹ mit einem aromatischen oder heteroaromatischen Ring aus Ar² durch eine bivalente Gruppe -O-, -S-, -Si(R⁵)₂-, -NR⁵- oder -C(R⁵)₂-verbrückt sein kann, wobei unverbrückte Gruppen Ar¹ und Ar² bevorzugt sind;
R¹, R², R³ und R⁴
   sind H, D, F, Cl, Br, I, C(=O)R⁶, CN, Si(R⁶)₃, NO₂, N(R⁶)₂, P(=O)(R⁶)₂, S(=O)R⁶, S(=O)₂R⁶, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, oder eine Aryloxygruppe mit 5 bis 60 aromatischen Ringatomen, die mit einem oder mehreren Resten R⁶ substituiert sein kann, oder einer Aralkylgruppe mit 5 bis 60 aromatischen Ringatomen, die jeweils mit einem oder mehreren Resten R⁶ substituiert sein kann,
   wobei die Reste R¹ und R² nicht identisch sein dürfen und die Reste R³ bis R⁵ bei jedem Auftreten gleich oder verschieden sein können aber identisch mit entweder R¹ oder mit R² sein können
   und wobei wenigstens einer der Reste aus R¹ und R² ein aromatisches oder heteroaromatisches Ringsystem mit 6 bis 30 aromatischen Ringatomen, das jeweils durch einen oder mehrere Reste R⁶ substituiert sein kann, darstellt;
R⁵
   ist H, D, C(=O)R⁶, CN, Si(R⁶)₃, NO₂, N(R⁶)₂, P(=O)(R⁶)₂, S(=O)R⁶, S(=O)₂R⁶, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, oder eine Aryloxygruppe mit 5 bis 60 aromatischen Ringatomen, die mit einem oder mehreren Resten R⁶ substituiert sein kann, oder einer Aralkylgruppe mit 5 bis 60 aromatischen Ringatomen, die jeweils mit einem oder mehreren Resten R⁶ substituiert sein kann,
R⁶ ist bei jedem Auftreten gleich oder verschieden H, D, F, Cl, Br, I, C(=O)R⁷, CN, Si(R⁷)₃, NO₂. P(=O)(R⁷)₂, S(=O)R⁷, S(=O)₂R⁷, N(R⁷)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁷ substituiert sein können und wobei eine oder mehrere CH₂-Gruppen in den oben genannten Gruppen durch -R⁷C=CR⁷-, -C=C-, Si(R⁷)₂, C=O, C=S, C=NR⁷, -C(=O)O-, -C(=O)NR⁷-, P(=O)(R⁷), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁷ substituiert sein kann, oder eine Aryloxy- oder Heteroaryloxygruppe mit 5 bis 30 aromatischen Ringatomen, die durch einen oder mehrere Reste R⁷ substituiert sein kann, wobei zwei oder mehr benachbarte Substituenten R⁶ miteinander ein mono- oder polycyclisches Ringsystem bilden können;
R⁷ ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R⁷ miteinander ein mono- oder polycyclisches Ringsystem bilden können;
R⁸
   ist H, D, C(=O)R⁹, CN, Si(R⁹)₃, NO₂, N(R⁹)₂, P(=O)(R⁹)₂, S(=O)R⁹, S(=O)₂R⁹, eine geradkettige Alkyl- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁹ substituiert sein können und wobei eine oder mehrere CH₂-Gruppen in den oben genannten Gruppen durch -R⁹C=CR⁹-, -C≡C-, Si(R⁹)₂, C=O, C=S, C=NR⁹, -C(=O)O-, -C(=O)NR⁹-, P(=O)(R⁹), -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁹ substituiert sein kann, oder eine Aryloxygruppe mit 5 bis 60 aromatischen Ringatomen, die mit einem oder mehreren Resten R⁹ substituiert sein kann, oder einer Aralkylgruppe mit 5 bis 60 aromatischen Ringatomen, die jeweils mit einem oder mehreren Resten R⁹ substituiert sein kann;
R⁹ ist bei jedem Auftreten gleich oder verschieden H, D, F, Cl, Br, I, C(=O)R¹⁰, CN, Si(R¹⁰)₃, NO₂, P(=O)(R¹⁰)₂, S(=O)R¹⁰, S(=O)₂R¹⁰, N(R¹⁰)₂, eine geradkettige Alkyl- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R¹⁰ substituiert sein können und wobei eine oder mehrere CH₂-Gruppen in den oben genannten Gruppen durch -R¹⁰C=CR¹⁰-, -C≡C-, Si(R¹⁰)₂, C=O, C=S, C=NR¹⁰, -C(=O)O-, -C(=O)NR¹⁰-, P(=O)(R¹⁰), -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R¹⁰ substituiert sein kann, oder eine Aryloxy- oder Heteroaryloxygruppe mit 5 bis 30 aromatischen Ringatomen, die durch einen oder mehrere Reste R¹⁰ substituiert sein kann, wobei zwei oder mehr benachbarte Substituenten R¹⁰ miteinander ein mono- oder polycyclisches Ringsystem bilden können;
R¹⁰ ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R¹⁰ miteinander ein mono- oder polycyclisches Ringsystem bilden können.

In einer bevorzugten Ausführungsform betrifft die vorliegende Erfindung eine Verbindung der allgemeinen Formel (255), dadurch charakterisiert, dass die Verbindung keine kondensierten aromatischen oder heteroaromatischen Ringsysteme mit mehr als 10 Ringatomen enthält.

Bevorzugt ist eine Verbindung der allgemeinen Formel (256) 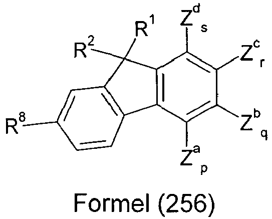 wobei für die verwendeten Symbole obige Definitionen gelten.

Ganz bevorzugt ist eine Verbindung der allgemeinen Formeln (257) bis (260)

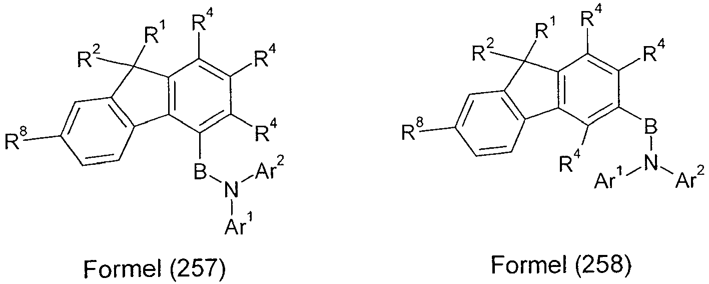

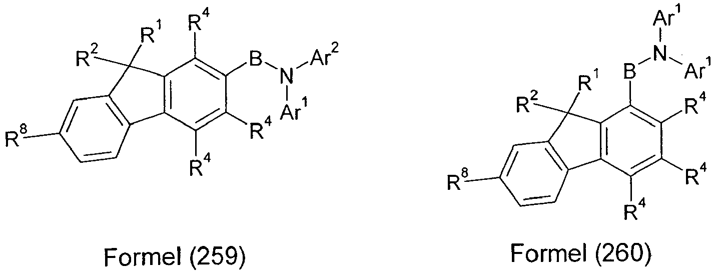

Ganz besonders bevorzugt ist eine Verbindung der allgemeinen Formeln (261) bis (264), wobei die der Formeln (261) und (263) insbesondere bevorzugt ist.

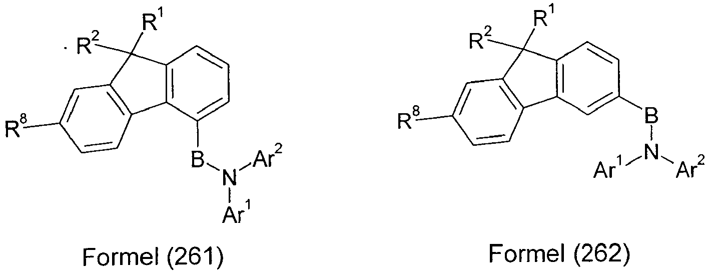

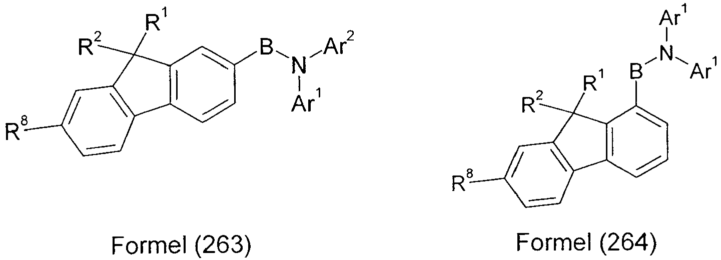

Für B und bevorzugte Ausführungsformen von B gelten die obigen Definitionen.

Weiterhin ganz besonders bevorzugt ist eine Verbindung der allgemeinen Formeln (265) bis (268), wobei die der Formeln (265) und (267) insbesondere bevorzugt ist.

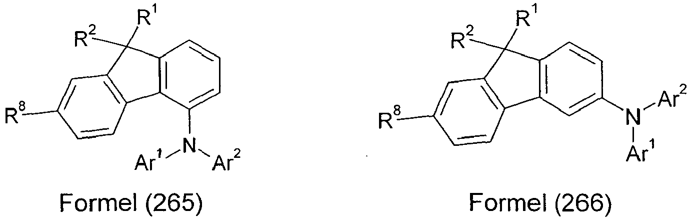

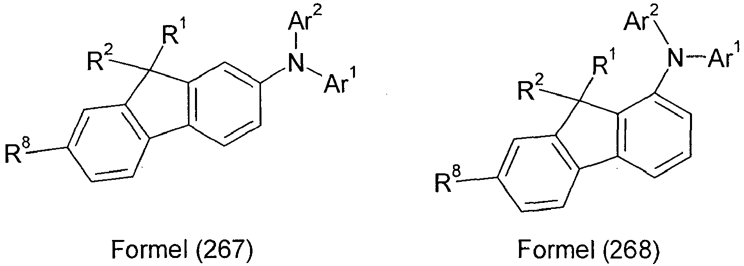

Bevorzugt sind Ar¹ und Ar² sind, gleich oder verschieden bei jedem Auftreten, bevorzugt ausgewählt aus einer Phenyl-Pyridyl-, Biphenyl-, Terphenyl- oder Quarterphenylgruppe, die mit einem oder mehreren Resten R⁶, die gleich oder verschieden voneinander sein können, substituiert sein können, wobei zwei der aromatischen oder heteroaromatischen Ringe in Ar¹ durch eine bivalente Gruppe -O-, -S-, -Si(R⁵)₂-,-C(R⁵)₂- oder NR⁵ verbrückt sein können bzw. zwei der aromatischen oder heteroaromatischen Ringe in Ar² durch eine bivalente Gruppe -O-, -S-, Si(R⁵)₂-, -C(R⁵)₂- oder NR⁵ verbrückt sein können, wobei unverbrückte Ringe bevorzugt sind, und wobei ein aromatischer oder heteroaromatischer Ring aus Ar¹ mit einem aromatischen oder heteroaromatischen Ring aus Ar² durch eine bivalente Gruppe -O-, -S-, -Si(R⁵)₂-, -NR⁵- oder - C(R⁵)₂- verbrückt sein kann, wobei unverbrückte Gruppen Ar¹ und Ar² bevorzugt sind.

Die vorliegende Erfindung betrifft weiterhin ein Verfahren zur Herstellung der Verbindung gemäß Formel (255), wobei, wie weiter oben beschrieben, die einstufige oder zweistufige Buchwald Kupplung verwendet wird.

Bevorzugte Beispiele für erfindungsgemäße Verbindungen sind die mit den Formeln (149) bis (153), (155) bis (177), (179) bis (254).

Die oben beschriebenen Verbindungen der Formel (1) sowie die erfindungsgemäßen Verbindungen nach Formel (255) können mit reaktiven Abgangsgruppen, wie Brom, Iod, Chlor, Boronsäure oder Boronsäureester substituiert werden. Diese können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formeln (1) oder (255), wobei die Bindung(en) zum Polymer, Oligomer oder Dendrimer an beliebigen in den Formeln (1) oder (255) möglichen Positionen lokalisiert sein können. Je nach Verknüpfung der Verbindung gemäß Formel (1) oder (255) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß den Formeln (1) oder (255) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß den Formeln (1) oder (255) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (1) oder (255) in Oligomeren, Dendrimeren und Polymeren gelten dieselben Bevorzugungen wie oben für Verbindungen gemäß den Formeln (1) oder (255) beschrieben.

Zur Herstellung der Oligomere oder Polymere werden die entsprechenden Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (1) oder (255) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Die erfindungsgemäßen Verbindungen, Polymere, Oligomere und Dendrimere können mit anderen organisch funktionellen Materialien, die in elektronischen Vorrichtungen verwendet werden, als Zusammensetzungen eingesetzt werden. Dem Fachmann ist hierbei eine Vielzahl möglicher organisch funktioneller Materialien aus dem Stand der Technik bekannt. Die vorliegende Erfindung betrifft daher auch eine Zusammensetzung enthaltend eine oder mehrere erfindungsgemäße Verbindungen der Formel (255) oder wenigstes ein erfindungsgemäßes Polymer, Oligomer oder Dendrimer und wenigstens ein weiteres organisch funktionelles Material ausgewählt aus der Gruppe bestehend aus fluoreszierenden Emitter, phosphoreszierenden Emitter, Host Materialien, Matrix Materialien, Elektronentransportmaterialien, Elektroneninjektionsmaterialien, Lochleitermaterialien, Lochinjektionsmaterialien, Elektronenblockiermaterialien und Lochblockiermaterialien.

Für die Verarbeitung der Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Gegenstand der Erfindung ist daher weiterhin eine Formulierung, insbesondere eine Lösung, Dispersion oder Miniemulsion, enthaltend mindestens eine erfindungsgemäße Verbindung, Polymer, Oligomer oder Dendrimer enthaltend mindestens eine Einheit gemäß Formel (1) oder (255) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (z.B. OLEDs oder OLECs). Abhängig von der Substitution werden die Verbindungen in unterschiedlichen Funktionen und Schichten eingesetzt.

Ein weiterer Gegenstand der vorliegenden Erfindung ist daher die Verwendung der Verbindung gemäß Formel (255) in elektronischen Vorrichtungen sowie elektronische Vorrichtungen selbst, welche eine oder mehrere Verbindungen gemäß Formel (255) enthalten. Dabei sind die elektronischen Vorrichtungen bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs und OLECs).

Gegenstand der Erfindung sind, wie bereits oben ausgeführt, elektronische Vorrichtungen, enthaltend mindestens eine Verbindung gemäß Formel (255). Dabei sind die elektronischen Vorrichtungen bevorzugt ausgewählt aus den oben genannten Vorrichtungen. Besonders bevorzugt sind organische Elektrolumineszenzvorrichtungen (OLEDs), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (255) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen können in solchen Vorrichtungen in einer Lochtransportschicht, einer emittierenden Schicht und/oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (1) oder (255) in einer organischen elektrolumineszierneden Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht oder in einer emittierenden Schicht, verwendet werden. Die Verbindung gemäß Formel (255) kann aber auch erfindungsgemäß in einer elektronischen Vorrichtung enthaltend einen oder mehrere fluoreszierende Dotanden eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (1) oder (255) in organischen Elektrolumineszenzvorrichtungen einsetzen.

Explizite Beispiele für geeignete phosphoreszierende Emitterverbindungen können weiterhin der folgenden Tabelle entnommen werden.

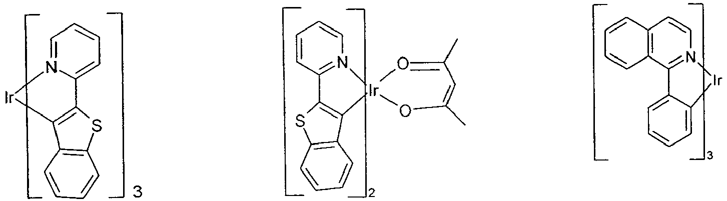

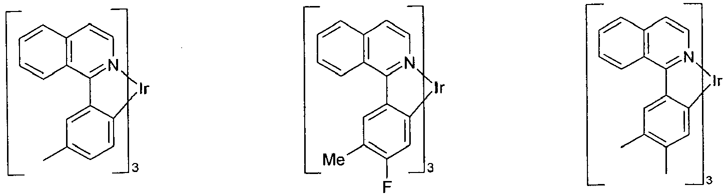

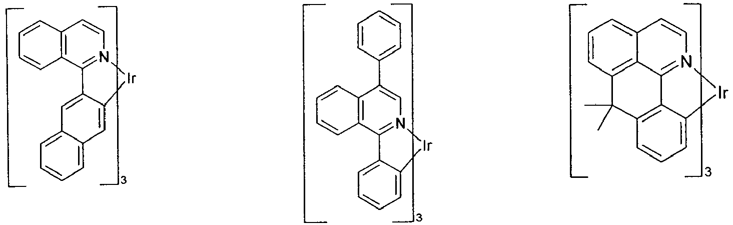

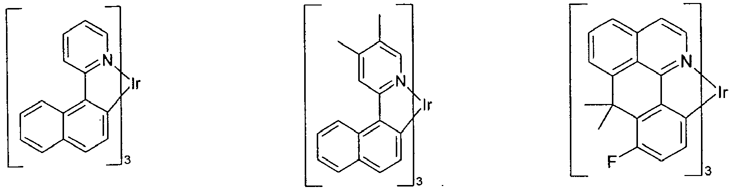

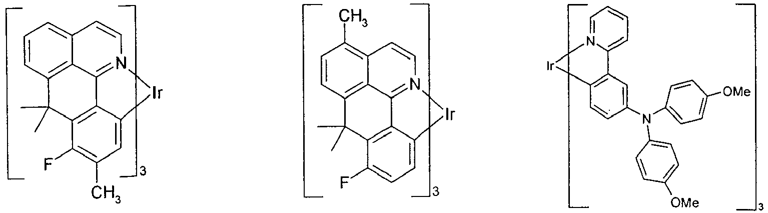

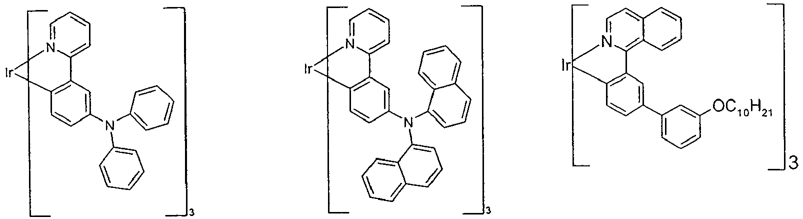

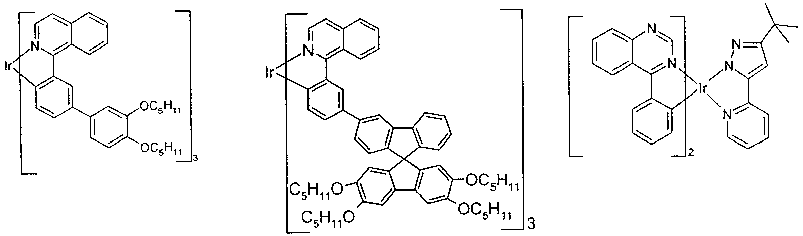

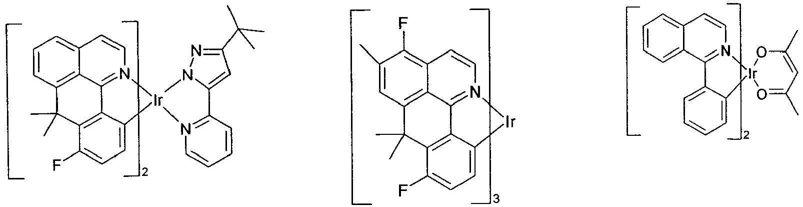

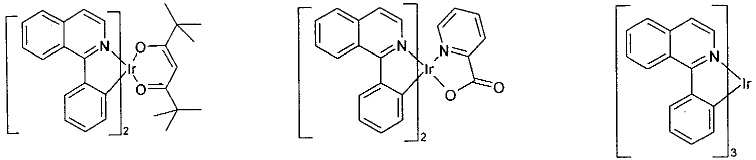

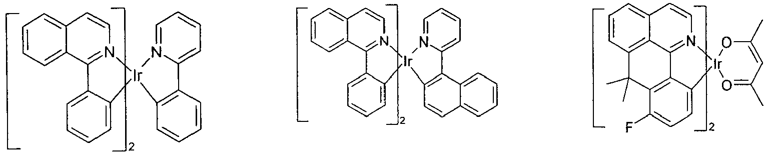

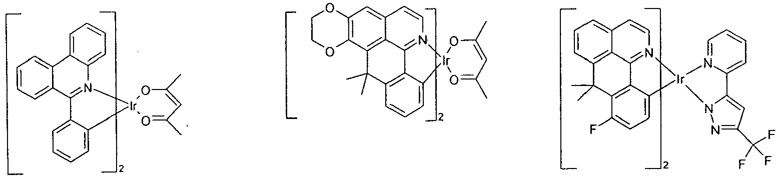

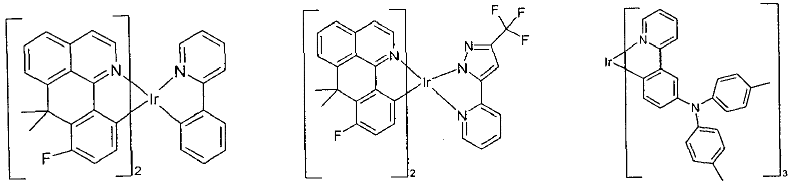

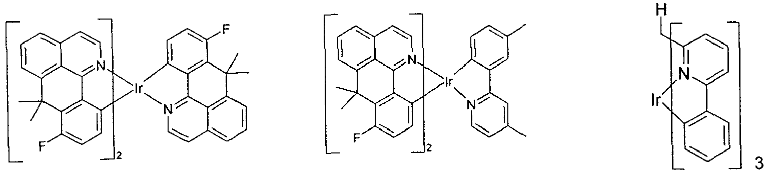

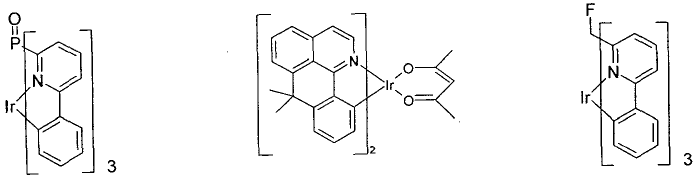

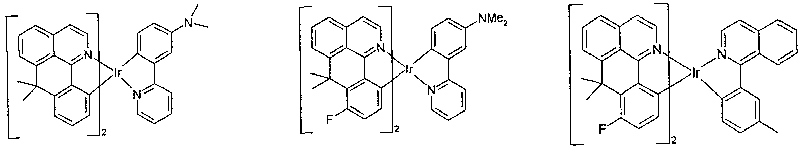

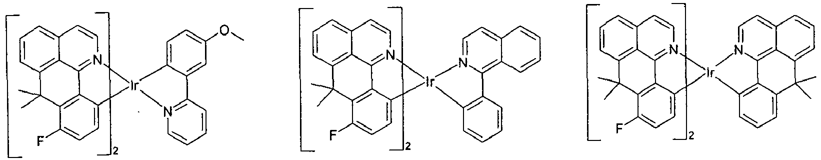

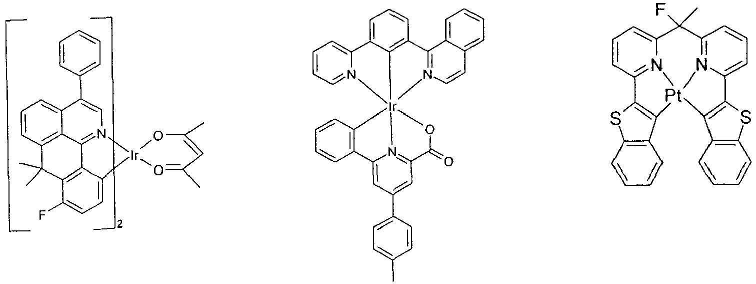

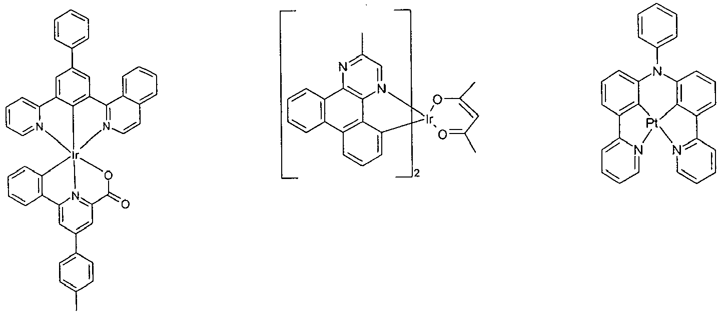

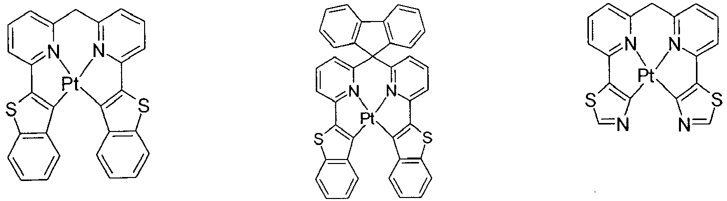

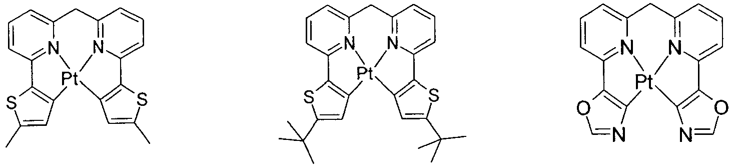

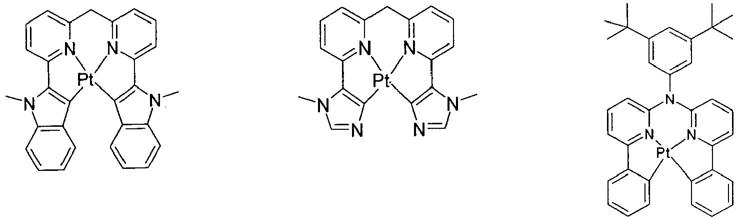

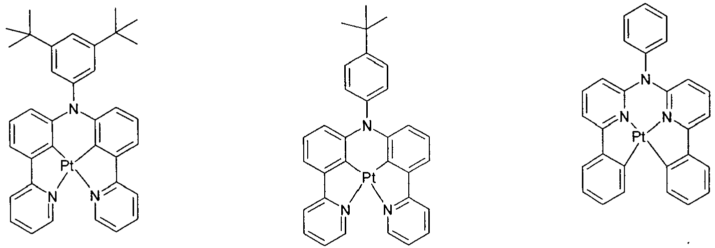

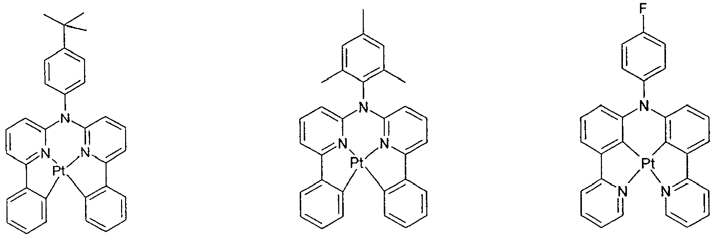

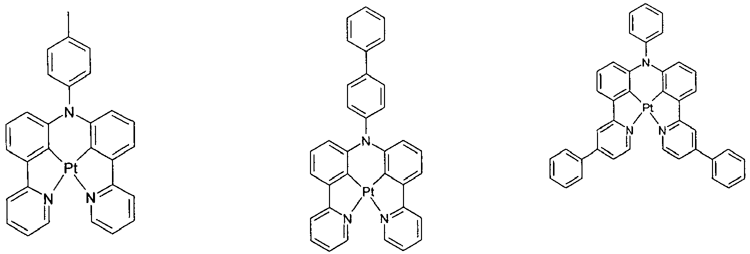

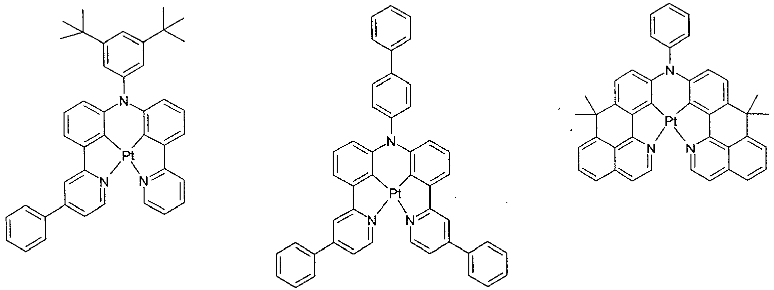

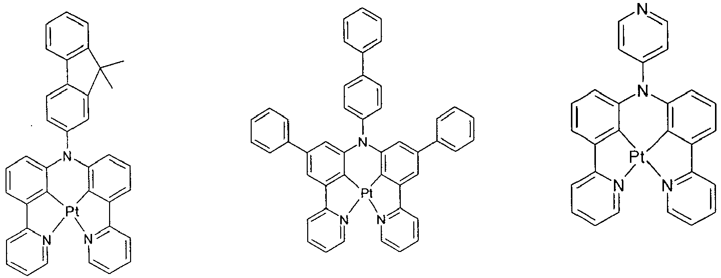

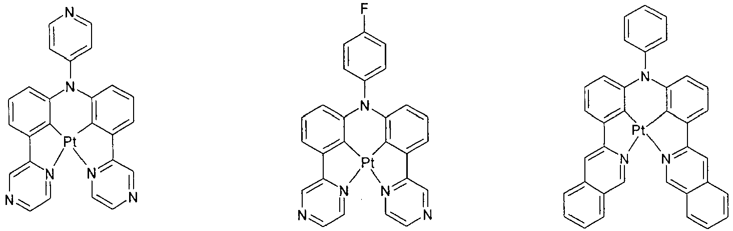

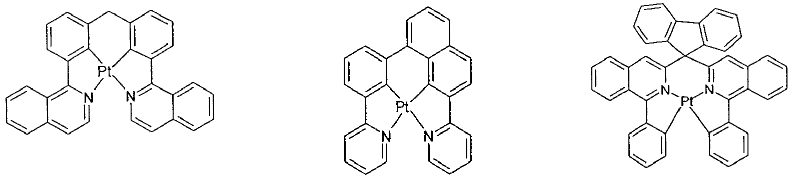

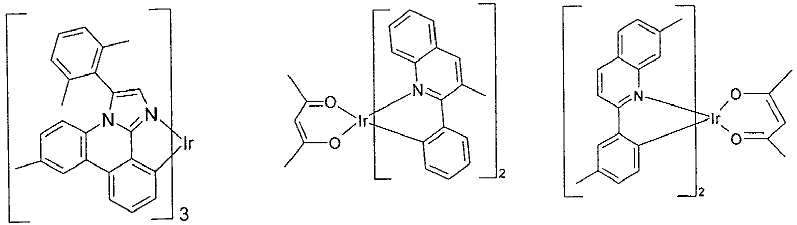

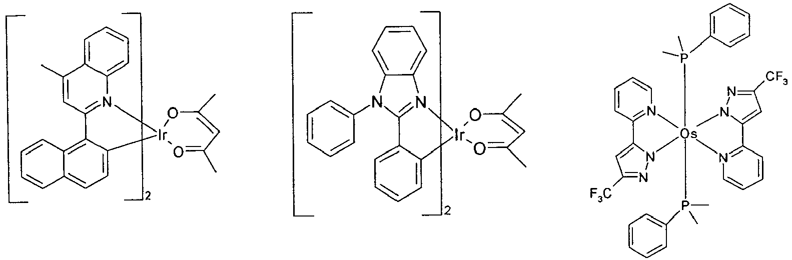

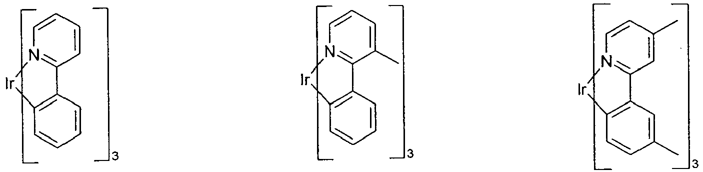

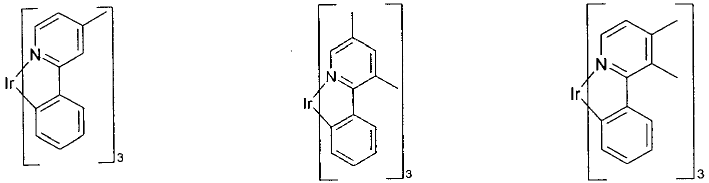

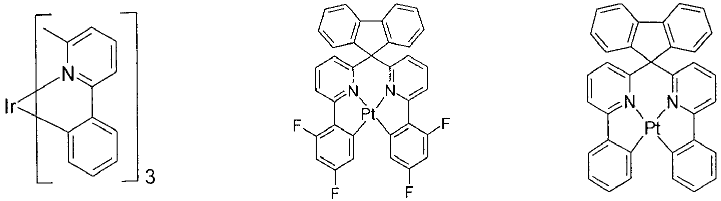

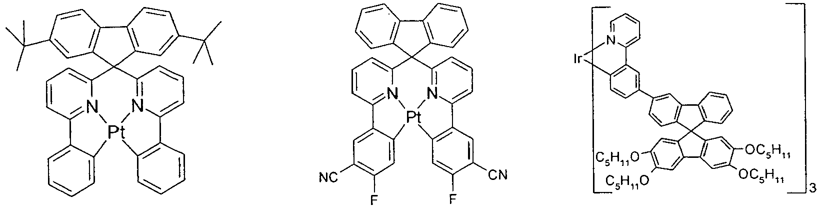

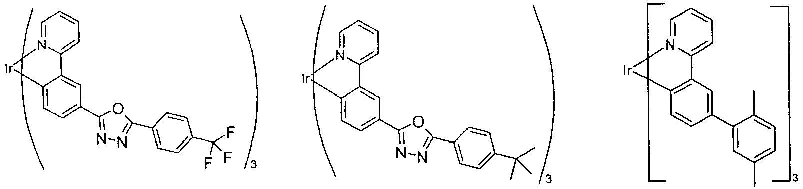

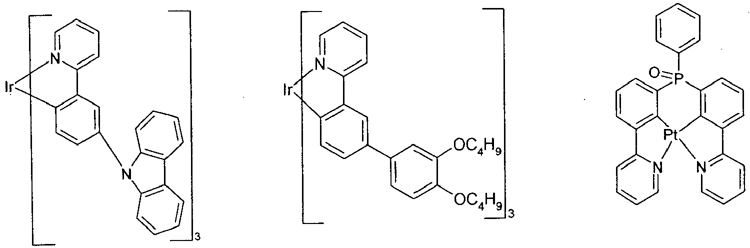

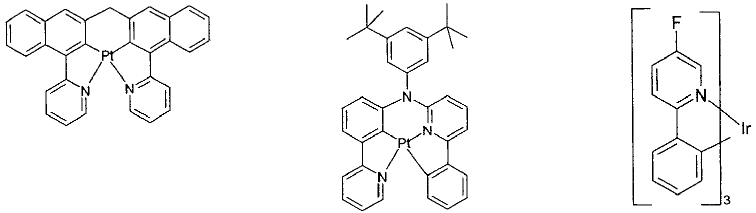

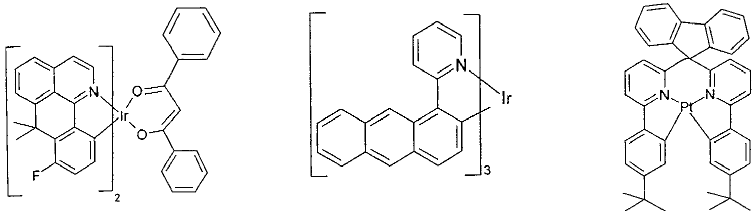

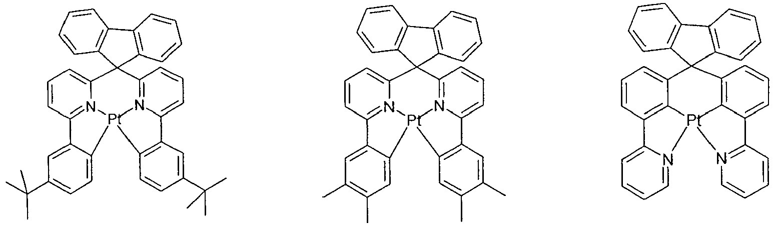

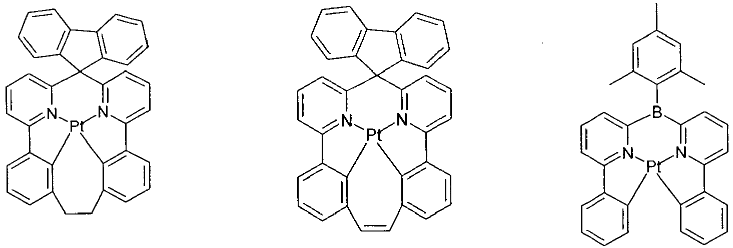

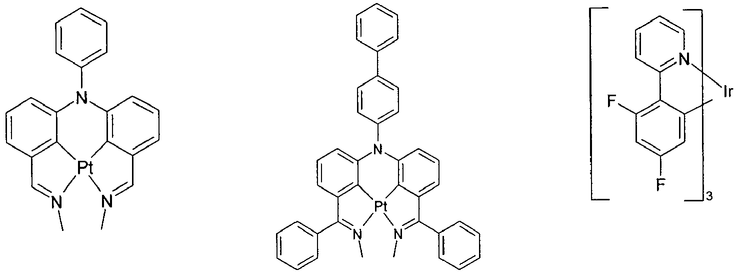

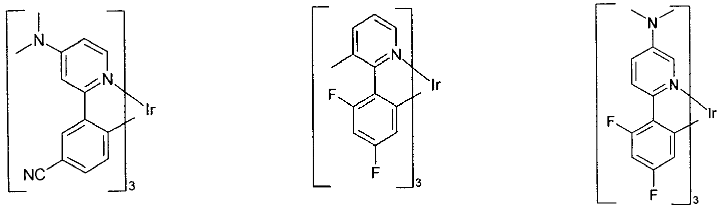

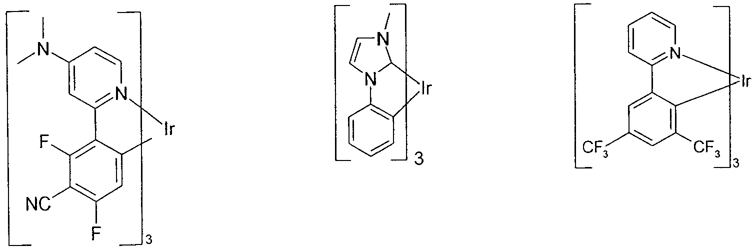

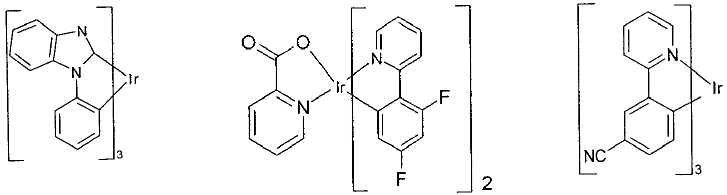

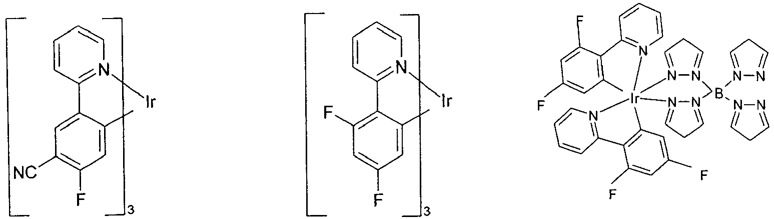

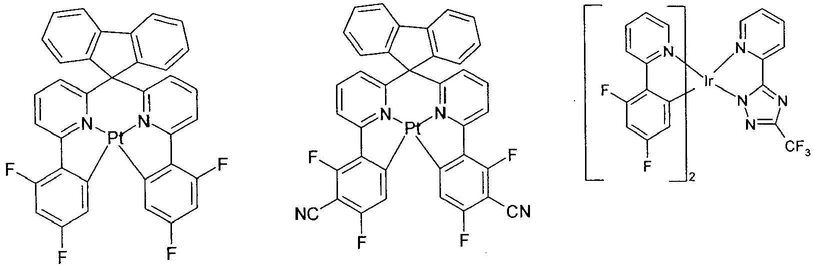

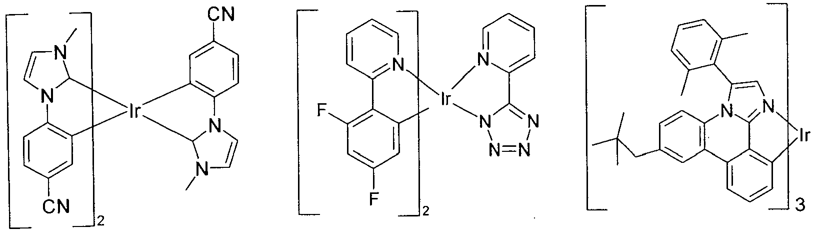

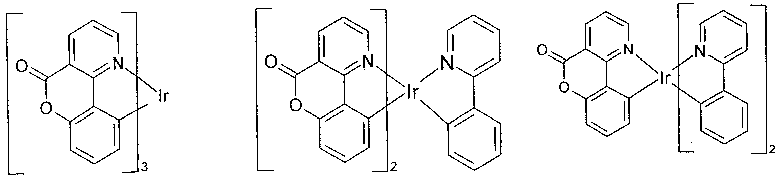

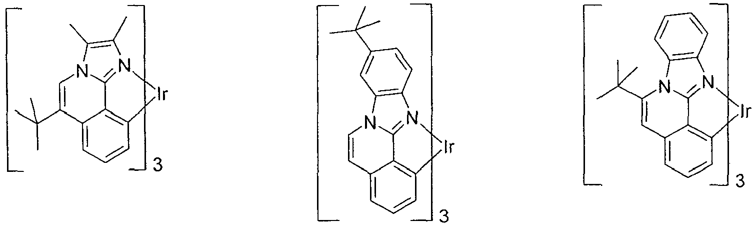

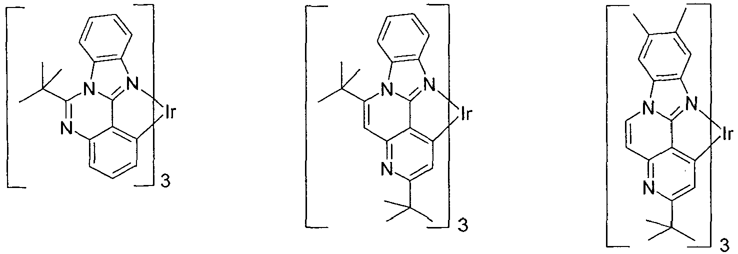

In einer bevorzugten Ausführungsform der Erfindung werden die Verbindungen gemäß der allgemeinen Formel (1) oder (255) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionschicht angrenzen. Wenn die Verbindungen gemäß Formel (1) oder (255) als Lochtransportmaterial oder als Lochinjektionsmaterial verwendet werden, kann es bevorzugt sein, wenn sie mit Elektronenakzeptor-Verbindungen dotiert sind (p-Doping), beispielsweise mit F₄-TCNQ, F₆-TNAP oder mit Verbindungen, wie sie in EP 1476881 oder EP 1596445 beschrieben werden. In einer weiteren bevorzugten Ausführungsform der Erfindung wird eine Verbindung gemäß Formel (1) oder (255) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Werden die Verbindungen gemäß der allgemeinen Formel (1) oder (255) als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In einer weiteren Ausführungsform der vorliegenden Erfindung werden die Verbindungen der allgemeinen Formeln (1) oder (255) als emittierende Materialien eingesetzt. Die Verbindungen werden hierzu bevorzugt in einer Emissionsschicht eingesetzt. Die Emissionsschicht enthält neben wenigstens einer der Verbindungen nach der allgemeinen Formel (1) oder (255) weiterhin wenigstens ein Host Material. Dabei kann der Fachmann aus den bekannten Hostmaterialien ohne Schwierigkeiten und ohne erfinderisch zu sein auswählen.

In einer weiteren Ausführungsform der vorliegenden Erfindung werden die Verbindungen der allgemeinen Formel (1) oder (255) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß der allgemeinen Formel (1) oder (255) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen, bevorzugt einen oder mehrere phosphoreszierende Dotanden. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die in der obenstehenden Tabelle aufgeführten phosphoreszierenden Dotanden.

Im Folgenden werden die in den erfindungsgemäßen Vorrichtungen in den betreffenden Funktionen bevorzugt eingesetzten Materialien aufgeführt.

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Als Matrixmaterialien, bevorzugt für fluoreszierende Dotanden, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAIQ.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) oder (255) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß der allgemeinen Formel (1) oder (255) in Displays, als Lichtquellen in Beleuchtungsanwendungen sowie als Lichtquellen in medizinischen und/oder kosmetischen Anwendungen (z.B. Lichttherapie) eingesetzt werden.

Vorrichtungen enthaltend die Verbindungen der allgemeinen Formel (1) oder (255) können sehr vielseitig eingesetzt werden. So können, beispielsweise, elektrolumineszierende Vorrichtuungen enthaltend eine oder mehrere Verbindungen nach der allgemeinen Formel (1) oder (255) in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen, z. B. in OLEDs oder OLECs, enthaltend wenigstens eine der Verbindung nach der allgemeinen Formel (1) oder (255) in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Lochtransportschicht oder einer Lochinjektionsschicht in elektronischen Vorrichtungen, wie beispielsweise in organischen Elektrolumineszenzvorrichtungen, insbesondere aufgrund ihrer hohen Lochbeweglichkeit.
2. Die erfindungsgemäßen Verbindungen weisen eine relativ niedrige Sublimationstemperatur, eine hohe Temperaturstabilität sowie eine hohe Oxidationsstabilität, eine hohe Glasübergangtemperatur und eine geringe Kristallinität auf, was sowohl für die Prozessierbarkeit, beispielweise aus Lösung oder aus der Gasphase, als auch für die Verbendung in elektronischen Vorrichtungen vorteilhaft ist.
3. Die Verwendung der erfindungsgemäßen Verbindungen in elektronischen Vorrichtungen, insbesondere eingesetzt als Lochtransport- oder Lochinjektionsmaterial führt zu hohen Effizienzen, geringen Betriebspannungen sowie zu langen Lebensdauern.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

### Beispiele

### Materialien

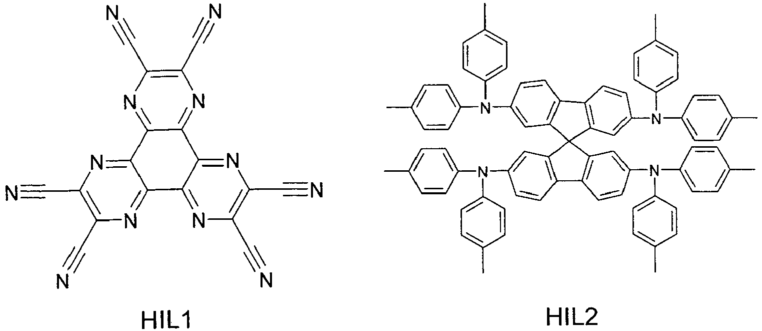

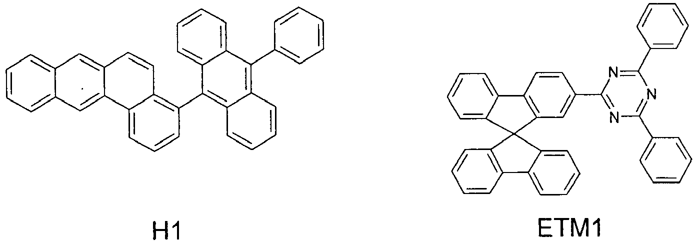

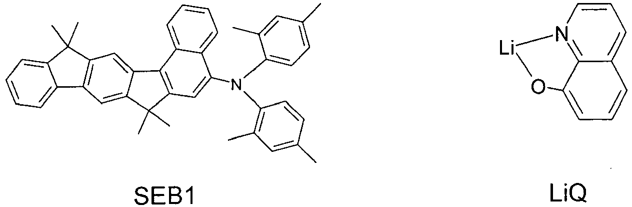

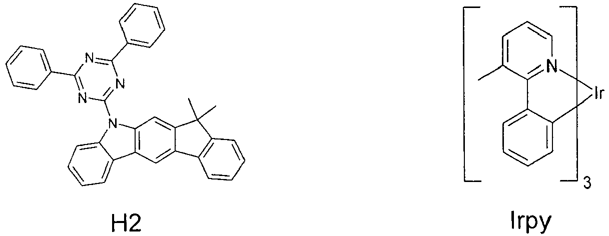

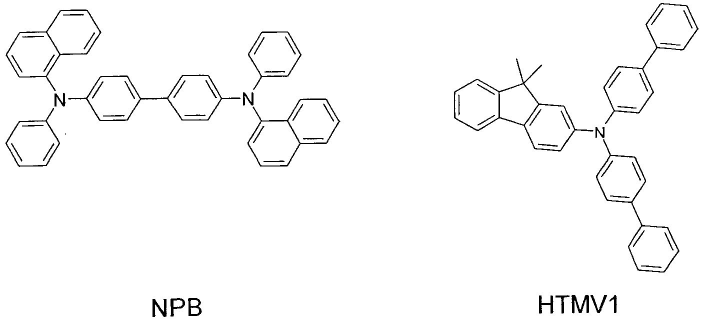

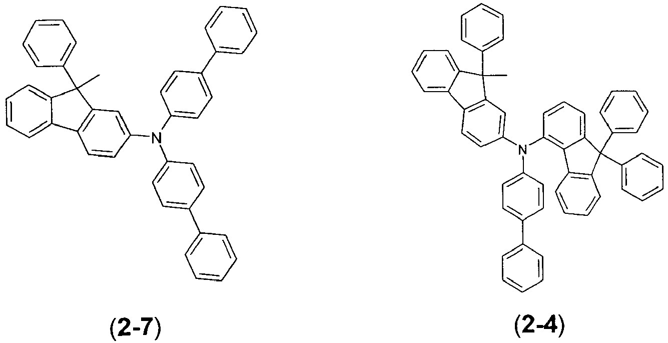

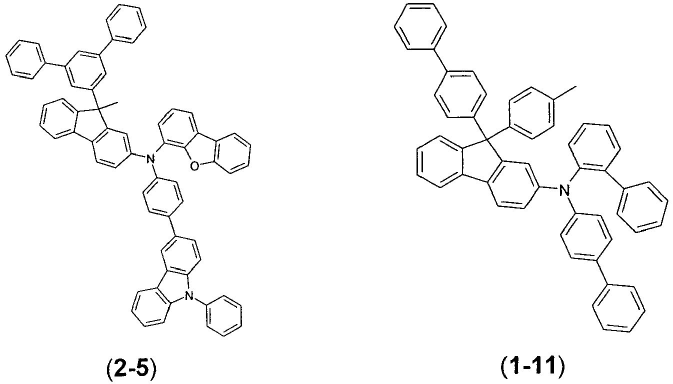

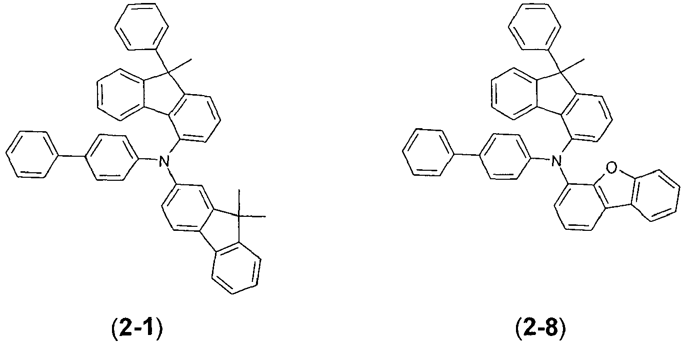

Die Materialien HIL1, HIL2 (EP 0676461), H1 (WO 2008/145239), ETM1 (WO 2005/053055), SEB1 (WO 2008/006449), LiQ und NPB, sind dem Fachmann aus dem Stand der Technik gut bekannt. Die Verbindung HTMV1 kann analog zu der in Beispiel 1 gezeigten Synthese hergestellt werden, wobei 2-Bromo-9,9-dimethyl-9H-fluoren in einer Buchwald Reaktion mit Bis-biphenyl-4-yl-amin umgesetzt wird. Die Verbindungen (**2-7**), (**2-4**), (**2-5**), **(1-11), (2-1)** und **(2-8)** sind erfindungsgemäß.

### Beispiel 1

### Synthese der Verbindung Biphenyl-2-yl-biphenyl-4-yl-(9-methyl-9-p-tolyl-9H-fluoren-2-yl)-amin (1-1) sowie der Verbindungen (1-2) bis (1-11)

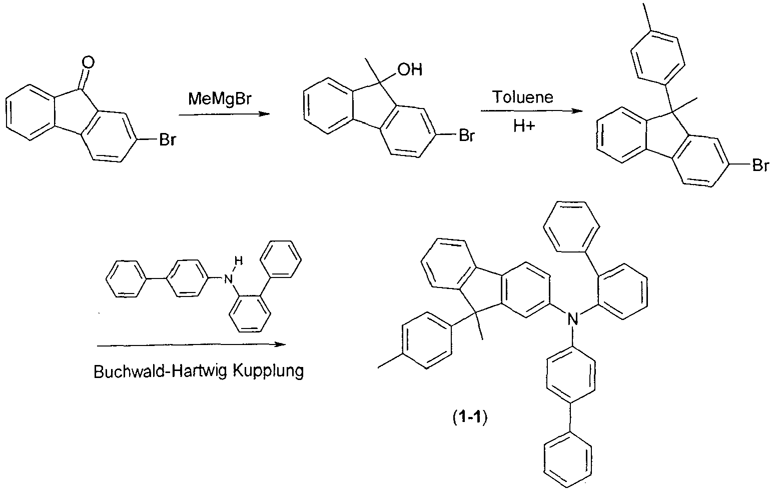

### 2-Bromo-9-methyl-9-p-tolyl-9H-fluoren

40 g (154 mmol) 2-Bromo-9H-fluorenon werden in einem ausgeheizten Kolben in 500 mL getrocknetem THF gelöst. Die Lösung wird mit N₂ gesättigt und mit 15,0 g (170 mmol) Cer(III)chlorid versetzt. Die klare Lösung wird auf -10°C abgekühlt und dann werden 121 mL (170 mmol) einer 1,4M-Methylmagnesiumbromid Lösung zugegeben. Die Reaktionsmischung wird langsam auf Raumtemperatur erwärmt und dann mit NH₄Cl gequencht (500 mL). Das Gemisch wird im Anschluss zwischen Essigester und Wasser verteilt, die organische Phase dreimal mit Wasser gewaschen, über Na₂SO₄ getrocknet und einrotiert. Die einrotierte Lösung wird mit 60 mL Toluol versetzt. Der Ansatz wird auf 50°C erhitzt und anschließend werden 27,2 mL Trifluoromethansulfonsäure (308 mmol) zugetropft. Nach eine Stunde wird die Reaktionsmischung auf Raumtemperatur abgekühlt und auf 1 L Wasser gegossen. Das Gemisch wird zwischen Toluol und Wasser verteilt, die organische Phase dreimal mit Wasser gewaschen und über Na₂SO₄ getrocknet und einrotiert. Nach Filtration des Rohproduktes über Kieselgel mit (Heptane:Essigsester, 1:1) erhielt man 32 g (60% der Theorie)

Analog dazu werden folgende bromierten Verbindungen hergestellt:

| **Edukt 1** | **Edukt 2** | **Edukt 3** | **Produkt** | **Ausbeute** |
|---|---|---|---|---|
| 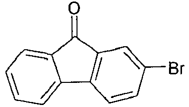 | 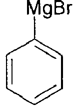 | 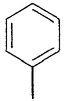 | 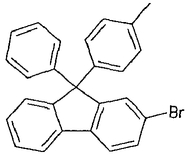 | 55% |
| 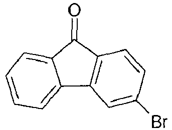 | 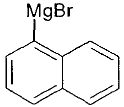 | 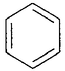 | 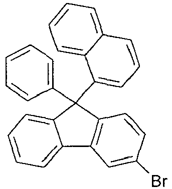 | 62% |
| 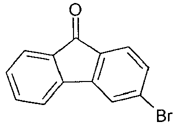 | 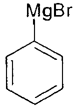 | 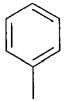 | 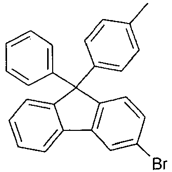 | 58% |
| 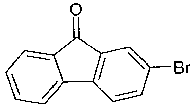 | 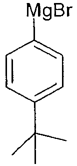 | 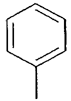 | 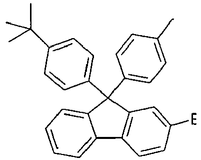 | 60% |
| 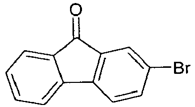 | 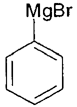 | 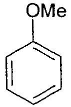 | 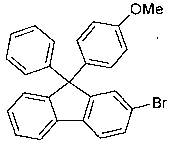 | 52% |
| 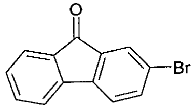 | 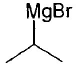 | 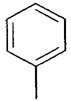 | 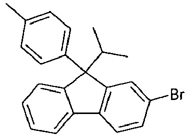 | 61% |
| 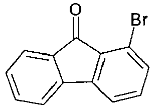 | MeMgBr | 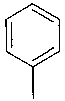 | 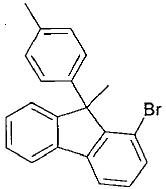 | 62% |
| 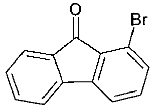 | 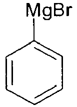 | 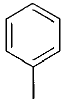 | 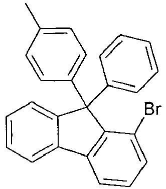 | 50% |
| 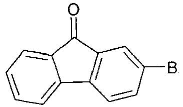 | 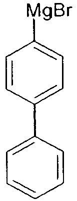 | 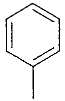 | 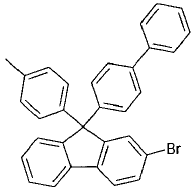 | 65% |

### Biphenyl-2-yl-biphenyl-4-yl-(9-methyl-9-p-tolyl-9H-fluoren-2-yl)-amin (1-1)

27.6 g Biphenyl-2-yl-biphenyl-4-yl-amin (85,9 mmol), 30.0 g 2-Bromo-fluoren (85.9 mmol) werden in 500 mL Toluol gelöst: Die Lösung wird entgast und mit N₂ gesättigt. Danach wird sie mit 4.3 mL (4.3 mmol) einer Tri-tert-Butylphosphin 1 M-Lösung und 0,48 g (2.15 mmol) Palladium(II)-acetat versetzt. Anschließend werden 20.6 g Natrium-tert-butylat (214.7 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 39.5 g (78% der Theorie).

Analog dazu werden Verbindungen (1-2) bis (1-11) hergestellt:

| **Edukt 1** | **Edukt 2** | **Produkt** | **Ausbeute** |
|---|---|---|---|
| 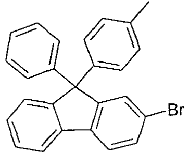 | 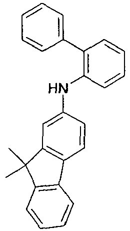 | 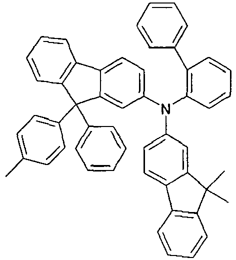 | 78% |
| | | (**1-2**) | |
| 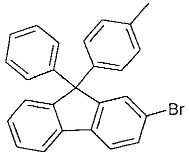 | 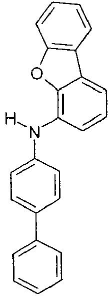 | 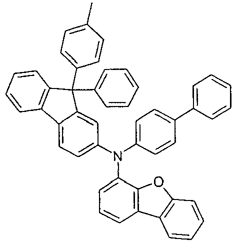 | 92% |
| | | **(1-3)** | |
| 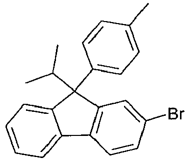 | 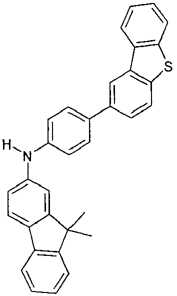 | 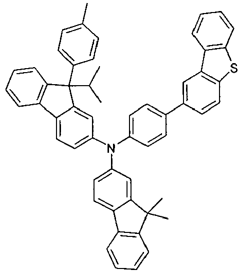 | 88% |
| | | **(1-4)** | |
| 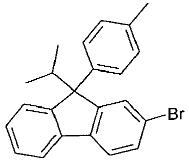 | 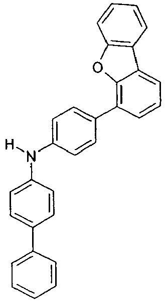 | 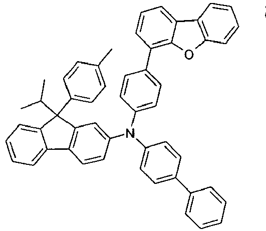 | 85% |
| | | **(1-5)** | |
| 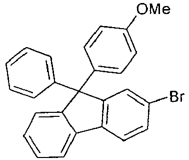 | 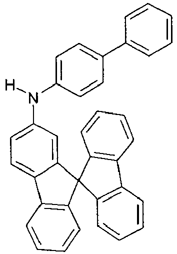 | 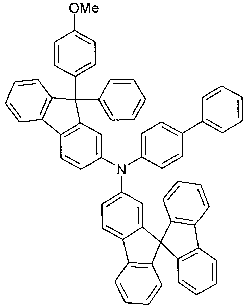 | 80% |
| | | **(1-6)** | |
| 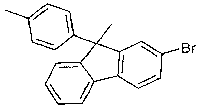 | 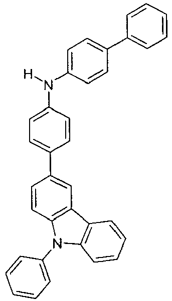 | 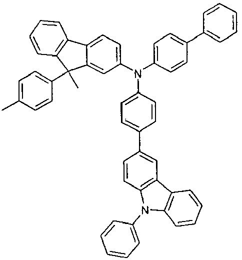 | 75% |
| | | **(1-7)** | |
| 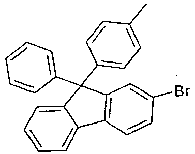 | 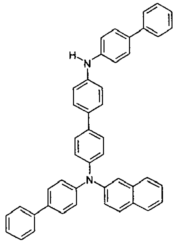 | 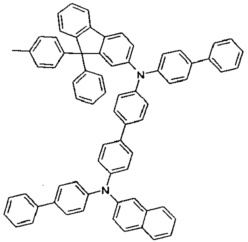 | 75% |
| | | **(1-8)** | |
| 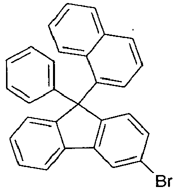 | 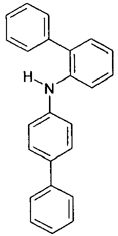 | 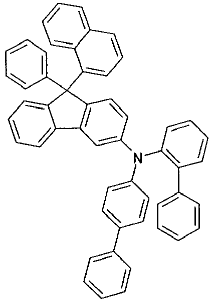 | 80% |
| | | **(1-9)** | |
| 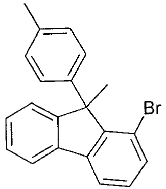 | 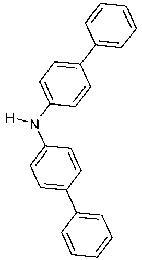 | 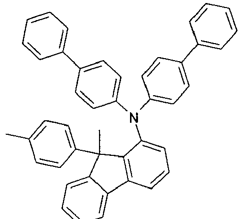 | 70% |
| | | **(1-10)** | |
| 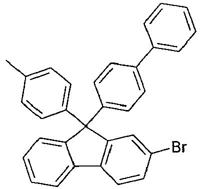 | 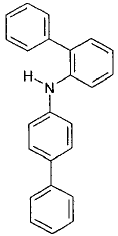 | 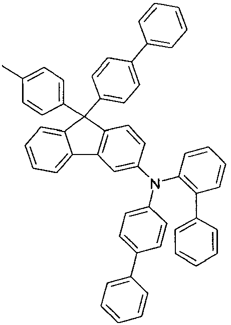 | 75% |
| | | **(1-11)** | |

### Beispiel 2

### Synthese der Verbindung Biphenyl-4-yl-(9,9-dimethyl-9H-fluoren-2-yl)-(9-methyl-9-phenyl-9H-fluoren-4-yl)-amin (2-1) sowie der Verbindungen (2-2) bis (2-8)

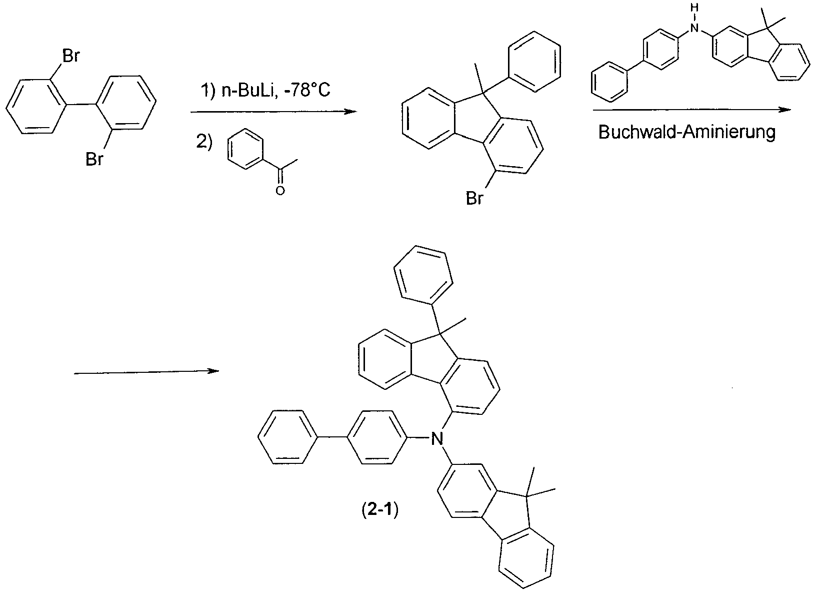

### 4-Bromo-9-methyl-9-phenyl-9H-fluoren

30 g (94 mmol) 2,2'-Dibromo-biphenyl werden in einem ausgeheizten Kolben in 200 mL getrocknetem THF gelöst. Die Reaktionsmischung wird auf -78°C gekühlt. Bei dieser Temperatur werden 37.7 mL einer 2,5 M-Lösung n-BuLi in Hexan (94 mmol) langsam zugetropft (Dauer: ca 1 h). Der Ansatz wird 1 h bei -70°C nachgerührt. Anschließend werden 11.1 mL Acetophenon (94 mmol) in 100 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 300 ml Essigsäure versetzt und anschließen werden 50 ml rauchende HCl zugegeben. Der Ansatz wird auf 75°C erhitzt und 6 h dort gehalten. Dabei fällt ein weißer Feststoff aus. Der Ansatz wird nun auf Raumtemperatur abgekühlt, der ausgefallene Feststoff wird abgesaugt und mit Methanol nachgewaschen. Der Rückstand wird im Vakuum bei 40°C getrocknet. Ausbeute beträgt 25.3 g (75 mmol) (80% der Theorie)

Analog dazu werden die folgenden bromierten Verbindungen hergestellt:

| **Edukt 1** | **Edukt 2** | **Produkt** | **Ausbeute** |
|---|---|---|---|
| 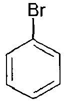 | 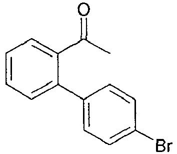 | 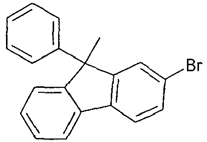 | 78 % |
| 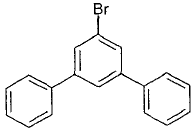 | 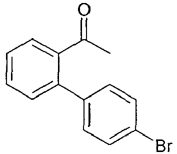 | 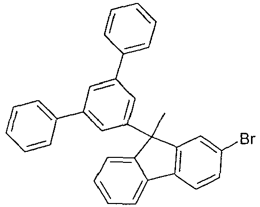 | 80% |
| 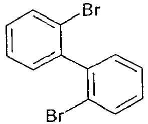 | 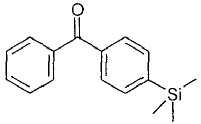 | 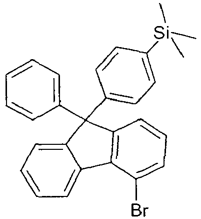 | 87% |

### Biphenyl-4-yl-(9,9-dimethyl-9H-fluoren-2-yl)-(9-methyl-9-phenyl-9H-fluoren-4-yl)-amin (2-1)

17.8 g Biphenyl-2-yl-biphenyl-4-yl-amin (49.4 mmol), 18.2 g 2.Bromo-(9-methyl-9-phenyl-9H-fluoren (54.3 mol) werden in 400 mL Toluol gelöst: Die Lösung wird entgast und mit N₂ gesättigt. Danach wird sie mit 2.96 mL (2.96 mmol) Tri-tert-Butylphosphin und 0.33 g (1.48 mmol) Palladium(II)-acetat versetzt und anschließend werden 9.8 g Natrium-tert-butylat (98.8 mmol) zugegeben. Die Reaktionsmischung wird 3 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 24.3 g (80% der Theorie).

Analog dazu werden die Verbindungen (**2-2)** bis (**2-8**) hergestellt:

| Edukt 1 | Edukt 2 | Produkt | Ausbeute |
|---|---|---|---|
| 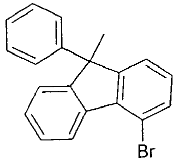 | 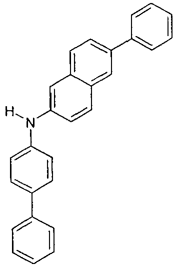 | 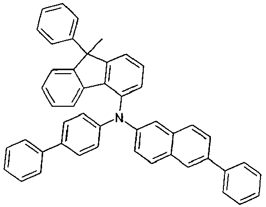 | 78% |
| | | **(2-2)** | |
| 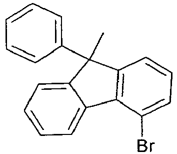 | 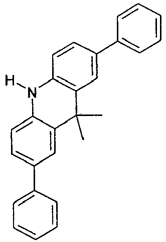 | 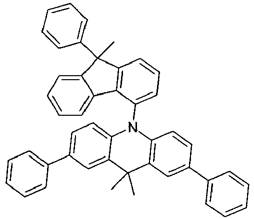 | 75% |
| | | **(2-3)** | |
| 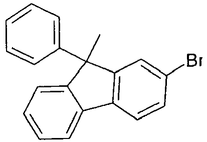 | 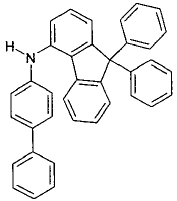 | 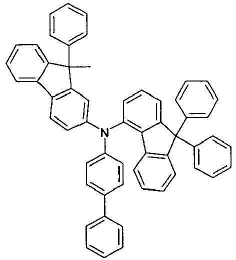 | 80% |
| | | **(2-4)** | |
| 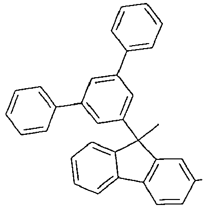 | 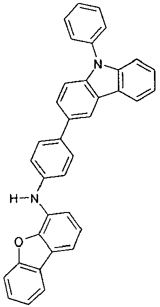 | 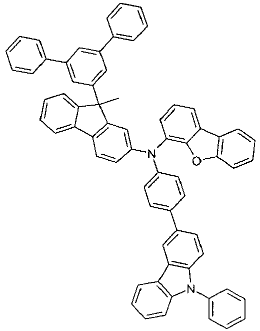 | 80% |
| | | **(2-5)** | |
| 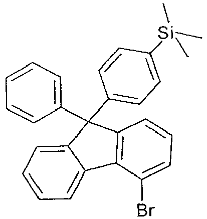 | 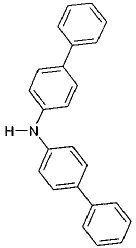 | 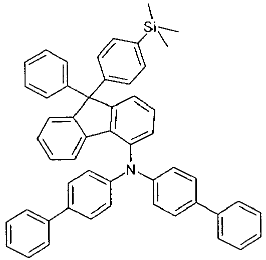 | 88% |
| | | **(2-6)** | |
| 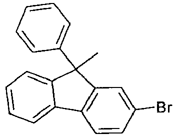 | 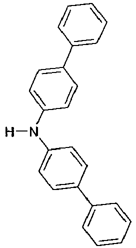 | 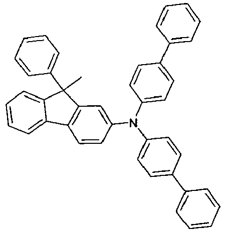 | 85% |
| | | **(2-7)** | |
| 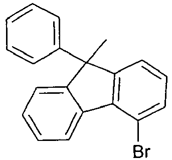 | 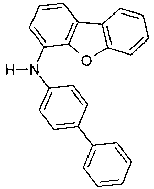 | 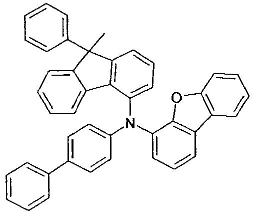 | 78% |
| | | **(2-8)** | |

### Beispiel 3

### Synthese der Verbindung Biphenyl-4-yl-biphenyl-2-yl-(7,9-diphenyl-9-p-tolyl-9H-fluoren-2-yl)-amin (3-1) sowie der Verbindungen (3-2) bis (3-4)

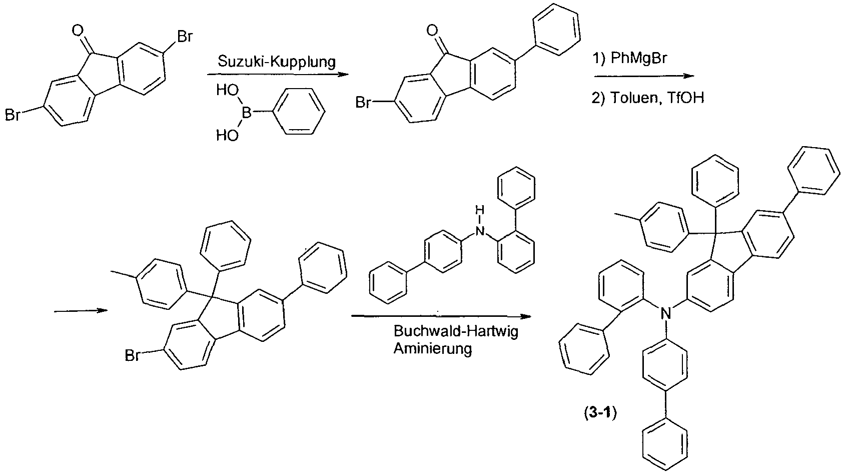

### 2-Bromo-7-phenyl-fluoren-9-on

21.6 g (178 mmol) Phenylboronsäure, 60 g (178 mmol) 2,7-Dibrom-fluorenon werden in 800 mL Dimethoxyethan und 265 mL einer 2 M Natriumcarbonat-Lösung (533 mmol) suspendiert. Zu dieser Suspension werden 6.154 g (5 mmol) Palladium tetrakis(triphenylphosphin) gegeben, und die Reaktionsmischung wird 18 h unter Rückfluss erhitzt. Nach dem Erkalten der Reaktionsmischung wird die organische Phase abgetrennt, über Kieselgel filtriert, dreimal mit 100 mL Wasser gewaschen und anschließend zur Trockene eingeengt. Nach Filtration des Rohproduktes über Kieselgel mit Toluol erhält man 38.6 g (85%) 2-Brom-7-phenyfluoren-9-on.

Analog dazu werden die folgenden bromierten Verbindungen hergestellt:

| **Edukt 1** | **Edukt 2** | **Produkt** | **Ausbeute** |
|---|---|---|---|
| 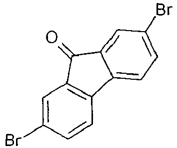 | 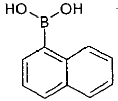 | 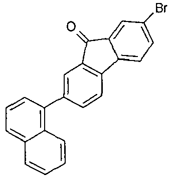 | 85% |
| 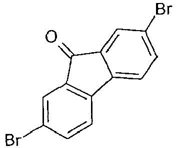 | 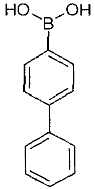 | 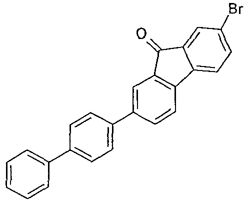 | 90% |

### 2-Bromo-7,9-diphenyl-9-p-tolyl-9H-fluoren

35 g (104 mmol) 2-Bromo-7-phenyl-fluorenon werden in einem ausgeheizten Kolben in 600 mL getrocknetem THF gelöst. Die klare Lösung wird auf -10°C abgekühlt und dann werden 38.3 mL (115 mmol) einer 3 M-Phenylmagnesiumbromid Lösung zugegeben. Die Reaktionsmischung wird langsam auf Raumtemperatur erwärmt und dann mit NH₄Cl gequencht (300 mL). Das Gemisch wird im Anschluss zwischen Essigester und Wasser verteilt, die organische Phase dreimal mit Wasser gewaschen, über Na₂SO₄ getrocknet und einrotiert. Die einrotierte Lösung wird mit 100 mL Toluol versetzt. Der Ansatz wird auf 50°C erhitzt und anschließend werden 20.4 mL Trifluoromethansulfonsäure (208 mmol) zugetropft. Nach eine Stunde wird die Reaktionsmischung auf Raumtemperatur abgekühlt und auf 1 L Wasser gegossen. Das Gemisch wird zwischen Toluol und Wasser verteilt, die organische Phase dreimal mit Wasser gewaschen und über Na₂SO₄ getrocknet und einrotiert. Nach Filtration des Rohproduktes über Kieselgel mit (Heptane:Essigsester, 1:1) erhält man 41 g (61% der Theorie).

Analog dazu werden die folgenden bromierten Verbindungen hergestellt:

| **Edukt 1** | **Edukt 2** | **Edukt 3** | **Produkt** | **Ausbeute** |
|---|---|---|---|---|
| 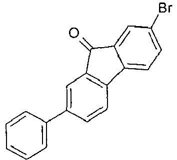 | 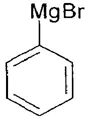 | 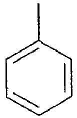 | 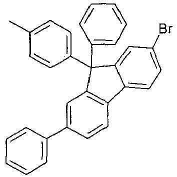 | 60% |
| 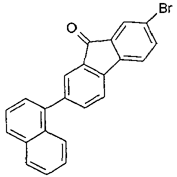 | 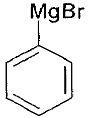 | 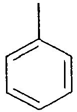 | 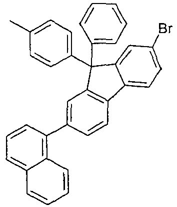 | 55% |
| 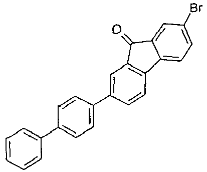 | CH3MgBr | 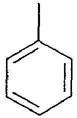 | 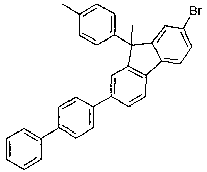 | 60% |

### Biphenyl-4-yl-biphenyl-2-yl-(7,9-diphenyl-9-p-tolyl-9H-fluoren-2-yl)-amin (3-1)

13.18 g Biphenyl-2-yl-biphenyl-4-yl-amine (41 mmol), 20 g 2-Bromo-7,9-diphenyl-9-p-tolyl-9H-fluorene (41mmol) werden in 350 mL Toluol gelöst: Die Lösung wird entgast und mit N₂ gesättigt. Danach wird sie mit 1,6 mL (1,6 mmol) Tri-tert-Butylphosphin und 184 mg (0.82 mmol) Palladium(II)-acetat versetzt. Anschließend werden 9.86 g Natrium-tert-butylat (102 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 21.8 g (73% der Theorie).

Analog dazu werden folgende Verbindungen hergestellt:

| **Edukt 1** | **Edukt 2** | **Produkt** | **Ausbeute** |
|---|---|---|---|
| 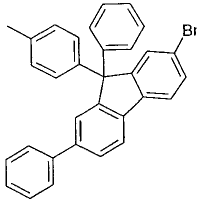 | 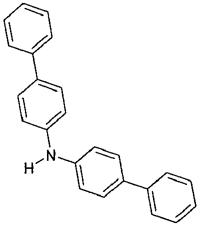 | 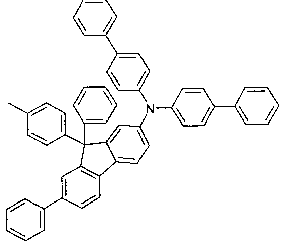 | 85% |
| | | **(3-2)** | |
| 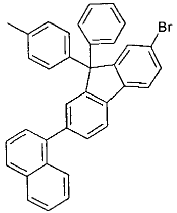 | 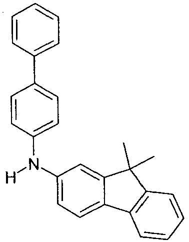 | 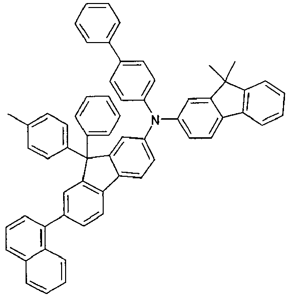 | 82% |
| | | **(3-3)** | |
| 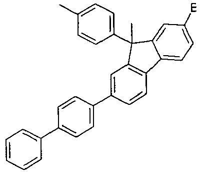 | 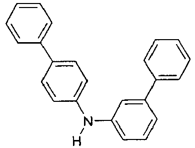 | 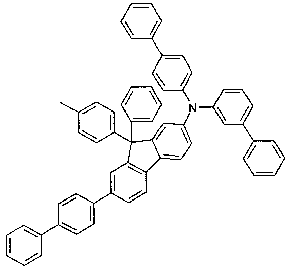 | 80% |
| | | **(3-4)** | |

### Beispiel 4

### Charakterisierung der Verbindungen

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen V1, V2 und E1 bis E3 (siehe Tabellen 1 und 2) werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / Lochinjektionsschicht (HIL1) / Lochtransportschicht (HTL) / Lochinjektionsschicht (HIL2) / Elektronenblockerschicht (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Der genaue Aufbau der OLEDs ist der Tabelle 1 zu entnehmen. Die zur Herstellung der OLEDs benötigten Materialien oben angegeben.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1 :SEB1 (95%:5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². LD80 @ 6000 cd/m² ist die Lebensdauer bis das OLED bei einer Helligkeit von 6000 cd/m² auf 80% der Anfangsintensität, also auf 4800 cd/m² abgefallen ist. Die Daten der verschiedenen OLEDs sind in der Tabelle 2 zusammengefasst.

### Verwendung von erfindungsgemäßen Verbindungen als Lochtransportmaterialien in fluorszierenden OLEDs

Insbesondere eignen sich erfindungsgemäße Verbindungen als HIL, HTL oder EBL in OLEDs. Sie eignen sich als Einzelschicht, aber auch als Mixed Komponente als HIL, HTL, EBL oder innerhalb der EML.
Verglichen mit NPB Referenz Bauteilen (V1) zeigen die Proben mit den erfindungsgemäßen Verbindungen sowohl höhere Effizienzen, als auch deutlich verbesserte Lebensdauern in Singulett blau.
Im Vergleich zum Referenzmaterial HTMV1 (V2) haben die erfindungsgemäßen Verbindungen (**2-7**), (**2-4**), (**2-5**), **(1-11), (2-1)** und **(2-8)** (E1-E4) eine bessere Lebensdauer.

In grünen Triplettbauteilen zeigen die erfindungsgemäßen Verbindungen (2-7), (2-4), (2-1) bessere Effizienzen und bessere Lebensdauern im Vergleich zu den Referenzbauteilen V3 (NPB) und V4 (HTMV1).

| **Tabelle 1: Aufbau der OLEDs** | | | | | | |
|---|---|---|---|---|---|---|
| (Schichtaufbau: Substrat/IL/HTL/IL/EBL/EML/ETL/EIL (1 nm LiQ)/Kathode) | | | | | | |
| ***Bsp*.** | ***HIL1*** | ***HTL*** | ***HIL2*** | ***EBL*** | ***EML*** | ***ETL*** |
| | *Dicke* / *nm* | *Dicke* / *nm* | *Dicke* / *nm* | *Dicke* / *nm* | *Dicke* / *nm* | *Dicke* / *nm* |
| *V1* | *HIL1* | *HIL2* | *HIL1* | *NPB* | *H1(95%):SEB1(5%)* | *ETM1(50%):LiQ(50%)* |
| | *5 nm* | *140 nm* | *5nm* | *20 nm* | *20 nm* | *30 nm* |
| *V2* | *HIL1* | *HIL2* | *HIL1* | *HTMV1* | *H1(95%):SEB1(5%)* | *ETM1(50%):LiQ(50%)* |
| | *5 nm* | *140 nm* | *5 nm* | *20 nm* | *20 nm* | *30 nm* |
| *E1* | *HIL1* | *HIL2* | *HIL1* | ***(2-7)*** | *H1(95%):SEB1(5%)* | *ETM1(50%):LiQ(50%)* |
| | *5nm* | *140 nm* | *5 nm* | *20 nm* | *20 nm* | *30 nm* |
| *E2* | *HIL1* | *HIL2* | *HIL1* | ***(2-4)*** | *H1(95%):SEB1(5%)* | *ETM1(50%):LiQ(50%)* |
| | *5nm* | *140 nm* | *5 nm* | *20 nm* | *20 nm* | *30 nm* |
| *E3* | *HIL1* | *HIL2* | *HIL1* | ***(2-5)*** | *H1(95%):SEB1(5%)* | *ETM1*(*50*%)*:LiQ*(*50*%) |
| | *5 nm* | *140 nm* | *5 nm* | *20 nm* | *20 nm* | *30 nm* |
| *E4* | *HIL1* | *HIL2* | *HIL1* | ***(1-11)*** | *H1(95%):SEB1(5%)* | *ETM1(50%):LiQ(50%)* |
| | *5 nm* | *140 nm* | *5 nm* | *20 nm* | *20 nm* | *30 nm* |
| *E5* | *HIL1* | *HIL2* | *HIL1* | ***(2-1)*** | *H1(95%):SEB1(5%)* | *ETM1(50%):LiQ(50%)* |
| | *5 nm* | *140 nm* | *5 nm* | *20 nm* | *20 nm* | *30 nm* |
| *E6* | *HIL1* | *HIL2* | *HIL1* | ***(2-8)*** | *H1(95%):SEB1(5%)* | *ETM1(50%):LiQ(50%)* |
| | *5 nm* | *140 nm* | *5 nm* | *20 nm* | *20 nm* | *30 nm* |

| **Tabelle 2: Daten der OLEDs** | | | | |
|---|---|---|---|---|
| ***Bsp***. | ***EQE* @ *1000 cd*/*m2*** | ***LD80* @ *6000 cd*/*m²*** | ***CIE*** | |
| | % | *[h]* | *x* | *y* |
| *V1* | *4.8* | *70* | 0.14 | 0.17 |
| *V2* | *7.0* | *130* | 0.13 | 0.15 |
| *E1* | *6.1* | *155* | 0.13 | 0.15 |
| *E2* | *7.0* | *161* | 0.14 | 0.15 |
| *E3* | *6.9* | *158* | 0.13 | 0.14 |
| *E4* | *6.5* | *155* | 0.13 | 0.15 |
| *E5* | *7.0* | *155* | 0.14 | 0.15 |
| *E6* | *6.9* | *160* | 0.14 | 0.15 |

| **Tabelle 3: Aufbau der OLEDs** | | | | | |
|---|---|---|---|---|---|
| (Schichtaufbau: Substrat/HTL/HIL2/EBL/EML/ETL/Kathode) | | | | | |
| ***Bsp.*** | ***HTL*** | ***HIL2*** | ***EBL*** | ***EML*** | ***ETL*** |
| | *Dicke* / *nm* | *Dicke* / *nm* | *Dicke* / *nm* | *Dicke* / *nm* | *Dicke* / *nm* |
| *V3* | *HIL2* | *HIL1* | *NPB* | *H2(88%):Irpy(12%)* | *ETM1(50%):LiQ(50%)* |
| | *70 nm* | *5 nm* | *20 nm* | *30 nm* | *40 nm* |
| *V4* | *HIL2* | *HIL1* | *HTMV1* | *H2(88%):Irpy(12%)* | *ETM1(50%):LiQ(50%)* |
| | *70 nm* | *5 nm* | *20 nm* | *30 nm* | *40 nm* |
| *E7* | *HIL2* | *HIL1* | (***2-7***) | *H2(88%):Irpy(12%)* | *ETM1(50%):LiQ(50%)* |
| | *70 nm* | *5 nm* | *20 nm* | *30 nm* | *40 nm* |
| *E8* | *HIL2* | *HIL1* | (***2-4***) | *H2(88%):Irpy(12%)* | *ETM1(50%):LiQ(50%)* |
| | *70 nm* | *5 nm* | *20 nm* | *30 nm* | *40 nm* |
| *E9* | *HIL2* | *HIL1* | *(**2-1**)* | *H2(88%):Irpy(12%)* | *ETM1(50%):LiQ(50%)* |
| | *70 nm* | *5 nm* | *20 nm* | *30 nm* | *40 nm* |

| **Tabelle 4: Daten der OLEDs** | | | | |
|---|---|---|---|---|
| ***Bsp.*** | ***Effizienz* @ *1000 cd*/*m2*** | ***LD80* @ *8000 cd*/*m²*** | ***CIE*** | |
| | % | *[h]* | *x* | Y |
| *V3* | *13.4* | *85* | 0.36 | 0.61 |
| *V4* | *17.0* | *170* | 0.35 | 0.62 |
| *E7* | *17.5* | *190* | 0,34 | 0,62 |
| *E8* | *18,3* | *215* | 0,35 | 0,62 |
| *E9* | *18,5* | *225* | 0,37 | 0,60 |

## Patentansprüche

1. Elektrolumineszenzvorrichtung enthaltend wenigstens eine Verbindung der allgemeinen Formel (1) 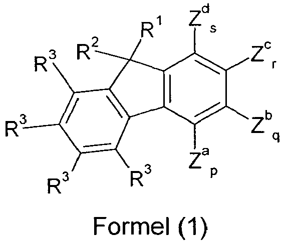 wobei für die verwendeten Symbole und Indices gilt:
p, q, r, s
sind 0 oder 1, wobei p + q + r + s = 1 gilt, bevorzugt ist p =1 oder r = 1 oder s = 1, ganz bevorzugt ist p = 1 oder r = 1;
Z^{a}₀, Z^{b}₀, Z^{c}₀, Z^{d}₀
sind gleich oder verschieden bei jedem Auftreten gleich R⁴
Z^{a}₁, Z^{b}₁, Z^{c}₁, Z^{d}₁ sind gleich 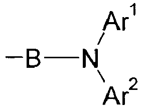
B
ist eine Einfachbindung, eine bivalente Arylgruppe mit 6 bis 30 Ringatomen oder eine bivalente Heteroarylgruppe mit 5 bis 30 Ringatomen, die jeweils mit einem oder mehreren Resten R⁶ substituiert sein können, wobei, wenn B eine Einfachbindung ist, das Stickstoffatom direkt an das Fluoren gebunden ist;
Ar¹, Ar²
sind bei jedem Auftreten gleich oder verschieden ein Arylgruppe mit 10 bis 60 Ringatomen oder eine Heteroarylgruppe 10 bis 60 Ringatomen, welche mit einem oder mehreren Resten R⁵, die gleich oder verschieden voneinander sind, substituiert sein können, wobei beide Gruppen Ar¹ oder Ar² jeweils mindestens zwei oder mehr aromatische oder heteroaromatische Ringe enthalten,
wobei zwei der aromatischen oder heteroaromatischen Ringe in Ar¹ und/oder zwei der aromatischen oder heteroaromatischen Ringe in Ar² kondensiert sein können, bevorzugt liegen sie nicht kondensiert vor,
und wobei zwei der aromatischen oder heteroaromatischen Ringe in Ar¹ durch eine bivalente Gruppe -O-, -S-, -Si(R⁵)₂-, -C(R⁵)₂ oder-NR⁵- verbrückt sein können bzw. zwei der aromatischen oder heteroaromatischen Ringe in Ar² durch eine bivalente Gruppe -O-,-S-, -Si(R⁵)₂-, -C(R⁵)₂,- oder -NR⁵- verbrückt sein können, wobei unverbrückte Ringe bevorzugt sind
und wobei ein aromatischer oder heteroaromatischer Ring aus Ar¹ mit einem aromatischen oder heteroaromatischen Ring aus Ar² durch eine bivalente Gruppe -O-, -S-, -Si(R⁵)₂-, -NR⁵- oder -C(R⁵)₂- verbrückt sein kann, wobei unverbrückte Gruppen Ar¹ und Ar² bevorzugt sind;
R¹, R², R³, R⁴, und R⁵
sind H, D, F, Cl, Br, I, C(=O)R⁶, CN, Si(R⁶)₃, NO₂, N(R⁶)₂, P(=O)(R⁶)₂, S(=O)_{R}⁶, S(=O)₂R⁶, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C=C-, Si(R⁶)₂, C=O, C=S, C=NR⁶, -C(=O)O-, -C(=O)NR⁶-, P(=O)(R⁶), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, oder eine Aryloxygruppe mit 5 bis 60 aromatischen Ringatomen, die mit einem oder mehreren Resten R⁶ substituiert sein kann, oder einer Aralkylgruppe mit 5 bis 60 aromatischen Ringatomen, die jeweils mit einem oder mehreren Resten R⁶ substituiert sein kann, wobei die Reste R¹ und R² nicht identisch sein dürfen und die Reste R³ bis R⁵ bei jedem Auftreten gleich oder verschieden sein können aber identisch mit entweder R¹ oder mit R² sein kann;
R⁶
ist bei jedem Auftreten gleich oder verschieden H, D, F, Cl, Br, I, C(=O)R⁷, CN, Si(R⁷)₃, NO₂, P(=O)(R⁷)₂, S(=O)R⁷, S(=O)₂R⁷, N(R⁷)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁷ substituiert sein können und wobei eine oder mehrere CH₂-Gruppen in den oben genannten Gruppen durch -R⁷C=CR⁷-, -C≡C-, Si(R⁷)₂, C=O, C=S, C=NR⁷, -C(=O)O-, -C(=O)NR⁷-, P(=O)(R⁷), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁷ substituiert sein kann, oder eine Aryloxy- oder Heteroaryloxygruppe mit 5 bis 30 aromatischen Ringatomen, die durch einen oder mehrere Reste R⁷ substituiert sein kann, wobei zwei oder mehr benachbarte Substituenten R⁶ miteinander ein mono- oder polycyclisches Ringsystem bilden können;
R⁷
ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R⁷ miteinander ein mono- oder polycyclisches Ringsystem bilden können.

2. Vorrichtung gemäß Anspruch 1, dadurch charakterisiert, dass die Verbindung die allgemeine Formel (2) hat 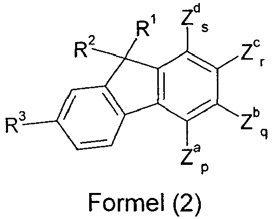 wobei die angegebenen Symbole und Indices wie in Anspruch 1 angegeben definiert sind.

3. Vorrichtung gemäß Anspruch 1 oder 2, dadurch charakterisiert, dass die Verbindung die allgemeine Formel (3) hat 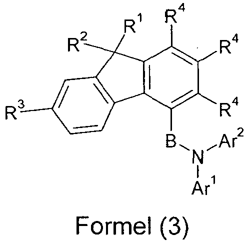 wobei die angegebenen Symbole und Indices wie in Anspruch 1 angegeben definiert sind.

4. Vorrichtung gemäß Anspruch 1 oder 2, dadurch charakterisiert, dass die Verbindung die allgemeine Formel (4) hat 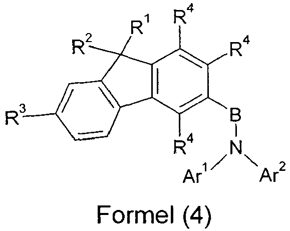 wobei die angegebenen Symbole und Indices wie in Anspruch 1 angegeben definiert sind.

5. Vorrichtung gemäß Anspruch 1 oder 2, dadurch charakterisiert, dass die Verbindung die allgemeine Formel (5) hat 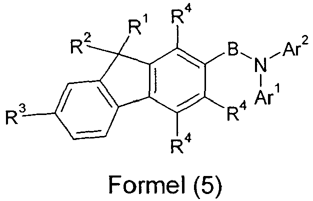 wobei die angegebenen Symbole und Indices wie in Anspruch 1 angegeben definiert sind.

6. Vorrichtung gemäß Anspruch 1 oder 2, dadurch charakterisiert, dass die Verbindung die allgemeine Formel (6) hat 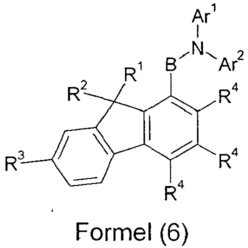 wobei die angegebenen Symbole und Indices wie in Anspruch 1 angegeben definiert sind.

7. Vorrichtung gemäß einem oder mehreren der Ansprüche 1 bis 6, dadurch charakterisiert, dass B eine Einfachbindung oder eine Phenylen-, Biphenylen-, Terphenylen-, Naphthylen-, Pyridinylen-, Pyrimidinylen-, Pyrazinylen-, Pyridazinylen-, Triazinylen-, Dibenzofuranylen-, Dibenzothiophenylen- Fluorenylen-, oder Carbazoylengruppe, die mit einem oder mehreren Resten R⁶ substituiert sein können, ist; bevorzugt ist B eine Einfachbindung oder eine Phenylen-, Biphenylen-, Terphenylen-, Naphthylen-, Dibenzofuranylen- oder Dibenzothiophenylen Fluorenylen-, oder Carbazoylengruppe, die mit einem oder mehreren Resten R⁶ substituiert sein können; ganz bevorzugt ist B eine Einfachbindung oder eine Phenylengruppe, die mit einem oder mehreren Resten R⁶ substituiert sein kann; ganz besonders bevorzugt ist B eine Einfachbindung.

8. Vorrichtung gemäß einem oder mehreren der Ansprüche 1 bis 7, dadurch charakterisiert, Ar¹ und Ar² sind, gleich oder verschieden bei jedem Auftreten, ausgewählt sind aus einer Phenyl-Pyridyl-, PhenylNaphthyl-, Biphenyl-, Terphenyl- oder Quarterphenylgruppe, die mit einem oder mehreren Resten R⁶, die gleich oder verschieden voneinander sein können, substituiert sein können, wobei zwei der aromatischen oder heteroaromatischen Ringe in Ar¹ durch eine bivalente Gruppe -O-, -S-, -C(R⁵)₂- oder -Si(R⁵)₂- verbrückt sein können bzw. zwei der aromatischen oder heteroaromatischen Ringe in Ar² durch eine bivalente Gruppe -O-, -S-, -C(R⁵)₂- oder -Si(R⁵)₂- verbrückt sein können, wobei unverbrückte Ringe bevorzugt sind, und wobei ein aromatischer oder heteroaromatischer Ring aus Ar¹ mit einem aromatischen oder heteroaromatischen Ring aus Ar² durch eine bivalente Gruppe -O-, -S-, -Si(R⁵)₂-, -NR⁵- oder -C(R⁵)₂- verbrückt sein kann, wobei unverbrückte Gruppen Ar¹ und Ar² bevorzugt sind.

9. Vorrichtung gemäß einem oder mehreren der Ansprüche 1 bis 8, dadurch charakterisiert, es sich bei der Verbindung der Formeln (1) bis (6) um eine Monoaminverbindung handelt.

10. Vorrichtung gemäß einem oder mehreren der Ansprüche 1 bis 9, dadurch charakterisiert, dass es sich bei der Vorrichtung um einen organischen lichtemittierenden Transistor (OLETs), eine organische Feld Quench-Vorrichtung (OFQDs), eine organische lichtemittierende elektrochemische Zellen (OLECs, LECs oder LEECs), eine organische Laserdiode (O-Laser) und um eine organische lichtemittierende Diode (OLEDs) handelt, bevorzugt handelt es sich um eine OLED.

11. Vorrichtung gemäß einem oder mehreren der Ansprüche 1 bis 10, dadurch charakterisiert, dass die wenigstens eine Verbindung der Formel (1) bis (6) mit den folgenden Funktionen und in den folgenden Schichten in der Vorrichtung eingesetzt wird:
• als Lochtransportmaterial in einer Lochtransport- oder Lochinjektionsschicht
• als Excitonenblockiermaterial,
• als Elektronenblockiermaterial,
• als Matrixmaterial in einer emittierenden Schicht,
• als Emitter in einer emittierende Schicht.

12. Verbindung der allgemeinen Formel (255) 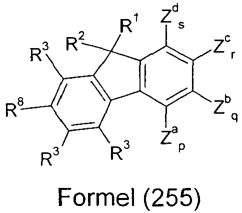 wobei für die verwendeten Symbole und Indices gilt:
p, q, r, s
sind 0 oder 1, wobei p + q + r + s = 1 gilt, bevorzugt ist p =1 oder r = 1 oder s = 1, ganz bevorzugt ist p = 1 oder r = 1;
Z^{a}₀, Z^{b}₀, Z^{c}₀, Z^{d}₀
sind gleich oder verschieden bei jedem Auftreten gleich R⁴ Z^{a}₁, Z^{b}₁, Z^{c}₁, Z^{d}₁ sind gleich 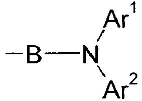
B
ist eine Einfachbindung, eine bivalente Arylgruppe mit 6 bis 30 Ringatomen oder eine bivalente Heteroarylgruppe mit 5 bis 30 Ringatomen, die jeweils mit einem oder mehreren Resten R⁶ substituiert sein können, wobei, wenn B eine Einfachbindung ist, das Stickstoffatom direkt an das Fluoren gebunden ist;
Ar¹, Ar²
sind bei jedem Auftreten gleich oder verschieden eine nicht kondensierte Arylgruppe mit 10 bis 60 Ringatomen oder eine Heteroarylgruppe 10 bis 60 Ringatomen, welche mit einem oder mehreren Resten R⁵, die gleich oder verschieden voneinander sind, substituiert sein können, wobei beide Gruppen Ar¹ oder Ar² jeweils mindestens zwei oder mehr aromatische oder heteroaromatische Ringe enthalten,
wobei zwei der aromatischen oder heteroaromatischen Ringe in Ar¹ durch eine bivalente Gruppe -O-, -S-, -Si(R⁵)₂-, -C(R⁵)₂- oder-NR⁵- verbrückt sein können bzw. zwei der aromatischen oder heteroaromatischen Ringe in Ar² durch eine bivalente Gruppe -O-,-S-, -Si(R⁵)₂-, -C(R⁵)₂- oder -NR⁵- verbrückt sein können, wobei unverbrückte Ringe bevorzugt sind
und wobei ein aromatischer oder heteroaromatischer Ring aus Ar¹ mit einem aromatischen oder heteroaromatischen Ring aus Ar² durch eine bivalente Gruppe -O-, -S-, -Si(R⁵)₂-, -NR⁵- oder -C(R⁵)₂- verbrückt sein kann, wobei unverbrückte Gruppen Ar¹ und Ar² bevorzugt sind;
R¹, R², R³ und R⁴
sind H, D, F, Cl, Br, I, C(=O)R⁶, CN, Si(R⁶)₃, NO₂, N(R⁶)₂, P(=O)(R⁶)₂, S(=O)_{R}⁶, S(=O)_{2R}⁶, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, oder eine Aryloxygruppe mit 5 bis 60 aromatischen Ringatomen, die mit einem oder mehreren Resten R⁶ substituiert sein kann, oder einer Aralkylgruppe mit 5 bis 60 aromatischen Ringatomen, die jeweils mit einem oder mehreren Resten R⁶ substituiert sein kann,
wobei die Reste R¹ und R² nicht identisch sein dürfen und die Reste R³ bis R⁵ bei jedem Auftreten gleich oder verschieden sein können aber identisch mit entweder R¹ oder mit R² sein können
und wobei wenigstens einer der Reste aus R¹ und R² ein aromatisches oder heteroaromatisches Ringsystem mit 6 bis 30 aromatischen Ringatomen, das jeweils durch einen oder mehrere Reste R⁶ substituiert sein kann, darstellt;
R⁵
ist H, D, C(=O)R⁶, CN, Si(R⁶)₃, NO₂, N(R⁶)₂, P(=O)(R⁶)₂, S(=O)R⁶, S(=O)₂R⁶, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁶ substituiert sein kann, oder eine Aryloxygruppe mit 5 bis 60 aromatischen Ringatomen, die mit einem oder mehreren Resten R⁶ substituiert sein kann, oder einer Aralkylgruppe mit 5 bis 60 aromatischen Ringatomen, die jeweils mit einem oder mehreren Resten R⁶ substituiert sein kann,
R⁶
ist bei jedem Auftreten gleich oder verschieden H, D, F, Cl, Br, I, C(=O)R⁷, CN, Si(R⁷)₃, NO₂, P(=O)(R⁷)₂, S(=O)R⁷, S(=O)₂R⁷, N(R⁷)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁷ substituiert sein können und wobei eine oder mehrere CH₂-Gruppen in den oben genannten Gruppen durch -R⁷C=CR⁷-, -C≡C-, Si(R⁷)₂, C=O, C=S, C=NR⁷, -C(=O)O-, -C(=O)NR⁷-, P(=O)(R⁷), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁷ substituiert sein kann, oder eine Aryloxy- oder Heteroaryloxygruppe mit 5 bis 30 aromatischen Ringatomen, die durch einen oder mehrere Reste R⁷ substituiert sein kann, wobei zwei oder mehr benachbarte Substituenten R⁶ miteinander ein mono- oder polycyclisches Ringsystem bilden können;
R⁷
ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R⁷ miteinander ein mono- oder polycyclisches Ringsystem bilden können;
R⁸
ist H, D, C(=O)R⁹, CN, Si(R⁹)₃, NO₂, N(R⁹)₂, P(=O)(R⁹)₂, S(=O)R⁹, S(=O)₂R⁹, eine geradkettige Alkyl- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁹ substituiert sein können und wobei eine oder mehrere CH₂-Gruppen in den oben genannten Gruppen durch -R⁹C=CR⁹-, -C≡C-, Si(R⁹)₂, C=O, C=S, C=NR⁹, -C(=O)O-, -C(=O)NR⁹-, P(=O)(R⁹), -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁹ substituiert sein kann, oder eine Aryloxygruppe mit 5 bis 60 aromatischen Ringatomen, die mit einem oder mehreren Resten R⁹ substituiert sein kann, oder einer Aralkylgruppe mit 5 bis 60 aromatischen Ringatomen, die jeweils mit einem oder mehreren Resten R⁹ substituiert sein kann;
R⁹
ist bei jedem Auftreten gleich oder verschieden H, D, F, Cl, Br, I, C(=O)R¹⁰, CN, Si(R¹⁰)₃, NO₂, P(=O)(R¹⁰)₂, S(=O)R¹⁰, S(=O)₂R¹⁰, N(R¹⁰)₂, eine geradkettige Alkyl- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R¹⁰ substituiert sein können und wobei eine oder mehrere CH₂-Gruppen in den oben genannten Gruppen durch-R¹⁰C=CR¹⁰-, -C≡C-, Si(R¹⁰)₂, C=O, C=S, C=NR¹⁰, -C(=O)O-, -C(=O)NR¹⁰-, P(=O)(R¹⁰), -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R¹⁰ substituiert sein kann, oder eine Aryloxy- oder Heteroaryloxygruppe mit 5 bis 30 aromatischen Ringatomen, die durch einen oder mehrere Reste R¹⁰ substituiert sein kann, wobei zwei oder mehr benachbarte Substituenten R¹⁰ miteinander ein mono- oder polycyclisches Ringsystem bilden können;
R¹⁰
ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R¹⁰ miteinander ein mono- oder polycyclisches Ringsystem bilden können.

13. Verbindung gemäß Anspruch 12, **dadurch gekennzeichnet, dass** sie die allgemeine Formel (256) hat 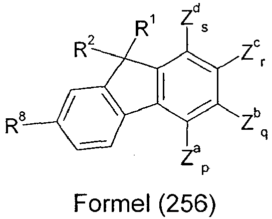 wobei die angegebenen Symbole und Indices wie in Anspruch 12 angegeben definiert sind.

14. Verfahren zur Herstellung der Verbindung gemäß Anspruch Verbindung gemäß Anspruch 12 oder 13 mittels Buchwald Kupplung.

15. Verwendung der Verbindung gemäß Anspruch 12 oder 13 in einer elektronischen Vorrichtung.

16. Elektronische Vorrichtung enthaltend wenigstens eine Verbindung gemäß Anspruch 12 oder 13, wobei die elektronische Vorrichtung bevorzugt ausgewählt ist aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser).

## Claims

1. Electroluminescent device comprising at least one compound of the general formula (1) 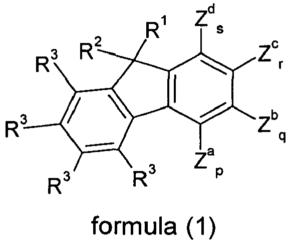 where the following applies to the symbols and indices used:
p, q, r, s
are 0 or 1, where p + q + r + s = 1, preferably p =1 or r = 1 or s = 1, very preferably p = 1 or r = 1;
Z^{a}₀, Z^{b}₀, Z^{c}₀, Z^{d}₀
are, identically or differently on each occurrence, equal to R⁴
Z^{a}₁, Z^{b}₁, Z^{c}₁, Z^{d}₁ are equal to 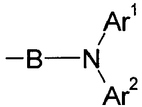
B
is a single bond, a divalent aryl group having 6 to 30 ring atoms or a divalent heteroaryl group having 5 to 30 ring atoms, each of which may be substituted by one or more radicals R⁶, where, if B is a single bond, the nitrogen atom is bonded directly to the fluorene;
Ar¹, Ar²
are on each occurrence, identically or differently, an aryl group having 10 to 60 ring atoms or a heteroaryl group 10 to 60 ring atoms, which may be substituted by one or more radicals R⁵, which are identical to or different from one another, where both groups Ar¹ or Ar² each contain at least two or more aromatic or heteroaromatic rings,
where two of the aromatic or heteroaromatic rings in Ar¹ and/or two of the aromatic or heteroaromatic rings in Ar² may be condensed, they are preferably in uncondensed form,
and where two of the aromatic or heteroaromatic rings in Ar¹ may be bridged by a divalent group -O-, -S-, -Si(R⁵)₂-, -C(R⁵)₂ or -NR⁵- or two of the aromatic or heteroaromatic rings in Ar² may be bridged by a divalent group -O-, -S-, -Si(R⁵)₂-, -C(R⁵)₂,- or -NR⁵-, where unbridged rings are preferred
and where an aromatic or heteroaromatic ring from Ar¹ may be bridged to an aromatic or heteroaromatic ring from Ar² by a divalent group -O-, -S-, -Si(R⁵)₂-, -NR⁵- or -C(R⁵)₂-, where unbridged groups Ar¹ and Ar² are preferred;
R¹, R², R³, R⁴, and R⁵
are H, D, F, Cl, Br, I, C(=O)R⁶, CN, Si(R⁶)₃, NO₂, N(R⁶)₂, P(=O)-(R⁶)₂, S(=O)R⁶, S(=O)₂R⁶, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁶ and where one or more CH₂ groups in the above-mentioned groups may be replaced by -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO or SO₂ and where one or more H atoms in the above-mentioned groups may be replaced by D, F, Cl, Br, I, CN or NO₂, or an aromatic or heteroaromatic ring system having 6 to 30 aromatic ring atoms, which may in each case be substituted by one or more radicals R⁶, or an aryloxy group having 5 to 60 aromatic ring atoms, which may be substituted by one or more radicals R⁶, or an aralkyl group having 5 to 60 aromatic ring atoms, which may in each case be substituted by one or more radicals R⁶, where the radicals R¹ and R² cannot be identical and the radicals R³ to R⁵ may on each occurrence be identical or different, but may be identical to either R¹ or to R²;
R⁶
is on each occurrence, identically or differently, H, D, F, Cl, Br, I, C(=O)R⁷, CN, Si(R⁷)₃, NO₂, P(=O)(R⁷)₂, S(=O)R⁷, S(=O)₂R⁷, N(R⁷)₂, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁷ and where one or more CH₂ groups in the above-mentioned groups may be replaced by -R⁷C=CR⁷-, -C=C-, Si(R⁷)₂, C=O, C=S, C=NR⁷, -C(=O)O-, -C(=O)NR⁷-, P(=O)(R⁷), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R⁷, or an aryloxy or heteroaryloxy group having 5 to 30 aromatic ring atoms, which may be substituted by one or more radicals R⁷, where two or more adjacent substituents R⁶ may form a mono- or polycyclic ring system with one another;
R⁷
is selected from the group consisting of H, D, F, an aliphatic hydrocarbon radical having 1 to 20 C atoms or an aromatic or heteroaromatic ring system having 5 to 30 C atoms, in which one or more H atoms may be replaced by D or F, where two or more adjacent substituents R⁷ may form a mono- or polycyclic ring system with one another.

2. Device according to Claim 1, **characterised in that** the compound has the general formula (2) 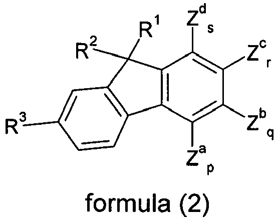 where the symbols and indices indicated are defined as indicated in Claim 1.

3. Device according to Claim 1 or 2, **characterised in that** the compound has the general formula (3) 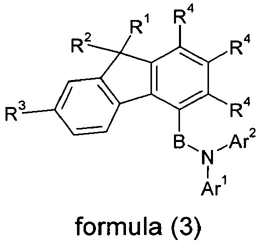 where the symbols and indices indicated are defined as indicated in Claim 1.

4. Device according to Claim 1 or 2, **characterised in that** the compound has the general formula (4) 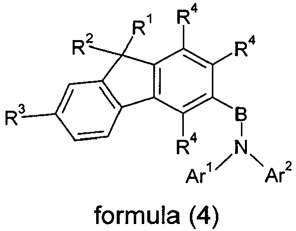 where the symbols and indices indicated are defined as indicated in Claim 1.

5. Device according to Claim 1 or 2, **characterised in that** the compound has the general formula (5) 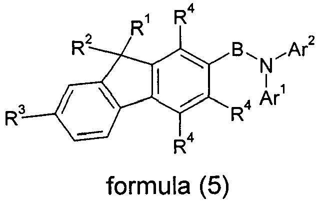 where the symbols and indices indicated are defined as indicated in Claim 1.

6. Device according to Claim 1 or 2, **characterised in that** the compound has the general formula (6) 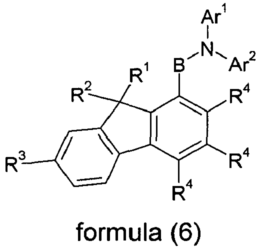 where the symbols and indices indicated are defined as indicated in Claim 1.

7. Device according to one or more of Claims 1 to 6, **characterised in that** B is a single bond or a phenylene, biphenylene, terphenylene, naphthylene, pyridinylene, pyrimidinylene, pyrazinylene, pyridazinylene, triazinylene, dibenzofuranylene, dibenzothiophenylene fluorenylene, or carbazoylene group, which may be substituted by one or more radicals R⁶; B is preferably a single bond or a phenylene, biphenylene, terphenylene, naphthylene, dibenzofuranylene or dibenzothiophenylene fluorenylene, or carbazoylene group, which may be substituted by one or more radicals R⁶; B is very preferably a single bond or a phenylene group, which may be substituted by one or more radicals R⁶; B is very particularly preferably a single bond.

8. Device according to one or more of Claims 1 to 7, **characterised in that** Ar¹ and Ar² are selected, identically or differently on each occurrence, from a phenylpyridyl, phenylnaphthyl, biphenyl, terphenyl or quaterphenyl group, which may be substituted by one or more radicals R⁶, which may be identical to or different from one another, where two of the aromatic or heteroaromatic rings in Ar¹ may be bridged by a divalent group -O-, -S-, -C(R⁵)₂- or -Si(R⁵)₂- or two of the aromatic or heteroaromatic rings in Ar² may be bridged by a divalent group -O-, -S-, -C(R⁵)₂- or -Si(R⁵)₂-, where unbridged rings are preferred, and where an aromatic or heteroaromatic ring from Ar¹ may be bridged to an aromatic or heteroaromatic ring from Ar² by a divalent group -O-, -S-, -Si(R⁵)₂-, -NR⁵- or -C(R⁵)₂-, where unbridged groups Ar¹ and Ar² are preferred.

9. Device according to one or more of Claims 1 to 8, **characterised in that** the compound of the formulae (1) to (6) is a monoamine compound.

10. Device according to one or more of Claims 1 to 9, **characterised in that** the device is an organic light-emitting transistor (OLETs), an organic field-quench device (OFQDs), an organic light-emitting electrochemical cells (OLECs, LECs or LEECs), an organic laser diode (O-laser) and an organic light-emitting diode (OLEDs), preferably an OLED.

11. Device according to one or more of Claims 1 to 10, **characterised in that** the at least one compound of the formula (1) to (6) is employed with the following functions and in the following layers in the device:
• as hole-transport material in a hole-transport or hole-injection layer
• as exciton-blocking material,
• as electron-blocking material,
• as matrix material in an emitting layer,
• as emitter in an emitting layer.

12. Compound of the general formula (255) 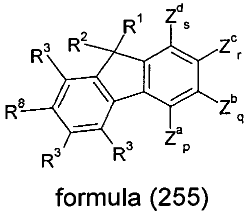 where the following applies to the symbols and indices used:
p, q, r, s
are 0 or 1, where p + q + r + s = 1, preferably p =1 or r = 1 or s = 1, very preferably p = 1 or r = 1;
Z^{a}₀, Z^{b}₀, Z^{c}₀, Z^{d}₀
are, identically or differently on each occurrence, equal to R⁴
Z^{a}₁, Z^{b}₁, Z^{c}₁, Z^{d}1 are equal to 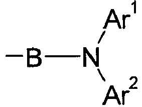
B
is a single bond, a divalent aryl group having 6 to 30 ring atoms or a divalent heteroaryl group having 5 to 30 ring atoms, each of which may be substituted by one or more radicals R⁶, where, if B is a single bond, the nitrogen atom is bonded directly to the fluorene;
Ar¹, Ar²
are on each occurrence, identically or differently, an uncondensed aryl group having 10 to 60 ring atoms or a heteroaryl group 10 to 60 ring atoms, which may be substituted by one or more radicals R⁵, which are identical to or different from one another, where both groups Ar¹ or Ar² each contain at least two or more aromatic or heteroaromatic rings,
where two of the aromatic or heteroaromatic rings in Ar¹ may be bridged by a divalent group -O-, -S-, -Si(R⁵)₂-, -C(R⁵)₂- or -NR⁵- or two of the aromatic or heteroaromatic rings in Ar² may be bridged by a divalent group -O-, -S-, -Si(R⁵)₂-, -C(R⁵)₂- or -NR⁵-, where unbridged rings are preferred
and where an aromatic or heteroaromatic ring from Ar¹ may be bridged to an aromatic or heteroaromatic ring from Ar² by a divalent group -O-, -S-, -Si(R⁵)₂-, -NR⁵- or -C(R⁵)₂-, where unbridged groups Ar¹ and Ar² are preferred;
R¹, R², R³ and R⁴
are H, D, F, Cl, Br, I, C(=O)R⁶, CN, Si(R⁶)₃, NO₂, N(R⁶)₂, P(=O)-(R⁶)₂, S(=O)R⁶, S(=O)₂R⁶, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁶ and where one or more CH₂ groups in the above-mentioned groups may be replaced by -R⁶C=CR⁶-, -C=C-, Si(R⁶)₂, C=O, C=S, C=NR⁶, -C(=O)O-, -C(=O)NR⁶-, P(=O)(R⁶), -O-, -S-, SO or SO₂ and where one or more H atoms in the above-mentioned groups may be replaced by D, F, Cl, Br, I, CN or NO₂, or an aromatic or heteroaromatic ring system having 6 to 30 aromatic ring atoms, which may in each case be substituted by one or more radicals R⁶, or an aryloxy group having 5 to 60 aromatic ring atoms, which may be substituted by one or more radicals R⁶, or an aralkyl group having 5 to 60 aromatic ring atoms, which may in each case be substituted by one or more radicals R⁶,
where the radicals R¹ and R² cannot be identical and the radicals R³ to R⁵ may on each occurrence be identical or different, but may be identical to either R¹ or to R²
and where at least one of the radicals from R¹ and R² represents an aromatic or heteroaromatic ring system having 6 to 30 aromatic ring atoms, which may in each case be substituted by one or more radicals R⁶;
R5
is H, D, C(=O)R⁶, CN, Si(R⁶)₃, NO₂, N(R⁶)₂, P(=O)(R⁶)₂, S(=O)R⁶, S(=O)₂R⁶, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁶ and where one or more CH₂ groups in the above-mentioned groups may be replaced by -R⁶C=CR⁶-, -C=C-, Si(R⁶)₂, C=O, C=S, C=NR⁶, -C(=O)O-, -C(=O)NR⁶-, P(=O)(R⁶), -O-, -S-, SO or SO₂ and where one or more H atoms in the above-mentioned groups may be replaced by D, F, Cl, Br, I, CN or NO₂, or an aromatic or heteroaromatic ring system having 6 to 30 aromatic ring atoms, which may in each case be substituted by one or more radicals R⁶, or an aryloxy group having 5 to 60 aromatic ring atoms, which may be substituted by one or more radicals R⁶, or an aralkyl group having 5 to 60 aromatic ring atoms, which may in each case be substituted by one or more radicals R⁶,
R6
is on each occurrence, identically or differently, H, D, F, Cl, Br, I, C(=O)R⁷, CN, Si(R⁷)₃, NO₂, P(=O)(R⁷)₂, S(=O)R⁷, S(=O)₂R⁷, N(R⁷)₂, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁷ and where one or more CH₂ groups in the above-mentioned groups may be replaced by -R⁷C=CR⁷-, -C=C-, Si(R⁷)₂, C=O, C=S, C=NR⁷, -C(=O)O-, -C(=O)NR⁷-, P(=O)(R⁷), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R⁷, or an aryloxy or heteroaryloxy group having 5 to 30 aromatic ring atoms, which may be substituted by one or more radicals R⁷, where two or more adjacent substituents R⁶ may form a mono- or polycyclic ring system with one another;
R⁷
is selected from the group consisting of H, D, F, an aliphatic hydrocarbon radical having 1 to 20 C atoms or an aromatic or heteroaromatic ring system having 5 to 30 C atoms, in which one or more H atoms may be replaced by D or F, where two or more adjacent substituents R⁷ may form a mono- or polycyclic ring system with one another;
R⁸
is H, D, C(=O)R⁹, CN, Si(R⁹)₃, NO₂, N(R⁹)₂, P(=O)(R⁹)₂, S(=O)R⁹, S(=O)₂R⁹, a straight-chain alkyl, alkoxy or thioalkyl group having 1 to 20 C atoms or a branched or cyclic alkyl or thioalkyl group having 3 to 20 C atoms or an alkenyl or alkynyl group having 2 to 20 C atoms, where the above-mentioned groups may each be substituted by one or more radicals R⁹ and where one or more CH₂ groups in the above-mentioned groups may be replaced by -R⁹C=CR⁹-, -C=C-, Si(R⁹)₂, C=O, C=S, C=NR⁹, -C(=O)O-, -C(=O)NR⁹-, P(=O)(R⁹), -S-, SO or SO₂ and where one or more H atoms in the above-mentioned groups may be replaced by D, F, Cl, Br, I, CN or NO₂, or an aromatic or heteroaromatic ring system having 6 to 30 aromatic ring atoms, which may in each case be substituted by one or more radicals R⁹, or an aryloxy group having 5 to 60 aromatic ring atoms, which may be substituted by one or more radicals R⁹, or an aralkyl group having 5 to 60 aromatic ring atoms, which may in each case be substituted by one or more radicals R⁹;
R⁹
is on each occurrence, identically or differently, H, D, F, Cl, Br, I, C(=O)R¹⁰, CN, Si(R¹⁰)₃, NO₂, P(=O)(R¹⁰)₂, S(=O)R¹⁰, S(=O)₂R¹⁰, N(R¹⁰)₂, a straight-chain alkyl or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R¹⁰ and where one or more CH₂ groups in the above-mentioned groups may be replaced by -R¹⁰C=CR¹⁰-, -C≡C-, Si(R¹⁰)₂, C=O, C=S, C=NR¹⁰, -C(=O)O-, -C(=O)NR¹⁰-, P(=O)(R¹⁰), -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R¹⁰, or an aryloxy or heteroaryloxy group having 5 to 30 aromatic ring atoms, which may be substituted by one or more radicals R¹⁰, where two or more adjacent substituents R¹⁰ may form a mono- or polycyclic ring system with one another;
R¹⁰
is selected from the group consisting of H, D, F, an aliphatic hydrocarbon radical having 1 to 20 C atoms or an aromatic or heteroaromatic ring system having 5 to 30 C atoms, in which one or more H atoms may be replaced by D or F, where two or more adjacent substituents R¹⁰ may form a mono- or polycyclic ring system with one another.

13. Compound according to Claim 12, **characterised in that** it has the general formula (256) 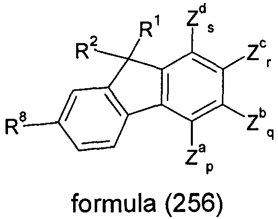 where the symbols and indices indicated are defined as indicated in Claim 12.

14. Process for the preparation of the compound according to Claim compound according to Claim 12 or 13 by means of Buchwald coupling.

15. Use of the compound according to Claim 12 or 13 in an electronic device.

16. Electronic device comprising at least one compound according to Claim 12 or 13, where the electronic device is preferably selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers).

## Revendications

1. Dispositif électroluminescent comprenant au moins un composé de la formule générale (1) : 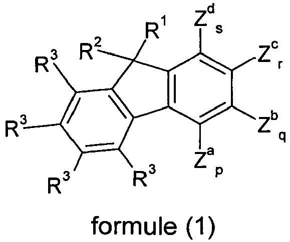 dans laquelle ce qui suit s'applique aux symboles et indices utilisés :
p, q, r, s
sont 0 ou 1, où p + q + r + s = 1, de façon préférable p =1 ou r = 1 ou s = 1, de façon très préférable, p = 1 ou r = 1 ;
Z^{a}₀, Z^{b}₀, Z^{c}₀, Z^{d}₀
sont, de manière identique ou différente pour chaque occurrence, égaux à R⁴,
Z^{a}₁, Z^{b}₁, Z^{c}₁, Z^{d}₁ sont égaux à 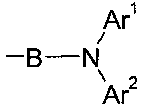
B
est une liaison simple, un groupe aryle divalent comportant 6 à 30 atomes de cycle ou un groupe hétéroaryle divalent comportant 5 à 30 atomes de cycle, dont chacun peut être substitué par un radical ou plusieurs radicaux R⁶, où, si B est une liaison simple, l'atome d'azote est lié directement au fluorène ;
Ar¹, Ar²
sont, pour chaque occurrence, de manière identique ou différente, un groupe aryle comportant 10 à 60 atomes de cycle ou un groupe hétéroaryle comportant 10 à 60 atomes de cycle, lesquels peuvent être substitués par un radical ou plusieurs radicaux R⁵, lesquels sont identiques les uns aux autres ou différents les uns des autres, où les deux groupes Ar¹ ou Ar² contiennent chacun au moins deux cycles aromatiques ou hétéroaromatiques ou plus,
où deux des cycles aromatiques ou hétéroaromatiques dans Ar¹ et/ou deux des cycles aromatiques ou hétéroaromatiques dans Ar² peuvent être condensés, ils sont de façon préférable sous forme non condensée,
et où deux des cycles aromatiques ou hétéroaromatiques dans Ar¹ peuvent être pontés par un groupe divalent -O-, -S-, -Si(R⁵)₂-, -C(R⁵)₂ ou -NR⁵- ou deux des cycles aromatiques ou hétéroaromatiques dans Ar² peuvent être pontés par un groupe divalent -O-, -S-, -Si(R⁵)₂-, -C(R⁵)₂,- ou -NR⁵-, où des cycles non pontés ont la préférence,
et où un cycle aromatique ou hétéroaromatique issu de Ar¹ peut être ponté sur un cycle aromatique ou hétéroaromatique issu de Ar² par un groupe divalent -O-, -S-, -Si(R⁵)₂-, -NR⁵- ou -C(R⁵)₂-, où des groupes non pontés Ar¹ et Ar² ont la préférence ;
R¹, R², R³, R⁴, et R⁵
sont H, D, F, Cl, Br, I, C(=O)R⁶, CN, Si(R⁶)₃, NO₂, N(R⁶)₂, P(=O)-(R⁶)₂, S(=O)R⁶, S(=O)₂R⁶, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁶ et où un ou plusieurs groupe(s) CH₂ dans les groupes mentionnés ci avant peut/peuvent être remplacé(s) par -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO ou SO₂ et où un ou plusieurs atome(s) de H dans les groupes mentionnés ci avant peut/peuvent être remplacé(s) par D, F, CI, Br, I, CN ou NO₂, ou un système de cycle aromatique ou hétéroaromatique comportant 6 à 30 atomes de cycle aromatique, lequel peut dans chaque cas être substitué par un radical ou plusieurs radicaux R⁶, ou un groupe aryloxy comportant 5 à 60 atomes de cycle aromatique, lequel peut être substitué par un radical ou plusieurs radicaux R⁶, ou un groupe aralkyle comportant 5 à 60 atomes de cycle aromatique, lequel peut dans chaque cas être substitué par un radical ou plusieurs radicaux R⁶, où les radicaux R¹ et R² ne peuvent pas être identiques et les radicaux R³ à R⁵ peuvent, pour chaque occurrence, être identiques ou différents, mais peuvent être identiques soit à R¹, soit à R² ;
R⁶
est, pour chaque occurrence, de manière identique ou différente, H, D, F, CI, Br, I, C(=O)R⁷, CN, Si(R⁷)₃, NO₂, P(=O)(R⁷)₂, S(=O)R⁷, S(=O)2R⁷, N(R⁷)₂, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁷ et où un ou plusieurs groupes CH₂ dans les groupes mentionnés ci avant peut/ peuvent être remplacé(s) par -R⁷C=CR⁷-, -C=C-, Si(R⁷)₂, C=O, C=S, C=NR⁷, -C(=O)O-, -C(=O)NR⁷-, P(=O)(R⁷), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 5 à 30 atomes de cycle aromatique, lequel peut dans chaque cas être substitué par un radical ou plusieurs radicaux R⁷, ou un groupe aryloxy ou hétéroaryloxy comportant 5 à 30 atomes de cycle aromatique, lequel peut être substitué par un radical ou plusieurs radicaux R⁷, où deux substituants R⁶ adjacents ou plus peuvent former un système de cycle monocyclique ou polycyclique l'un avec l'autre ou les uns avec les autres ;
R⁷
est choisi parmi le groupe constitué par H, D, F, un radical hydrocarbone aliphatique comportant 1 à 20 atome(s) de C ou un système de cycle aromatique ou hétéroaromatique comportant 5 à 30 atomes de C, où un ou plusieurs atome(s) de H peut/peuvent être remplacé(s) par D ou F, où deux substituants R⁷ adjacents ou plus peuvent former un système de cycle monocyclique ou polycyclique l'un avec l'autre ou les uns avec les autres.

2. Dispositif selon la revendication 1, **caractérisé en ce que** le composé présente la formule générale (2) : 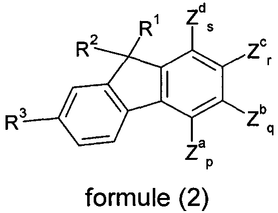 dans laquelle les symboles et indices indiqués sont définis comme indiqué selon la revendication 1.

3. Dispositif selon la revendication 1 ou 2, **caractérisé en ce que** le composé présente la formule générale (3) : 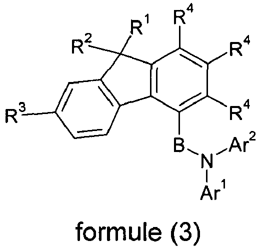 dans laquelle les symboles et indices indiqués sont définis comme indiqué selon la revendication 1.

4. Dispositif selon la revendication 1 ou 2, **caractérisé en ce que** le composé présente la formule générale (4) : 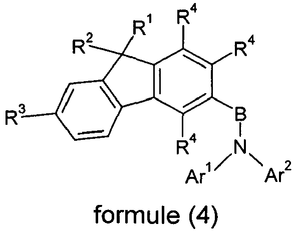 dans laquelle les symboles et indices indiqués sont définis comme indiqué selon la revendication 1.

5. Dispositif selon la revendication 1 ou 2, **caractérisé en ce que** le composé présente la formule générale (5) : 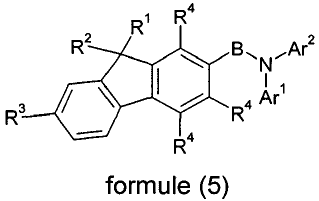 dans laquelle les symboles et indices indiqués sont définis comme indiqué selon la revendication 1.

6. Dispositif selon la revendication 1 ou 2, **caractérisé en ce que** le composé présente la formule générale (6) : 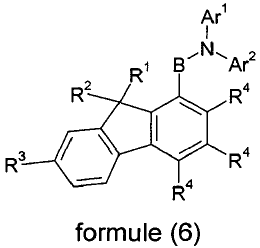 dans laquelle les symboles et indices indiqués sont définis comme indiqué selon la revendication 1.

7. Dispositif selon une ou plusieurs des revendications 1 à 6, **caractérisé en ce que** B est une liaison simple ou un groupe phénylène, biphénylène, terphénylène, naphtylène, pyridinylène, pyrimidinylène, pyrazinylène, pyridazinylène, triazinylène, dibenzofuranylène, dibenzothiophénylène fluorénylène, ou carbazoylène, lequel peut être substitué par un radical ou plusieurs radicaux R⁶ ; B est, de façon préférable, une liaison simple ou un groupe phénylène, biphénylène, terphénylène, naphtylène, dibenzofuranylène ou dibenzothiophénylène fluorénylène, ou carbazoylène, lequel peut être substitué par un radical ou plusieurs radicaux R⁶ ; B est, de façon très préférable, une liaison simple ou un groupe phénylène, lequel peut être substitué par un radical ou plusieurs radicaux R⁶ ; B est, de façon très particulièrement préférable, une liaison simple.

8. Dispositif selon une ou plusieurs des revendications 1 à 7, **caractérisé en ce que** Ar¹ et Ar² sont choisis, de manière identique ou différente pour chaque occurrence, parmi un groupe phénylpyridyle, phénylnaphtyle, biphényle, terphényle ou quaterphényle, lesquels groupes peuvent être substitués par un radical ou plusieurs radicaux R⁶, lesquels peuvent être identiques les uns aux autres ou différents les uns des autres, où deux des cycles aromatiques ou hétéroaromatiques dans Ar¹ peuvent être pontés par un groupe divalent -O-, -S-, -C(R⁵)₂- ou -Si(R⁵)₂- ou deux des cycles aromatiques ou hétéroaromatiques dans Ar² peuvent être pontés par un groupe divalent -O-, -S-, -C(R⁵)₂- ou -Si(R⁵)₂-, où des cycles non pontés ont la préférence, et où un cycle aromatique ou hétéroaromatique dans Ar¹ peut être ponté sur un cycle aromatique ou hétéroaromatique issu de Ar² par un groupe divalent -O-, -S-, -Si(R⁵)₂-, -NR⁵- ou -C(R⁵)₂-, où des groupes non pontés Ar¹ et Ar² ont la préférence.

9. Dispositif selon une ou plusieurs des revendications 1 à 8, **caractérisé en ce que** le composé des formules (1) à (6) est un composé monoamine.

10. Dispositif selon une ou plusieurs des revendications 1 à 9, **caractérisé en ce que** le dispositif est un transistor à émission de lumière organique (OLET), un dispositif à extinction de champ organique (OFQD), une cellule électrochimique à émission de lumière organique (OLEC, LEC ou LEEC), une diode laser organique (O-laser) et une diode à émission de lumière organique (OLED), de façon préférable, une OLED.

11. Dispositif selon une ou plusieurs des revendications 1 à 10, **caractérisé en ce que** l'au moins un composé des formules (1) à (6) est utilisé avec les fonctions qui suivent et dans les couches qui suivent dans le dispositif :
• en tant que matériau de transport de trous dans une couche de transport de trous ou d'injection de trous,
• en tant que matériau de blocage d'excitons,
• en tant que matériau de blocage d'électrons,
• en tant que matériau de matrice dans une couche d'émission,
• en tant qu'émetteur dans une couche d'émission.

12. Composé de la formule générale (255) : 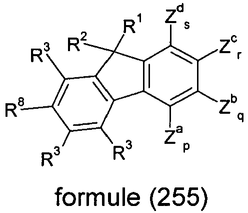 dans laquelle ce qui suit s'applique aux symboles et indices utilisés :
p, q, r, s
sont 0 ou 1, où p + q + r + s = 1, de façon préférable p =1 ou r = 1 ou s = 1, de façon très préférable, p = 1 ou r = 1 ;
Z^{a}₀, Z^{b}₀, Z^{c}₀, Z^{d}₀
sont, de manière identique ou différente pour chaque occurrence, égaux à R⁴
Z^{a}₁, Z^{b}₁, Z^{c}₁, Z^{d}₁ sont égaux à 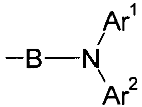
B
est une liaison simple, un groupe aryle divalent comportant 6 à 30 atomes de cycle ou un groupe hétéroaryle divalent comportant 5 à 30 atomes de cycle, dont chacun peut être substitué par un radical ou plusieurs radicaux R⁶, où, si B est une liaison simple, l'atome d'azote est lié directement au fluorène ;
Ar¹, Ar²
sont, pour chaque occurrence, de manière identique ou différente, un groupe aryle non condensé comportant 10 à 60 atomes de cycle ou un groupe hétéroaryle comportant 10 à 60 atomes de cycle, lesquels peuvent être substitués par un radical ou plusieurs radicaux R⁵, lesquels sont identiques les uns aux autres ou différents les uns des autres, où les deux groupes Ar¹ ou Ar² contiennent chacun au moins deux cycles aromatiques ou hétéroaromatiques ou plus,
où deux des cycles aromatiques ou hétéroaromatiques dans Ar¹ peuvent être pontés par un groupe divalent -O-, -S-, -Si(R⁵)₂-, -C(R⁵)₂- ou -NR⁵- ou deux des cycles aromatiques ou hétéroaromatiques dans Ar² peuvent être pontés par un groupe divalent -O-, -S-, -Si(R⁵)₂-, -C(R⁵)₂- ou -NR⁵-, où des cycles non pontés ont la préférence,
et où un cycle aromatique ou hétéroaromatique issu de Ar¹ peut être ponté sur un cycle aromatique ou hétéroaromatique issu de Ar² par un groupe divalent -O-, -S-, -Si(R⁵)₂-, -NR⁵- ou -C(R⁵)₂-, où des groupes non pontés Ar¹ et Ar² ont la préférence ;
R¹, R², R³ et R⁴
sont H, D, F, Cl, Br, I, C(=O)R⁶, CN, Si(R⁶)₃, NO₂, N(R⁶)₂, P(=O)-(R⁶)₂, S(=O)R⁶, S(=O)₂R⁶, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁶ et où un ou plusieurs groupe(s) CH₂ dans les groupes mentionnés ci avant peut/peuvent être remplacé(s) par -R⁶C=CR⁶-, -C=C-, Si(R⁶)₂, C=O, C=S, C=NR⁶, -C(=O)O-, -C(=O)NR⁶-, P(=O)(R⁶), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 6 à 30 atomes de cycle aromatique, lequel peut, dans chaque cas, être substitué par un radical ou plusieurs radicaux R⁶, ou un groupe aryloxy comportant 5 à 60 atomes de cycle aromatique, lequel peut être substitué par un radical ou plusieurs radicaux R⁶, ou un groupe aralkyle comportant 5 à 60 atomes de cycle aromatique, lequel peut, dans chaque cas, être substitué par un radical ou plusieurs radicaux R⁶,
où les radicaux R¹ et R² ne peuvent pas être identiques et les radicaux R³ à R⁵ peuvent, pour chaque occurrence, être identiques ou différents, mais peuvent être identiques soit à R¹, soit à R²,
et où au moins l'un des radicaux pris parmi R¹ et R² représente un système de cycle aromatique ou hétéroaromatique comportant 6 à 30 atomes de cycle aromatique, lequel peut, dans chaque cas, être substitué par un radical ou plusieurs radicaux R⁶ ;
R⁵
est H, D, C(=O)R⁶, CN, Si(R⁶)₃, NO₂, N(R⁶)₂, P(=O)(R⁶)₂, S(=O)R⁶, S(=O)₂R⁶, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁶ et où un ou plusieurs groupe(s) CH₂ dans les groupes mentionnés ci avant peut/peuvent être remplacé(s) par -R⁶C=CR⁶-, -C=C-, Si(R⁶)₂, C=O, C=S, C=NR⁶, -C(=O)O-, -C(=O)NR⁶-, P(=O)(R⁶), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 6 à 30 atomes de cycle aromatique, lequel peut, dans chaque cas, être substitué par un radical ou plusieurs radicaux R⁶, ou un groupe aryloxy comportant 5 à 60 atomes de cycle aromatique, lequel peut être substitué par un radical ou plusieurs radicaux R⁶, ou un groupe aralkyle comportant 5 à 60 atomes de cycle aromatique, lequel peut, dans chaque cas, être substitué par un radical ou plusieurs radicaux R⁶,
R⁶
est, pour chaque occurrence, de manière identique ou différente, H, D, F, Cl, Br, I, C(=O)R⁷, CN, Si(R⁷)₃, NO₂, P(=O)(R⁷)₂, S(=O)R⁷, S(=O)₂R⁷, N(R⁷)₂, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁷ et où un ou plusieurs groupe(s) CH₂ dans les groupes mentionnés ci avant peut/ peuvent être remplacé(s) par -R⁷C=CR⁷-, -C=C-, Si(R⁷)₂, C=O, C=S, C=NR⁷, -C(=O)O-, -C(=O)NR⁷-, P(=O)(R⁷), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 5 à 30 atomes de cycle aromatique, lequel peut, dans chaque cas, être substitué par un radical ou plusieurs radicaux R⁷, ou un groupe aryloxy ou hétéroaryloxy comportant 5 à 30 atomes de cycle aromatique, lequel peut être substitué par un radical ou plusieurs radicaux R⁷, où deux substituants R⁶ adjacents ou plus peuvent former un système de cycle monocylique ou polycyclique l'un avec l'autre ou les uns avec les autres ;
R⁷
est choisi parmi le groupe constitué par H, D, F, un radical hydrocarbone aliphatique comportant 1 à 20 atome(s) de C ou un système de cycle aromatique ou hétéroaromatique comportant 5 à 30 atomes de C, où un ou plusieurs atome(s) de H peut/peuvent être remplacé(s) par D ou F, où deux substituants R⁷ adjacents ou plus peuvent former un système de cycle monocyclique ou polycyclique l'un avec l'autre ou les uns avec les autres ;
R⁸
est H, D, C(=O)R⁹, CN, Si(R⁹)₃, NO₂, N(R⁹)₂, P(=O)(R⁹)₂, S(=O)R⁹, S(=O)₂R⁹, un groupe alkyle, alcoxy ou thioalkyle en chaîne droite comportant 1 à 20 atome(s) de C ou un groupe alkyle ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁹ et où un ou plusieurs groupes CH₂ dans les groupes mentionnés ci avant peut/peuvent être remplacé(s) par -R⁹C=CR⁹-, -C=C-, Si(R⁹)₂, C=O, C=S, C=NR⁹, -C(=O)O-, -C(=O)NR⁹-, P(=O)(R⁹), -S-, SO ou SO₂ et où un ou plusieurs atomes de H dans les groupes mentionnés ci avant peut/ peuvent être remplacé(s) par D, F, Cl, Br, I, CN ou NO₂, ou un système de cycle aromatique ou hétéroaromatique comportant 6 à 30 atomes de cycle aromatique, lequel peut, dans chaque cas, être substitué par un radical ou plusieurs radicaux R⁹, ou un groupe aryloxy comportant 5 à 60 atomes de cycle aromatique, lequel peut être substitué par un radical ou plusieurs radicaux R⁹, ou un groupe aralkyle comportant 5 à 60 atomes de cycle aromatique, lequel peut, dans chaque cas, être substitué par un radical ou plusieurs radicaux R⁹ ;
R⁹
est, pour chaque occurrence, de manière identique ou différente, H, D, F, Cl, Br, I, C(=O)R¹⁰, CN, Si(R¹⁰)3, NO₂, P(=O)(R¹⁰)₂, S(=O)R¹⁰, S(=O)₂R¹⁰, N(R¹⁰)₂, un groupe alkyle ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R¹⁰ et où un ou plusieurs groupe(s) CH₂ dans les groupes mentionnés ci avant peut/peuvent être remplacé(s) par -R¹⁰C=CR¹⁰-, -C=C-, Si(R¹⁰)₂, C=O, C=S, C=NR¹⁰, -C(=O)O-, -C(=O)NR¹⁰-, P(=O)(R¹⁰), -S-, SO ou SO₂ et où un ou plusieurs atome(s) de H dans les groupes mentionnés ci avant peut/peuvent être remplacé(s) par D, F, CI, Br, I, CN ou NO₂, ou un système de cycle aromatique ou hétéroaromatique comportant 5 à 30 atomes de cycle aromatique, lequel peut, dans chaque cas, être substitué par un radical ou plusieurs radicaux R¹⁰, ou un groupe aryloxy ou hétéroaryloxy comportant 5 à 30 atomes de cycle aromatique, lequel peut être substitué par un radical ou plusieurs radicaux R¹⁰, où deux substituants R¹⁰ adjacents ou plus peuvent former un système de cycle monocyclique ou polycyclique l'un avec l'autre ou les uns avec les autres ;
R¹⁰
est choisi parmi le groupe constitué par H, D, F, un radical hydrocarbone aliphatique comportant 1 à 20 atome(s) de C ou un système de cycle aromatique ou hétéroaromatique comportant 5 à 30 atomes de C, où un ou plusieurs atome(s) de H peut/peuvent être remplacé(s) par D ou F, où deux substituants R¹⁰ adjacents ou plus peuvent former un système de cycle monocyclique ou polycyclique l'un avec l'autre ou les uns avec les autres.

13. Composé selon la revendication 12, **caractérisé en ce qu'**il présente la formule générale (256) : 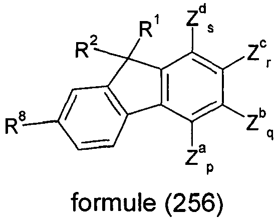 dans laquelle les symboles et indices indiqués sont définis comme indiqué selon la revendication 12.

14. Procédé pour la préparation du composé selon la revendication 12 ou 13 au moyen d'un couplage de Buchwald.

15. Utilisation du composé selon la revendication 12 ou 13 dans un dispositif électronique.

16. Dispositif électronique comprenant au moins un composé selon la revendication 12 ou 13, où le dispositif électronique est de façon préférable choisi parmi le groupe constitué par des circuits intégrés organiques (OIC), des transistors à effet de champ organiques (OFET), des transistors à film mince organiques (OTFT), des transistors à émission de lumière organiques (OLET), des cellules solaires organiques (OSC), des détecteurs optiques organiques, des photorécepteurs organiques, des dispositifs à extinction de champ organiques (OFQD), des cellules électrochimiques à émission de lumière organiques (OLEC), des diodes laser organiques (O-laser).
